# EUROPEAN PATENT SPECIFICATION

(11) **EP 4 390 399 B1**
(45) Date of publication and mention of the grant of the patent: **16.07.2025**
(21) Application number: 22216509.4
(22) Date of filing: 23.12.2022
(51) Int. Cl.: G01N 33/68

(54) **SERUM PEPTIDE PATTERNS FOR DIAGNOSTICS OF ACUTE ISCHEMIC STROKE IN HUMANS AND DIFFERENTIAL DAIGNOSIS OF ACUTE ISCHEMIC STROKE FROM INTRACRANIAL HEMORRHAGIC STROKE**
SERUMPEPTIDMUSTER ZUR DIAGNOSE AKUTER ISCHÄMISCHER SCHLAGANFÄLLE BEIM MENSCHEN UND DIFFERENTIELLER DALIGNOSE AKUTER ISCHÄMISCHER SCHLAGANFÄLLE AUFGRUND INTRAKRANIALER HÄMORRHAGISCHER SCHLAGANFÄLLE
MOTIFS PEPTIDIQUES SÉRIQUES POUR LE DIAGNOSTIC D'UN ACCIDENT VASCULAIRE CÉRÉBRAL ISCHÉMIQUE AIGU CHEZ L'HOMME ET LA DAIGNOSE DIFFÉRENTIELLE D'UN ACCIDENT VASCULAIRE CÉRÉBRAL HÉMORRAGIQUE INTRACRÂNIENS

(43) Date of publication of application: 26.06.2024
(73) Proprietor: Uniwersytet Gdanski, 80-309 Gdansk (PL)
(72) Inventor: Kote, Sachin, 80-116 Gdansk (PL); Marek-Trzonkowska, Natalia, 80-752 Gdansk (PL); Czaplewska, Paulina, 80-461 Gdansk (PL); Müller, Marc, 80-462 Gdansk (PL); Pirog, Artur, 80-312 Gdansk (PL); Faktor, Jakub, 80-170 Gdansk (PL)
(74) Representative: Czarnik, Maciej

(56) References cited:
- EP-A1- 3 029 466
- LEE JIYEONG ET AL: "Proteomics-Based Identification of Diagnostic Biomarkers Related to Risk Factors and Pathogenesis of Ischemic Stroke", DIAGNOSTICS, vol. 10, no. 5, 25 May 2020 (2020-05-25), pages 1 - 14, XP093055289, DOI: 10.3390/diagnostics10050340
- ROSSI ROSANNA ET AL: "Potential Biomarkers of Acute Ischemic Stroke Etiology Revealed by Mass Spectrometry-Based Proteomic Characterization of Formalin-Fixed Paraffin-Embedded Blood Clots", FRONTIERS IN NEUROLOGY, vol. 13, 19 April 2022 (2022-04-19), pages 1 - 12, XP093055311, DOI: 10.3389/fneur.2022.854846
- ARNAB DATTA ET AL: "The Proteomics Identifications (PRIDE) database and associated tools: Status in 2013", PLOS ONE, vol. 41, no. 4, 21 April 2014 (2014-04-21), pages D1063, XP055337644, DOI: 10.1371/journal.pone.0094663
- MALICEK DAVID ET AL: "Proteomics-Based Approach to Identify Novel Blood Biomarker Candidates for Differentiating Intracerebral Hemorrhage From Ischemic Stroke-A Pilot Study", FRONTIERS IN NEUROLOGY, vol. 12, 17 December 2021 (2021-12-17), XP093047042, ISSN: 1664-2295, DOI: 10.3389/fneur.2021.713124
- HONGQIANG QIN ET AL: "Highly Efficient Extraction of Serum Peptides by Ordered Mesoporous Carbon", ANGEWANDTE CHEMIE INTERNATIONAL EDITION, VERLAG CHEMIE, HOBOKEN, USA, vol. 50, no. 51, 26 October 2011 (2011-10-26), pages 12218 - 12221, XP072085211, ISSN: 1433-7851, DOI: 10.1002/ANIE.201103666
- KODALI PHANICHAND ET AL: "A preliminary method development study to identify potential stroke biomarkers in plasma using multiple chromatographies with nanoLC-ESIMS detection", JOURNAL OF NEURAL TRANSMISSION, vol. 120, no. 10, 13 April 2013 (2013-04-13), Vienna, pages 1 - 10, XP093061991, ISSN: 0300-9564, Retrieved from the Internet <URL:http://link.springer.com/article/10.1007/s00702-013-1018-9/fulltext.html> DOI: 10.1007/s00702-013-1018-9

## Description

The invention refers to the serum peptide patterns for diagnostics of acute ischemic stroke (AIS) in humans and differential diagnosis from intracranial hemorrhagic stroke.

### State of the art

The invention is related to serum peptide patterns for diagnostics of acute ischemic stroke (AIS) in humans and differential diagnosis from intracranial hemorrhagic stroke. The invention is based on the identification (qualitative analysis) and relative quantification (quantitative analysis) of unique for AIS multi-biomarker panel of serum peptides (identified based on amino acid sequences). 100 of the markers are increased 2 folds or more in sera of the patients with AIS as compared with the individuals without stroke and patients with intracranial hemorrhagic stroke (quantitative analysis), while the next 54 markers are detectable only in patients with AIS, but not in individuals without stroke and with intracranial hemorrhagic stroke (ICH). This serum peptide pattern can be used to diagnose and monitor AIS and patient response to the applied treatment.

As grade of the fold increase of the quantitative peptides correlates positively with their predictive significance for AIS diagnostics, we also define a serum peptide patterns 2, 3, 4 and 5. The serum peptide pattern 2 is a list of 32 peptides which levels are increased 30 folds or more in sera of AIS patients as compared with individuals without stroke and in patients with ICH. The serum peptide pattern 3 is a list of 24 peptides which levels are increased 50 folds or more in sera of AIS patients as compared with individuals without stroke and in patients with ICH. The serum peptide pattern 4 is a list of 16 peptides which levels are increased 100 folds or more in sera of AIS patients as compared with individuals without stroke and in patients with ICH. The serum peptide pattern 5 is a one peptide which levels are increased 1000 folds or more in sera of AIS patients as compared with individuals without stroke and in patients with ICH. Each of five serum peptide patterns can be used as a separate independent panel of markers for diagnostics of AIS and differentiation of AIS from ICH, as well as for monitoring AIS and patient response to the applied treatment.

Stroke is a type of cardiovascular disease (CVD) and one of the most frequent causes of death or disability in high-income countries. However, up to date, it is impossible to differentiate without magnetic resonance imaging (MRI) between AIS and ICH, while fast differential diagnostics is crucial for immediate implementation of the proper treatment that can save patient life and prevent from disability. As AIS and ICH have different pathophysiology, they require different treatment. Acute ischemic stroke is the primary cause of death and disabilities worldwide, contributing to 80% of all strokes [1], with long-lasting effects in western societies. It develops due to blockage of the neck arteries due to blood clotting. Over the past two decades, the approach to treating acute ischemic stroke (AIS) has changed. As opposed to previous pessimism regarding treatment, recanalization and reperfusion have proven successful in treating acute ischemic strokes. Mechanical thrombectomy and chemical thrombolysis with recombinant tissue plasminogen activator (rtPA) are the main treatments used to achieve reperfusion. Patients significantly improve the treatment outcome [2] when combined with general stroke unit procedures. For recombinant tissue plasminogen activator (rtPA) treatment, the administration timeframe is considered essential. To effectively improve the outcome, it is recommended to induce the treatment within the first 4 hours after symptom onset. Even here, the benefit increases the sooner the treatment starts. However, it still needs to be specific enough to discriminate the patients with strokes. Fast and effective discrimination of the patients with AIS from those with ICH without the need for specialized equipment. This issue is of great importance in developing countries where the access to the specialized diagnostic equipment is limited and during emergency situations like recent SARS-CoV2 pandemics, where number of patients with stroke dramatically increased as a complication of COVID19 [3], [4] boosting the need for fast and efficient discrimination between AIS and ICH stroke.

Peptidomics targets and analyses peptides (their length, distribution, protein origin, or de-novo sequencing). Peptides are protein fragments with various functions. As they regulate multiple physiological and pathological processes, alterations in peptidome in cells and various body fluids may reflect both beneficial or deleterious phenomena. Thus, peptidome analysis in body fluids such as blood samples is a promising approach for identifying peptide biomarkers of various conditions and monitoring patient response to the applied treatment. The peptidome is considered as low molecular-weight peptides/proteins [5] (less than 15 kilo Daltons; kDa). Endogenous peptides primarily act as a messenger (cytokines, hormones, growth factors, etc.) and indicate various biological processes. Hence, these peptides can reflect the health or disease state of the person. Even though peptidomics has been introduced since the decade, the serum peptidome analysis has consistently been restricted for various reasons; a) enormous complexity because serum contain elements of all proteins produced in the body, b) physiologically status of high abundant proteins such as serum albumin, immunoglobulins are almost 90% of total serum protein by weight, hence these abundant proteins mask the detection of other proteins, especially low abundance proteins or peptides, c) simultaneously limit detection of low abundant peptides with diagnostic potential, d) unequal concentrations of low and high abundant proteins (around 95% of high abundant proteins and less than 5% presence of low abundant) in the total serum protein concentrations, e) instability of low abundant proteins or peptides (hormones or small secreted proteins or possible diagnostic or prognostic biomarkers), f) depletion steps, fractionation and precipitation methods for the removal of high abundant proteins are overwhelming that compromise the number, yield of diagnostic peptides identified and important biological information can be lost in the background noise. Hence, all previously described methods for serum peptide analysis were characterized by low numbers of identified peptides, and no quantitative methods for serum peptidome analysis were introduced.

In the past, few patents were published with methods, devices, kits aiming to tackle AIS biomarker discovery with different approaches such as protein/peptide levels and immunoassay [6] [7] [8], mass spectrometry [21], plasma enzymes levels [9], etc. In addition, several past reports focused only on single proteins or enzyme biomarkers for stroke patients. However, considering a single biomarker such protein alpha-1 antitrypsin [10], L-glutamine hydroxylamine glutamyl transferase (L-GHGT) and gamma glutamyl hydroxamate synthetase (GGHS) activity [9] for stroke might be too risky to rely on accurate diagnosis. On the contrary, our approach enables us to find multi biomarkers of naturally occurring peptides from acute ischemic stroke (AIS). Data from previous literature showed that the serum peptidomics approach had been used for the diagnosis of various ranges of diseases. However, the ultimate success of these previously published methods is severely limited due to the low number of identified markers. On the contrary, we provide unique serum peptide patterns for diagnostics of AIS in humans and differential diagnosis from ICH. 100 of the markers are increased 2-fold or more in sera of the patients with AIS as compared with the individuals without stroke and patients with ICH (quantitative peptides), while the next 54 markers are detectable only in patients with AIS, but not in individuals without stroke and with ICH (qualitative peptides). As grade of the fold increase of the 100 quantitative peptides corresponds with their predictive significance for AIS diagnostics, we also define a panel of 32 peptides, panel of 24 peptides, panel of 16 peptides and panel of 1 peptide which can be used alone for AIS diagnostics or differentiation from ICH when their fold increase is equal or higher than 30, 50, 100 and 1000, respectively as compared with serum levels in patients without stroke and patients with ICH.

### Essence of the invention - description of the invention

The invention is disclosed in the claims. The invention is serum peptide patterns for diagnostics of acute ischemic stroke (AIS) in human and differential diagnosis from intracranial hemorrhagic stroke. The first serum peptide pattern is a list of 100 peptides (quantitative peptides) which levels are increased 2 folds or more in sera of the patients with AIS as assessed with relative comparison with serum samples of individuals without stroke and patients with ICH. The serum peptide pattern also comprises 54 peptides (qualitative peptides) that are detectable only in the patients with AIS but not in serum samples of individuals without stroke and patients with ICH. The 100 quantitative peptides are as follow (amino acid sequence of each peptide is given): Val-Ser-Glu-Thr-Glu-Ser-Arg-Gly-Ser-Glu-Ser-Gly-Ile-Phe-Thr-Asn-Thr-Lys, His-Arg-His-Pro-Asp-Glu-Ala-Ala-Phe-Phe-Asp-Thr-Ala-Ser-Thr, Phe-Phe-Asp-Thr-Ala-Ser-Thr-Gly-Lys, Gln-Leu-Gly-Leu-Pro-Gly-Pro-Pro-Asp-Val-Pro-Asp-His-Ala-Ala, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Ala-Ala-Thr-Val-Gly-Ser-Leu-Ala-Gly-Gln-Pro-Leu-Gln-Glu-Arg, Pro-Gly-Ser-Thr-Gly-Thr-Trp-Asn-Pro-Gly-Ser-Ser-Glu-Arg, His-Asp-Trp-Gly-His-Glu-Lys-Gln-Arg, His-Pro-Asp-Glu-Ala-Ala-Phe-Phe-Asp-Thr-Ala-Ser-Thr-Gly-Lys, Ser-Ser-His-His-Pro-Gly-Ile-Ala-Glu-Phe-Pro-Ser-Arg, Val-His-Leu-Thr-Pro-Glu-Glu-Lys-Ser-Ala-Val-Thr-Ala-Leu, Glu-Ser-Ser-Ser-His-His-Pro-Gly-Ile-Ala-Glu-Phe-Pro-Ser-Arg, Thr-Ala-Asn-Asp-Ser-Gly-Pro-Arg-Arg-Tyr-Thr-Ile-Ala-Ala-Leu, Arg-His-Asp-Trp-Gly-His-Glu-Lys-Gln-Arg, Val-Lys-Leu-Val-Asn-Glu-Val-Thr-Glu, Gln-Phe-Thr-Ser-Ser-Thr-Ser-Tyr-Asn, Ser-Gly-Asn-Val-Ser-Pro-Gly-Thr-Arg-Arg-Glu-Tyr-His-Thr-Glu-Lys, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Leu-Asp-Asp-Asp-Leu-Glu-His-Gln-Gly-Gly-His-Val-Leu-Asp-His-Gly-His, Ala-Phe-Arg-Gln-Pro-Ser-Ser-Ala, Phe-Val-Ser-Glu-Thr-Glu-Ser-Arg-Gly-Ser-Glu-Ser-Gly-Ile-Phe-Thr-Asn-Thr, Phe-Ser-Thr-Tyr-Asp-Arg-Asp-Asn-Asp-Gly-Trp, Thr-Val-Ile-Gln-Asn-Arg-Gln-Asp-Gly-Ser-Val-Asp-Phe, Arg-Glu-Tyr-His-Thr-Glu-Lys-Leu-Val-Thr-Ser, Gln-Ala-Gly-Ala-Ala-Gly-Ser-Arg-Met-Asn-Phe-Arg-Pro-Gly-Val-Leu-Ser-Ser, Ser-Pro-Gly-Ser-Gly-Asn-Ala-Arg-Pro-Asn-Asn-Pro-Asp-Trp-Gly-Thr-Phe, Ser-Asp-Ile-Ser-Ser-Thr-Arg-Gly-Phe-Pro-Ser-Val-Leu-Arg, Ile-Thr-His-Arg-Ile-His-Trp-Glu-Ser-Ala-Ser-Leu-Leu, Val-Ser-Gly-Trp-Gly-Arg-Asn-Ala-Asn-Phe, Phe-Asp-Thr-Ala-Ser-Thr-Gly-Lys-Thr-Phe-Pro-Gly-Phe, Ser-Ala-Gly-Ser-Trp-Asn-Ser-Gly-Ser-Ser-Gly-Pro-Gly-Ser-Thr-Gly-Asn-Arg-Asn, Val-His-Leu-Thr-Pro-Glu-Glu-Lys, Asp-Ala-Val-Arg-Gly-Ser-Pro-Ala-Ile-Asn-Val, Asp-Glu-Ala-Ala-Phe-Phe-Asp-Thr, Val-His-Leu-Thr-Pro-Glu-Glu-Lys-Ser-Ala-Val-Thr, Pro-Gly-Ser-Pro-Arg-Pro-Gly-Ser-Thr-Gly-Thr-Trp-Asn-Pro-Gly-Ser-Ser-Glu-Arg, Gly-Ser-Ser-Gly-Thr-Gly-Gly-Thr-Ala-Thr-Trp-Lys, Gly-Ser-Trp-Asn-Ser-Gly-Ser-Ser-Gly-Pro-Gly-Ser-Thr-Gly-Asn-Arg, Ser-Glu-Thr-Glu-Ser-Arg-Gly-Ser-Glu-Ser-Gly-Ile-Phe-Thr-Asn-Thr-Lys, Glu-Leu-Glu-Arg-Pro-Gly-Gly-Asn-Glu-Ile, Leu-Gly-Glu-Phe-Val-Ser-Glu-Thr-Glu-Ser-Arg, Ala-Leu-Glu-Glu-Tyr-Thr-Lys-Lys-Leu-Asn-Thr-Gln, Glu-Leu-Glu-Arg-Pro-Gly-Gly-Asn-Glu, Asp-Glu-Ala-Gly-Ser-Glu-Ala-Asp-His-Glu-Gly-Thr-His-Ser-Thr, Arg-His-Asp-Trp-Gly-His-Glu-Lys, Ala-Ala-Ser-Arg-Tyr-Leu-Asp-Lys-Thr-Glu-Gln, Arg-His-Pro-Asp-Glu-Ala-Ala-Phe, Pro-Asn-Ile-Leu-Arg-Leu-Glu-Ser-Glu-Glu-Thr, Ile-Glu-Gly-Val-Asp-Ala-Glu-Asp-Gly-His-Gly-Pro-Gly-Glu-Gln-Gln-Lys, Pro-Ser-Leu-Arg-Pro-Ala-Pro-Pro-Pro-Ile-Ser-Gly-Gly-Gly-Tyr, Thr-Asn-Thr-Lys-Glu-Ser-Ser-Ser-His-His-Pro-Gly-Ile-Ala-Glu-Phe-Pro-Ser-Arg-Gly, Ala-Glu-Val-Gly-Arg-Val-Gly-Tyr-Val-Ser-Gly-Trp-Gly-Arg-Asn-Ala-Asn-Phe, Asp-Glu-Ala-Ala-Phe-Phe-Asp-Thr-Ala-Ser-Thr-Gly-Lys, Lys-Val-Glu-Gln-Ala-Val-Glu-Thr-Glu-Pro-Glu-Pro-Glu-Leu-Arg-Gln-Gln-Thr, Met-Ala-Asp-Glu-Ala-Gly-Ser-Glu-Ala-Asp-His-Glu-Gly-Thr-His-Ser-Thr-Lys, Ser-Gly-Pro-Ser-Ser-Pro-Gln-Gln-Ala-Pro-Pro-Ala-Val-His, Gly-Ser-Glu-Ser-Gly-Ile-Phe-Thr-Asn-Thr-Lys-Glu-Ser-Ser-Ser-His-His-Pro-Gly-Ile-Ala-Glu-Phe-Pro-Ser-Arg-Gly, Arg-Glu-Glu-Ala-Pro-Ser-Leu-Arg, Glu-Glu-Val-Ser-Gly-Asn-Val-Ser-Pro-Gly-Thr, Glu-Asn-Glu-Gly-Phe-Thr-Val-Thr-Ala-Glu-Gly-Lys, Val-Thr-Glu-Pro-Ile-Ser-Ala-Glu-Ser-Gly-Glu-Gln-Val-Glu-Arg, Gly-Asp-Asp-Pro-Ser-Asp-Lys-Phe, Gly-Pro-Pro-Asp-Val-Pro-Asp-His-Ala, Glu-Glu-Val-Ser-Gly-Asn-Val-Ser-Pro-Gly-Thr-Arg-Arg-Glu, Tyr-His-Thr-Glu-Lys-Leu-Val, Tyr-Gln-Glu-Pro-Val-Leu-Gly-Pro-Val, Leu-Leu-Val-Arg-Tyr-Thr-Lys-Lys-Val-Pro-Gln-Val-Ser-Thr-Pro-Thr-Leu, Ile-Gln-Ile-Tyr-Lys-Lys-Leu-Arg-Thr-Ser-Ser-Phe, Ser-Val-Ser-Ala-Gly-Ser-Pro-His-Pro-Ala-Ile-Ala-Arg-Leu, His-Pro-Gly-Ile-Ala-Glu-Phe-Pro-Ser-Arg-Gly, Thr-Phe-Glu-Ser-Lys-Ser-Tyr, Ile-Glu-Gly-Val-Asp-Ala-Glu-Asp-Gly-His-Gly-Pro-Gly-Glu-Gln-Gln-Lys-Arg, Val-Leu-Val-Pro-Ala-Gly-Ser-Ala-Arg-Pro-Val-Ala-Phe, Asp-Ala-His-Lys-Ser-Glu-Val-Ala-His-Arg-Phe-Lys-Asp-Leu-Gly-Glu-Glu-Asn-Phe, Lys-Val-Lys-Glu-Lys-Leu-Lys-Ile-Asp-Ser, Glu-Phe-Val-Ser-Glu-Thr-Glu-Ser, His-Ala-Leu-Gln-Leu-Asn-Asn-Arg-Gln, Ser-Glu-Ser-Gly-Ile-Phe-Thr-Asn-Thr-Lys, Tyr-Val-Gly-Lys-Lys-Gln-Leu, Leu-Thr-Gln-Val-Lys-Glu-Ser-Leu-Ser-Ser-Tyr, Ser-Ser-His-His-Pro-Gly-Ile-Ala-Glu-Phe-Pro-Ser, Gly-Phe-Tyr-Glu-Ser-Asp-Val-Met-Gly-Arg, Gly-Gly-His-Leu-Asp-Ala-Lys-Gly-Ser-Phe, Asp-Val-Leu-Lys-Asp-Ser-Gly-Arg-Asp, His-Arg-His-Pro-Asp-Glu-Ala-Ala-Phe-Phe-Asp-Thr-Ala, Trp-Ser-Thr-Val-Lys-Asp-Lys-Phe-Ser-Glu-Phe, Arg-Gly-Ser-Glu-Ser-Gly-Ile-Phe-Thr-Asn-Thr, Glu-Glu-Ala-Pro-Ser-Leu-Arg-Pro-Ala-Pro-Pro-Pro-Ile-Ser-Gly-Gly-Gly-Tyr-Arg, Gly-Thr-Phe-Glu-Glu-Val-Ser-Gly-Asn-Val-Ser-Pro-Gly-Thr-Arg-Arg-Glu-Tyr-His-Thr-Glu, Gly-Ser-Ser-Gly-Thr-Gly-Gly-Thr-Ala-Thr-Trp-Lys-Pro-Gly-Ser-Ser, Ser-Ser-Ser-Tyr-Ser-Lys-Gln-Phe-Thr-Ser-Ser-Thr-Ser-Tyr-Asn-Arg-Gly-Asp-Ser-Thr-Phe-Glu-Ser-Lys-Ser-Tyr-Lys, Trp-Glu-Ser-Ala-Lys-Thr-Ala-Ala-Gln-Asn-Leu, Phe-Ser-Lys-Ala-Leu-Pro-Arg, Pro-Ser-Glu-Leu-Arg-Asp-Glu-His, Val-Val-Phe-Thr-Ala-Asn-Asp-Ser-Gly-Pro-Arg-Arg-Tyr-Thr-Ile-Ala, Leu-Gly-Glu-Phe-Val-Ser-Glu-Thr-Glu-Ser-Arg (Table 1)

The 54 qualitative peptides are (amino acid sequence of each peptide is given): Asp-Asp-Tyr-Val-Asn-Thr-Gln-Gly-Ala-Ser-Leu, Tyr-Pro-Trp-Thr-Gln-Arg-Phe, Phe-Arg-Leu-Leu-Gly-Asn-Val-Leu, Tyr-Gln-Lys-Val-Val-Ala-Gly-Val-Ala-Asn-Ala-Leu-Ala-His-Lys-Tyr-His, Phe-Ser-Glu-Thr-Phe-Gln-Lys-Val-Lys-Glu-Lys-Leu-Lys-Ile-Asp-Ser, Val-Lys-Glu-Lys-Leu-Lys-Ile-Asp-Ser, Tyr-Glu-Lys-Thr-Tyr-Leu-Pro-Ala-Val-Asp-Glu-Lys-Leu, Thr-Gln-Gln-Pro-Gln-Gln-Asp-Glu-Met-Pro-Ser-Pro-Thr-Phe-Leu, Thr-Gly-Ile-Phe-Thr-Asp-Gln-Val-Leu, Ser-Thr-Val-Lys-Asp-Lys-Phe-Ser-Glu-Phe, Tyr-Trp-Ser-Thr-Val-Lys-Asp-Lys-Phe, Tyr-Trp-Ser-Thr-Val-Lys-Asp-Lys-Phe-Ser-Glu-Phe, His-Thr-Asp-Leu-Ser-Gly-Lys-Val, His-Thr-Asp-Leu-Ser-Gly-Lys-Val-Phe-Val-Phe, Tyr-Val-Ile-Ile-Lys-Pro-Leu-Val-Trp-Val, Ala-Val-Arg-Gly-Ser-Pro-Ala-Ile-Asn, Glu-Val-Val-Phe-Thr-Ala-Asn-Asp-Ser-Gly-Pro-Arg-Arg-Tyr-Thr-Ile-Ala, Glu-Val-Val-Phe-Thr-Ala-Asn-Asp-Ser-Gly-Pro-Arg-Arg-Tyr-Thr-Ile-Ala-Ala, Phe-Thr-Ala-Asn-Asp-Ser-Gly-Pro-Arg-Arg-Tyr-Thr-Ile-Ala-Ala, Phe-Val-Glu-Gly-Ile-Tyr-Lys-Val-Glu, Leu-Leu-Ser-Pro-Tyr-Ser-Tyr-Ser-Thr-Thr-Ala, Leu-Ser-Pro-Tyr-Ser-Tyr-Ser-Thr-Thr-Ala, Thr-Ala-Asn-Asp-Ser-Gly-Pro-Arg-Arg-Tyr-Thr-Ile-Ala-Ala-Leu-Leu-Ser-Pro-Tyr-Ser-Tyr-Ser-Thr-Thr-Ala, Val-Val-Phe-Thr-Ala-Asn-Asp-Ser-Gly-Pro-Arg-Arg-Tyr-Thr-Ile-Ala-Ala, Phe-Lys-Asp-Ser-Ala-His-Gly-Phe, Phe-Ser-Ser-Pro-His-Gly-Lys-Asp-Leu-Leu-Phe-Lys-Asp-Ser-Ala-His-Gly-Phe, Asn-Gln-Ala-Gln-Glu-His-Phe-Gly-Lys-Asp-Lys-Ser-Lys-Glu-Phe-Gln-Leu, Tyr-Ala-Val-Ala-Val-Val-Lys-Lys-Asp, Tyr-Ala-Val-Ala-Val-Val-Lys-Lys-Asp-Ser, Tyr-Val-Thr-Ala-Ile-Arg-Asn-Leu-Arg-Glu, Ser-Lys-Glu-Asp-Pro-Gln-Thr-Phe-Tyr-Tyr-Ala-Val-Ala, Tyr-Gly-Ser-Lys-Glu-Asp-Pro-Gln-Thr-Phe, Ala-Tyr-Leu-Ala-Pro-Asn-Asn-Leu-Lys-Pro-Val-Val-Ala-Glu, Ala-Tyr-Leu-Ala-Pro-Asn-Asn-Leu-Lys-Pro-Val-Val-Ala-Glu-Phe, Ala-Tyr-Leu-Ala-Pro-Asn-Asn-Leu-Lys-Pro-Val-Val-Ala-Glu-Phe-Tyr-Gly-Ser-Lys-Glu-Asp, Asp-Ala-Gly-Leu-Val-Tyr-Asp-Ala-Tyr, Asp-Ala-Tyr-Leu-Ala-Pro-Asn-Asn-Leu-Lys-Pro-Val-Val-Ala-Glu-Phe, Tyr-Leu-Ala-Pro-Asn-Asn-Leu-Lys-Pro-Val-Val-Ala-Glu, Tyr-Leu-Ala-Pro-Asn-Asn-Leu-Lys-Pro-Val-Val-Ala-Glu-Phe, Tyr-Leu-Ala-Pro-Asn-Asn-Leu-Lys-Pro-Val-Val-Ala-Glu-Phe-Tyr-Gly-Ser-Lys-Glu-Asp, Ser-Ala-Leu-Thr-Ser-Asp-Asn-His-Gly-Ala-Thr-Tyr, Ser-Ala-Leu-Thr-Ser-Asp-Asn-His-Gly-Ala-Thr-Tyr-Ala-Phe-Ser-Gly-Thr-His-Tyr, Phe-Arg-Gln-Gly-His-Asn-Ser-Val-Phe, Val-Ala-Glu-Lys-Ala-Leu-Gln-Glu-Tyr-Arg-Lys-Lys-His-Arg-Glu-Glu, Phe-Thr-Arg-Glu-Pro-Gln-Asp-Thr-Tyr-His-Tyr-Leu-Pro, Arg-Pro-His-Phe-Phe-Phe-Pro, Arg-Pro-His-Phe-Phe-Phe-Pro-Lys-Ser-Arg-Ile-Val, Phe-Leu-Arg-Asp-Thr-Lys-Thr-Leu, Val-Ile-Glu-Pro-Gln-Gly-Leu-Arg-Phe, Phe-Val-Thr-Pro-Leu-Thr-Ser-Met, Val-His-Leu-Thr-Pro-Glu-Glu-Lys-Ser, Trp-Gly-Lys-Val-Asn-Val-Asp-Glu-Val-Gly-Gly-Glu-Ala-Leu-Gly-Arg-Leu, Arg-Gly-Pro-Leu-Val-Lys-Tyr-Ser-Ser-Asp-Tyr, Ala-Ser-Glu-Gly-Gly-Phe-Thr-Ala-Thr-Gly-Gln-Arg (Table 1).

Grade of the fold increase of the quantitative peptides correlates positively with their predictive significance for AIS diagnostics. Therefore, we also define a second serum peptide pattern that is a list of 32 peptides selected from 100 quantitative peptides that can be used as a separate independent panel of markers for diagnostics of AIS and differentiation of AIS from ICH when their levels are increased 30 folds or more in sera of AIS patients as compared with individuals without stroke and in patients with ICH. These peptides are:
Val-Ser-Glu-Thr-Glu-Ser-Arg-Gly-Ser-Glu-Ser-Gly-Ile-Phe-Thr-Asn-Thr-Lys, His-Arg-His-Pro-Asp-Glu-Ala-Ala-Phe-Phe-Asp-Thr-Ala-Ser-Thr, Phe-Phe-Asp-Thr-Ala-Ser-Thr-Gly-Lys, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Ala-Ala-Thr-Val-Gly-Ser-Leu-Ala-Gly-Gln-Pro-Leu-Gln-Glu-Arg, Pro-Gly-Ser-Thr-Gly-Thr-Trp-Asn-Pro-Gly-Ser-Ser-Glu-Arg, His-Asp-Trp-Gly-His-Glu-Lys-Gln-Arg, His-Pro-Asp-Glu-Ala-Ala-Phe-Phe-Asp-Thr-Ala-Ser-Thr-Gly-Lys, Ser-Ser-His-His-Pro-Gly-Ile-Ala-Glu-Phe-Pro-Ser-Arg, Glu-Ser-Ser-Ser-His-His-Pro-Gly-Ile-Ala-Glu-Phe-Pro-Ser-Arg, Ser-Gly-Asn-Val-Ser-Pro-Gly-Thr-Arg-Arg-Glu-Tyr-His-Thr-Glu-Lys, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Leu-Asp-Asp-Asp-Leu-Glu-His-Gln-Gly-Gly-His-Val-Leu-Asp-His-Gly-His, Phe-Val-Ser-Glu-Thr-Glu-Ser-Arg-Gly-Ser-Glu-Ser-Gly-Ile-Phe-Thr-Asn-Thr, Arg-Glu-Tyr-His-Thr-Glu-Lys-Leu-Val-Thr-Ser, Gly-Ser-Ser-Gly-Thr-Gly-Gly-Thr-Ala-Thr-Trp-Lys, Gly-Ser-Trp-Asn-Ser-Gly-Ser-Ser-Gly-Pro-Gly-Ser-Thr-Gly-Asn-Arg, Glu-Leu-Glu-Arg-Pro-Gly-Gly-Asn-Glu-Ile, Leu-Gly-Glu-Phe-Val-Ser-Glu-Thr-Glu-Ser-Arg, Glu-Leu-Glu-Arg-Pro-Gly-Gly-Asn-Glu, Asp-Glu-Ala-Gly-Ser-Glu-Ala-Asp-His-Glu-Gly-Thr-His-Ser-Thr, Asp-Glu-Ala-Ala-Phe-Phe-Asp-Thr-Ala-Ser-Thr-Gly-Lys, Met-Ala-Asp-Glu-Ala-Gly-Ser-Glu-Ala-Asp-His-Glu-Gly-Thr-His-Ser-Thr-Lys, Arg-Glu-Glu-Ala-Pro-Ser-Leu-Arg, Glu-Glu-Val-Ser-Gly-Asn-Val-Ser-Pro-Gly-Thr, Tyr-Gln-Glu-Pro-Val-Leu-Gly-Pro-Val, Ser-Glu-Ser-Gly-Ile-Phe-Thr-Asn-Thr-Lys, His-Arg-His-Pro-Asp-Glu-Ala-Ala-Phe-Phe-Asp-Thr-Ala (Table 2).

In addition, we define a third serum peptide pattern that is a list of 24 peptides selected from 100 quantitative peptides that can be used as a separate independent panel of markers for diagnostics of AIS and differentiation of AIS from ICH when their levels are increased 50 folds or more in sera of AIS patients as compared with individuals without stroke and in patients with ICH. These peptides are:
Val-Ser-Glu-Thr-Glu-Ser-Arg-Gly-Ser-Glu-Ser-Gly-Ile-Phe-Thr-Asn-Thr-Lys, Phe-Phe-Asp-Thr-Ala-Ser-Thr-Gly-Lys, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Pro-Gly-Ser-Thr-Gly-Thr-Trp-Asn-Pro-Gly-Ser-Ser-Glu-Arg, His-Pro-Asp-Glu-Ala-Ala-Phe-Phe-Asp-Thr-Ala-Ser-Thr-Gly-Lys, Glu-Ser-Ser-Ser-His-His-Pro-Gly-Ile-Ala-Glu-Phe-Pro-Ser-Arg, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Leu-Asp-Asp-Asp-Leu-Glu-His-Gln-Gly-Gly-His-Val-Leu-Asp-His-Gly-His, Arg-Glu-Tyr-His-Thr-Glu-Lys-Leu-Val-Thr-Ser, Gly-Ser-Ser-Gly-Thr-Gly-Gly-Thr-Ala-Thr-Trp-Lys, Gly-Ser-Trp-Asn-Ser-Gly-Ser-Ser-Gly-Pro-Gly-Ser-Thr-Gly-Asn-Arg, Glu-Leu-Glu-Arg-Pro-Gly-Gly-Asn-Glu-Ile, Leu-Gly-Glu-Phe-Val-Ser-Glu-Thr-Glu-Ser-Arg, Glu-Leu-Glu-Arg-Pro-Gly-Gly-Asn-Glu, Asp-Glu-Ala-Ala-Phe-Phe-Asp-Thr-Ala-Ser-Thr-Gly-Lys, Arg-Glu-Glu-Ala-Pro-Ser-Leu-Arg, Glu-Glu-Val-Ser-Gly-Asn-Val-Ser-Pro-Gly-Thr, Tyr-Gln-Glu-Pro-Val-Leu-Gly-Pro-Val, Ser-Glu-Ser-Gly-Ile-Phe-Thr-Asn-Thr-Lys, His-Arg-His-Pro-Asp-Glu-Ala-Ala-Phe- Phe-Asp-Thr-Ala. (Table 3).

Next, we define a fourth serum peptide pattern that is a list of 16 peptides selected from 100 quantitative peptides that can be used as a separate independent panel of markers for diagnostics of AIS and differentiation of AIS from ICH when their levels are increased 100 folds or more in sera of AIS patients as compared with individuals without stroke and in patients with ICH. These peptides are:
Val-Ser-Glu-Thr-Glu-Ser-Arg-Gly-Ser-Glu-Ser-Gly-Ile-Phe-Thr-Asn-Thr-Lys, Phe-Phe-Asp-Thr-Ala-Ser-Thr-Gly-Lys, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Pro-Gly-Ser-Thr-Gly-Thr-Trp-Asn-Pro-Gly-Ser-Ser-Glu-Arg, Glu-Ser-Ser-Ser-His-His-Pro-Gly-Ile-Ala-Glu-Phe-Pro-Ser-Arg, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Arg-Glu-Tyr-His-Thr-Glu-Lys-Leu-Val-Thr-Ser, Gly-Ser-Ser-Gly-Thr-Gly-Gly-Thr-Ala-Thr-Trp-Lys, Glu-Leu-Glu-Arg-Pro-Gly-Gly-Asn-Glu-Ile, Arg-Glu-Glu-Ala-Pro-Ser-Leu-Arg, Glu-Glu-Val-Ser-Gly-Asn-Val-Ser-Pro-Gly-Thr, Ser-Glu-Ser-Gly-Ile-Phe-Thr-Asn-Thr-Lys. (Table 4).

Finally, we define a fifth serum peptide pattern that is a list of 1 peptide selected from 100 quantitative peptides that can be used as a separate independent marker for diagnostics of AIS and differentiation of AIS from ICH when its level is increased 1000 folds or more in sera of AIS patients as compared with individuals without stroke and in patients with ICH. This peptide is:
Val-Ser-Glu-Thr-Glu-Ser-Arg-Gly-Ser-Glu-Ser-Gly-Ile-Phe-Thr-Asn-Thr-Lys. (Table 5).

The serum peptide patterns for diagnostics of AIS are based on qualitative and quantitative analysis of serum peptides with tandem mass spectrometry. However, the method for peptide identification and quantification is not the limitation of the invention. The same peptide patterns can be detected and measured with different techniques for peptide identification and used for AIS diagnostics and monitoring. All 5 serum peptide patters were selected during the research - approach to provide the diagnostic methods for AIS. The peptides listed in table 1 (all peptides, 154) were checked in the serum samples - confirmed during provision of the invention - approach with the adjusted p value ≤ 0.05 confirming statistically significance.

For all 100 quantitative peptides a MS/MS (tandem mass spectrometry) relative signaling intensity measurement is defined as fold change. The fold change is obtained after comparison of the signal intensity of quantitative peptides in serum of patients with AIS with signal intensity of quantitative peptides in serum of matched control individuals that are individuals without stroke and patients with ICH. Fold-increase values provided in the table 6 for peptides 1-100 set how many times more the peptide MS/MS (tandem mass spectrometry) signaling intensities present in the serum of AIS patients was higher than in serum samples from individuals without stroke and the patients with the ICH. The serum peptide patterns for diagnostics of acute ischemic stroke (AIS) in humans and differential diagnosis from ICH stroke that are the matter of the current invention were defined and analyzed with tandem mass spectrometry. However, the method of peptide identification and quantification is not the limitation of the invention and can be different than tandem mass spectrometry. In the example provided in this patent application serum samples designated for peptide identification and quantification were treated as follow:
I. Tested serum sample preparation according to which the following steps are performed:
   a) enrichment of peptides in the analyzed serum sample by incubation of the sample with a citrate-phosphate buffer with pH 3.1-3.6 in water with a purity ≥ 99.9%, wherein the buffer and serum volume ratio is 29:1 and the buffer and serum are mixed at a temperature of range 4 to 8 °C to dissociate peptides from highly concentrated serum proteins;
   b) removing of proteins equal or bigger than 15kDa from peptide enriched and simultaneously purification of peptides using hydrophilic-lipophilic balanced columns to obtain peptides smaller than 15 kDa;
   c) washing the hydrophilic-lipophilic balanced columns with high-grade water purity ≥ 99.9% in order to remove the unbound and low-binding peptides from the columns;
   d) eluting of serum peptides bound to the hydrophilic-lipophilic balanced columns by using water/80% methanol/0.2% formic acid volume per volume (v/v);
   e) filtrating of the sample through 3kDa molecular weight filters to collect peptides smaller than 3kDa;
   f) providing lyophilization of the obtained peptides by drying the eluted peptides through lyophilization for tandem mass spectrometry analysis.

With the applied technique the diagnostic peptides used for detection of AIS were separated and purified in a single step highly concentrated serum proteins > 16 to 170 kilodaltons (kDa) (e.g. albumin, globulin, transferrin, haptoglobin, alpha-1-antitrypsin, etc.)

Then, peptides were analyzed for amino acid sequences with a tandem mass spectrometry.

The data analysis workflow for quantitative analysis of the peptides sequenced with a tandem mass spectrometry comprised the following steps within the Skyline, MSStats pipeline:
a). the MS/MS DDA (data-dependent acquisition) data were searched in more than 1 search engine strategy, resulting in creation of a comprehensive spectral library;
b). the comprehensive spectral library was used to extract quantitative peptidomics data (independent MS/MS data; DIA);
c). extraction of the product ion chromatograms and integration of the product ion peak group areas (area under the curve, AUC);
d). the false discovery rate (FDR) of quantitative extraction/analysis was controlled by determining peak group's FDR values in the mProphet module;
e). performing the inference of product ion peak areas to peptides and their summed peak area statistics in MSstats module was done in a peptide-centric view;
f). further downstream analyses such as graphical data visualization including volcano plots, dendrograms, heatmaps, functional stroke serum peptide enrichment maps were done using quantitative matrix from MSstats. Thus, the downstream analyses is not the limitation of the method. The serum peptide diagnostic patters for AIS are listed in tables 1-5.

Table 1. The serum peptide pattern 1 for diagnostics of AIS and differential diagnosis of AIS from ICH. The sequences of diagnostic peptides, as well as the source protein identifier (*) for each peptide is given. The source protein for the given peptide is the protein which contains in its structure the given peptide. The peptides derive from the source proteins that underwent various types of degradations. The fold change (***) refers only to quantitative peptides and it shows how many times more signaling intensities present in the sera of AIS patients were higher than signaling intensities in serum samples from individuals without stroke and the patients with ICH.

One to 100 quantitative peptides (serial numbers 1-100) with fold change equal or higher than 2 (fold change ≥ 2) have diagnostic value and confirms the diagnosis of AIS. In case of qualitative peptides- detection of 54 of qualitative peptides (serial numbers 101-154) the peptide confirms the diagnosis of AIS.

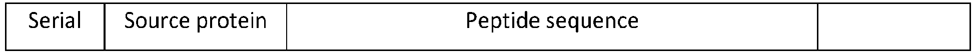

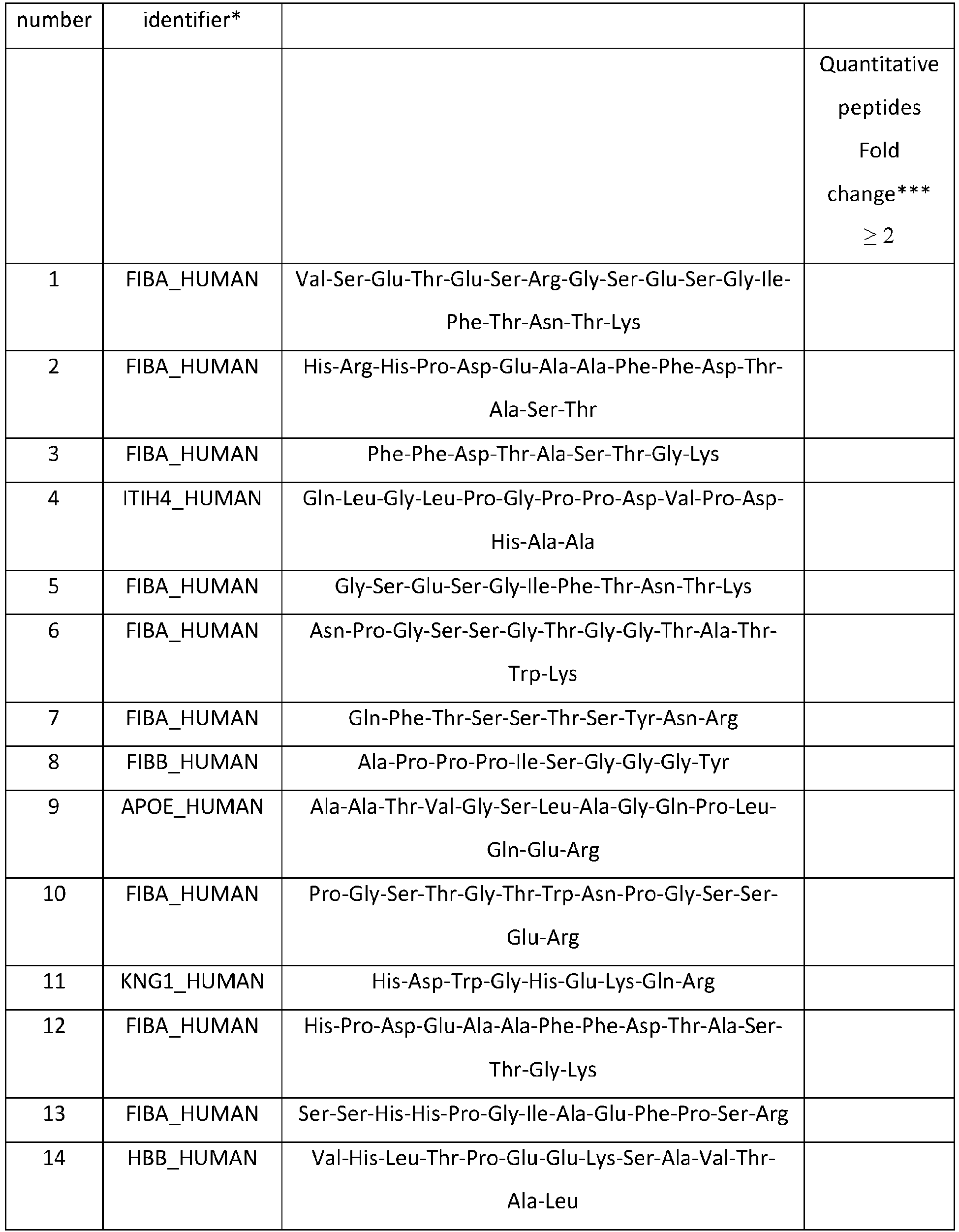

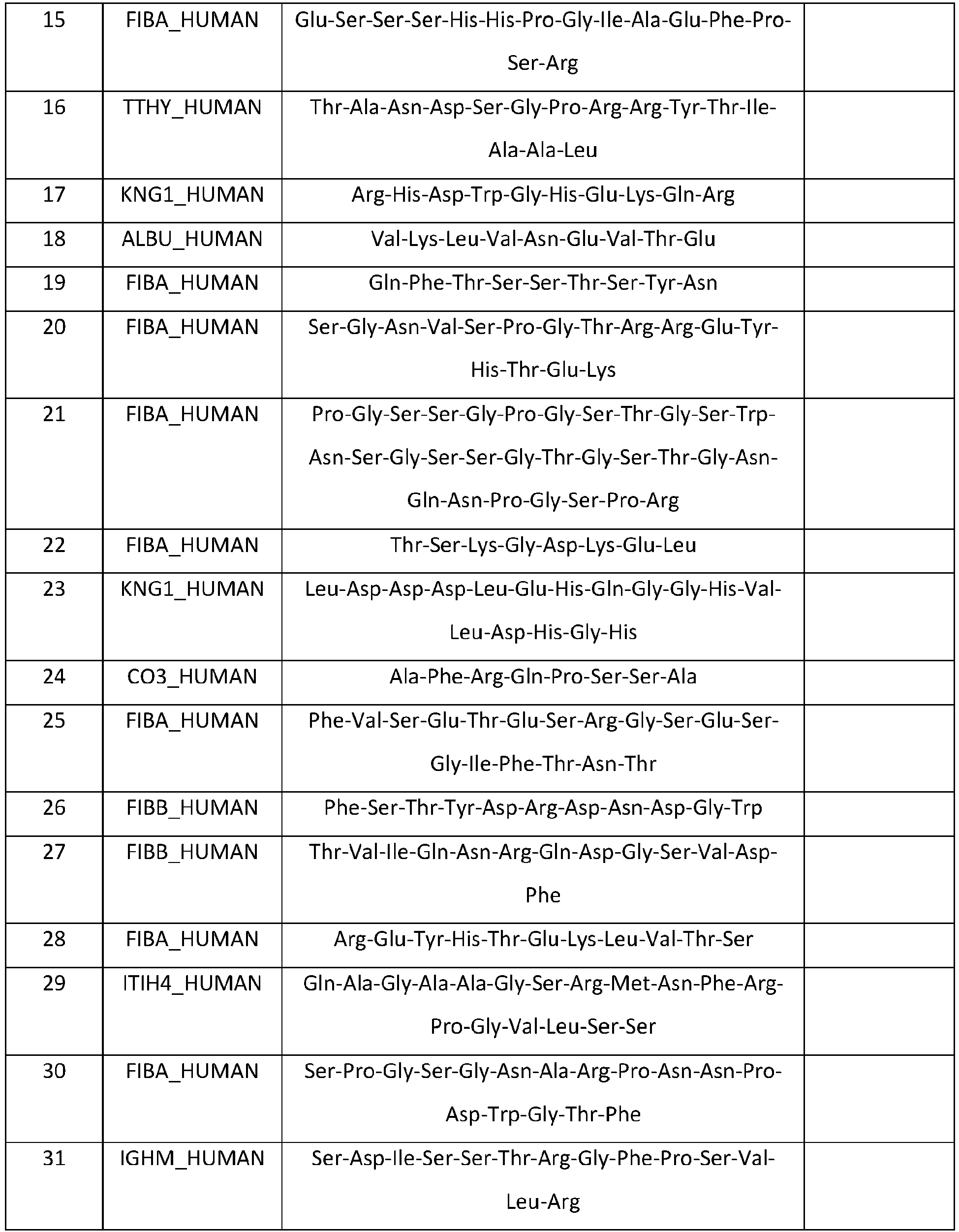

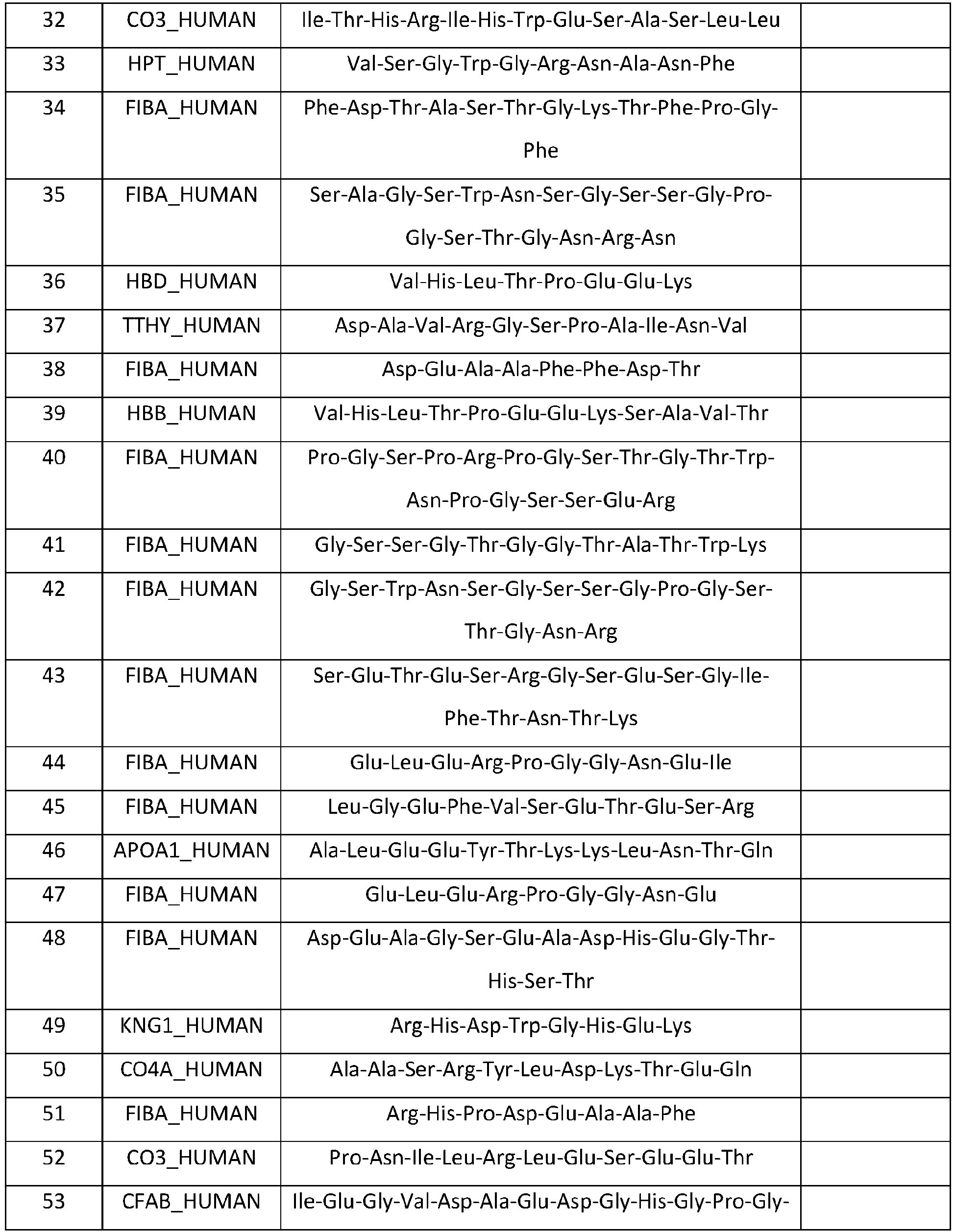

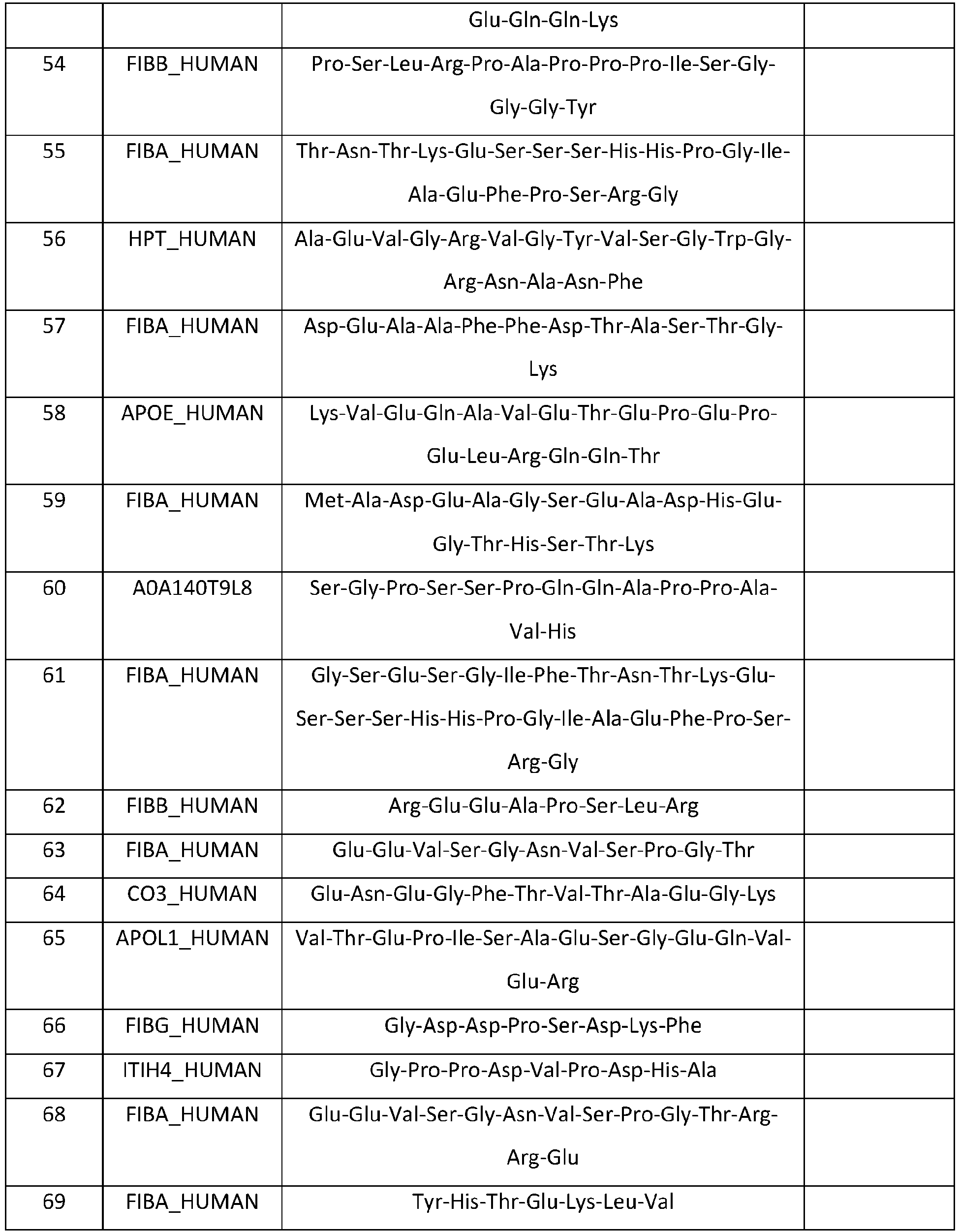

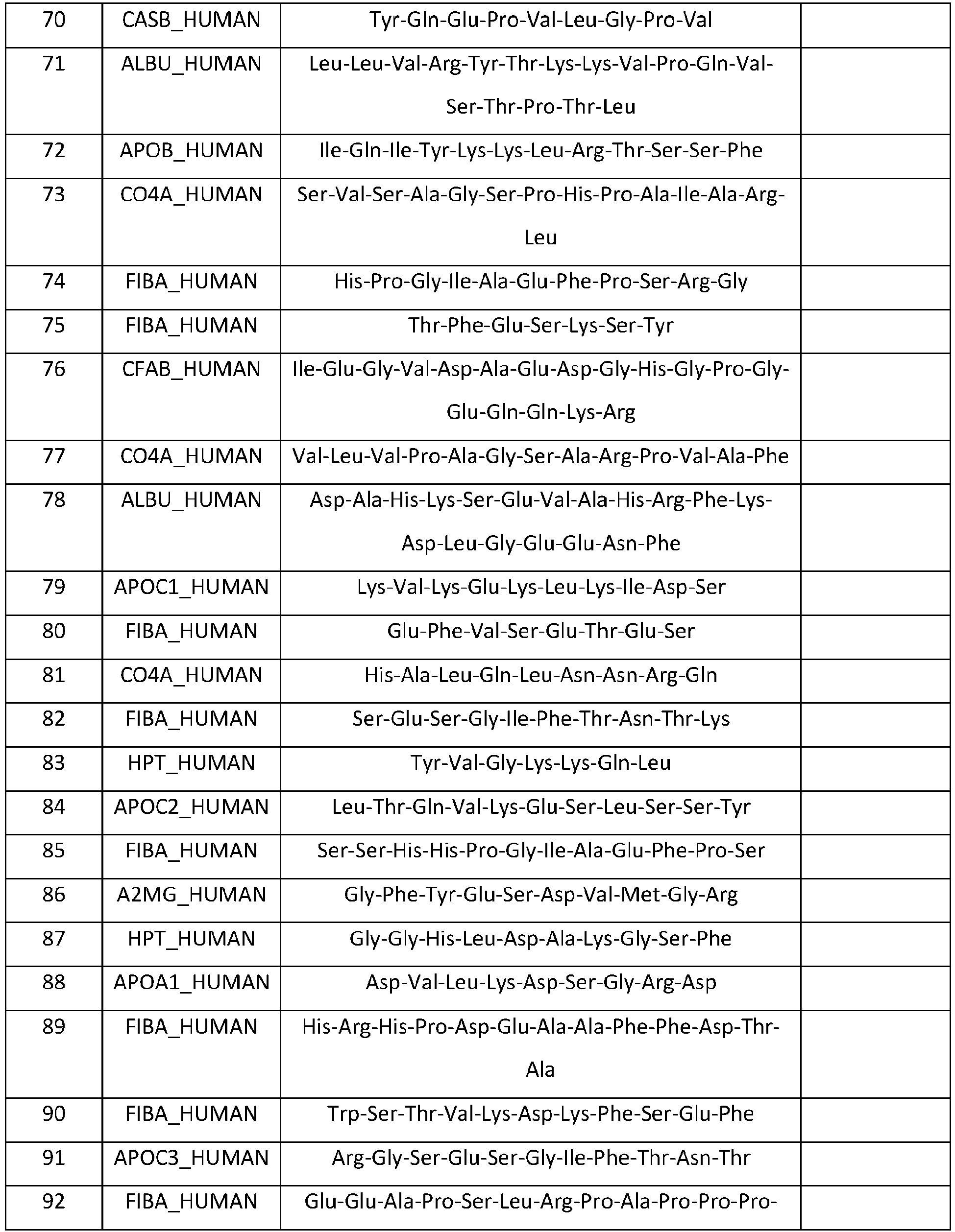

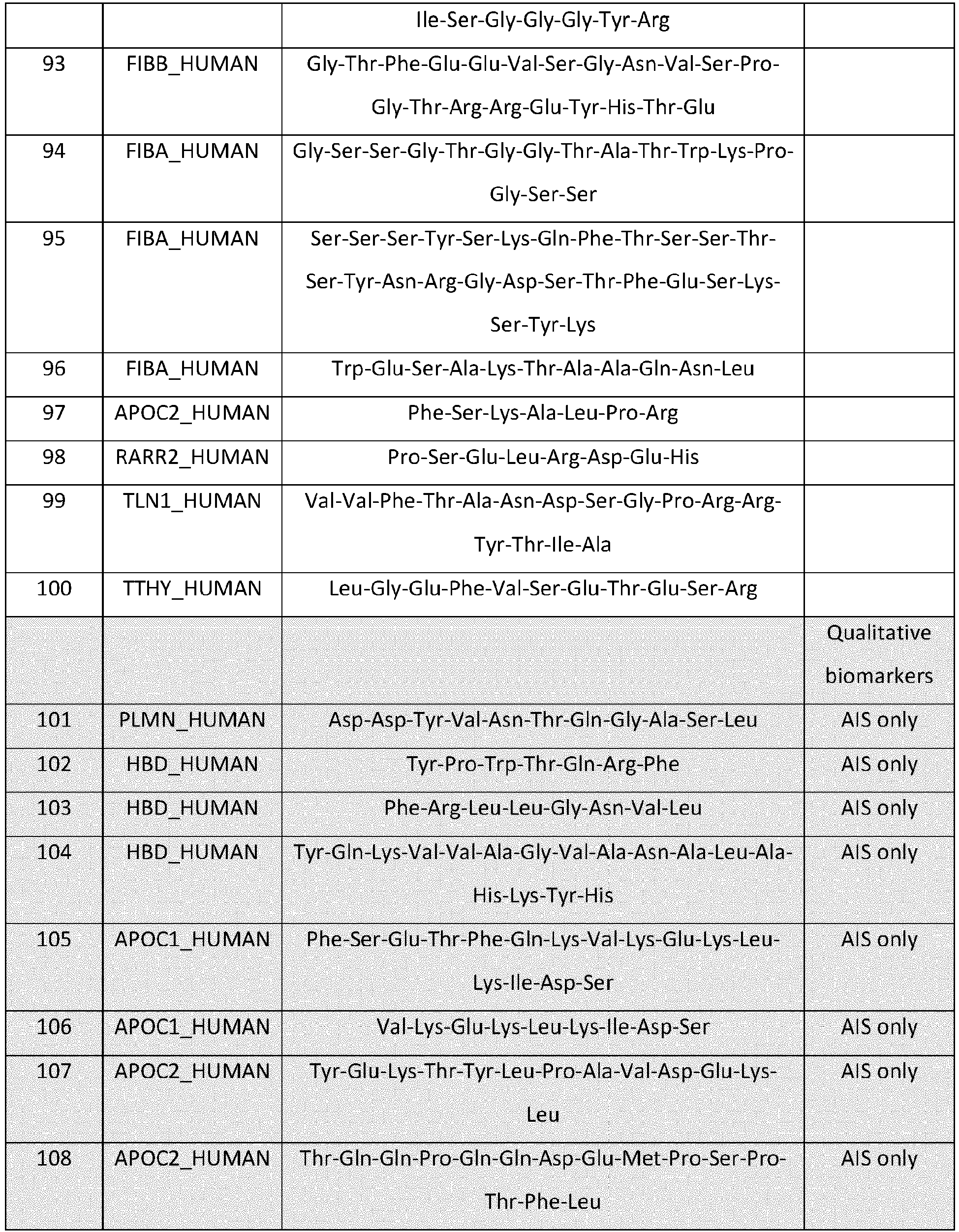

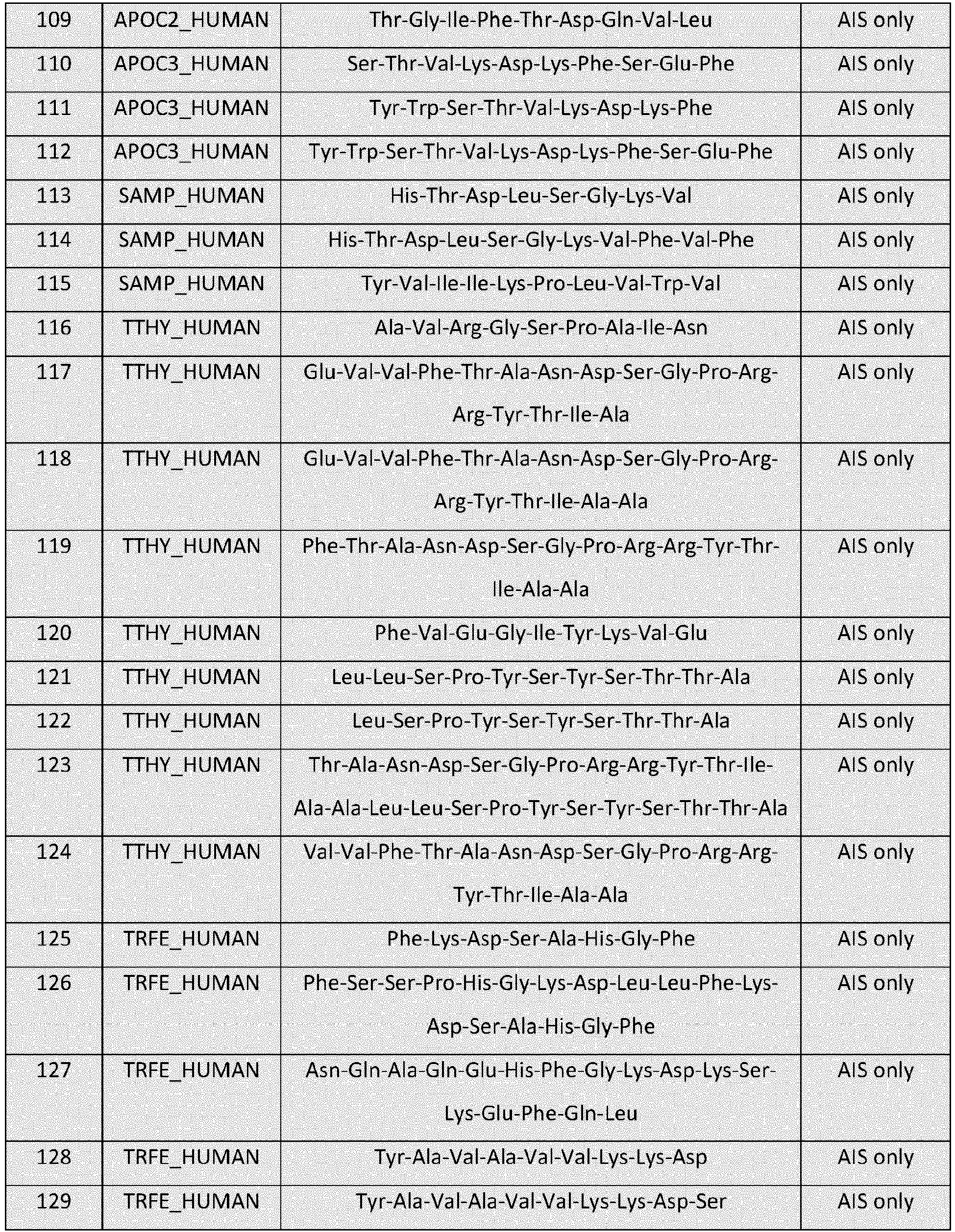

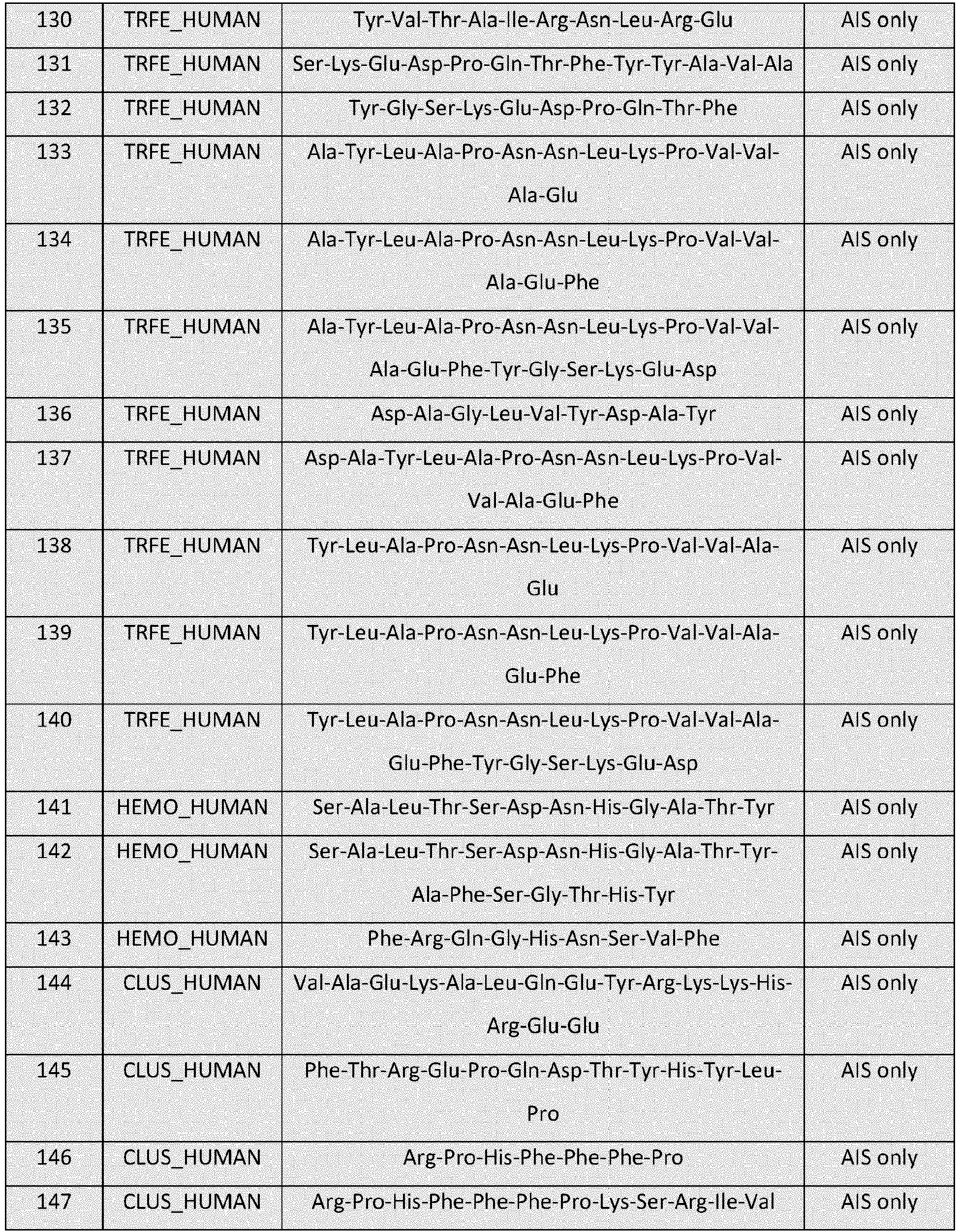

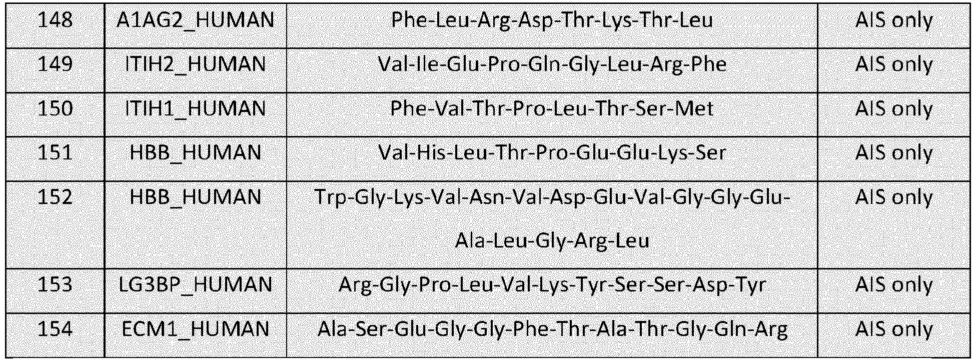

**Table 2. The serum peptide pattern 2 for diagnostics of AIS and differential diagnosis of AIS from ICH. The sequences of diagnostic peptides, as well as the source protein identifier (*) for each peptide is given. The source protein for the given peptide is the protein which contains in its structure the given peptide. The peptides derive from the source proteins that underwent various types of degradations. The fold change (***) refers only to quantitative peptides and it shows how many times more signaling intensities present in the sera of AIS patients were higher than signaling intensities in serum samples from individuals without stroke and the patients with ICH. The listed quantitative peptides with fold change equal or higher than 30 (fold change ≥ 30) have diagnostic value and confirms the diagnosis of AIS.**

| Serial number | Source protein identifier* | Peptide sequence | |
|---|---|---|---|
| | | | Quantitative peptides Fold change*** ≥ 30 |
| 1 | FIBA_HUMAN | Val-Ser-Glu-Thr-Glu-Ser-Arg-Gly-Ser-Glu-Ser-Gly-Ile- Phe-Thr-Asn-Thr-Lys | |
| 2 | FIBA_HUMAN | His-Arg-His-Pro-Asp-Glu-Ala-Ala-Phe-Phe-Asp-Thr-Ala- Ser-Thr | |
| 3 | FIBA_HUMAN | Phe-Phe-Asp-Thr-Ala-Ser-Thr-Gly-Lys | |
| 4 | FIBA_HUMAN | Gly-Ser-Glu-Ser-Gly-Ile-Phe-Thr-Asn-Thr-Lys | |
| 5 | FIBA_HUMAN | Asn-Pro-Gly-Ser-Ser-Gly-Thr-Gly-Gly-Thr-Ala-Thr-Trp-Lys | |
| 6 | FIBA_HUMAN | Gln-Phe-Thr-Ser-Ser-Thr-Ser-Tyr-Asn-Arg | |
| 7 | FIBB_HUMAN | Ala-Pro-Pro-Pro-Ile-Ser-Gly-Gly-Gly-Tyr | |
| 8 | APOE_HUMAN | Ala-Ala-Thr-Val-Gly-Ser-Leu-Ala-Gly-Gln-Pro-Leu-Gln- Glu-Arg | |
| 9 | FIBA_HUMAN | Pro-Gly-Ser-Thr-Gly-Thr-Trp-Asn-Pro-Gly-Ser-Ser-Glu- Arg | |
| 10 | KNG1_HUMAN | His-Asp-Trp-Gly-His-Glu-Lys-Gln-Arg | |
| 11 | FIBA_HUMAN | His-Pro-Asp-Glu-Ala-Ala-Phe-Phe-Asp-Thr-Ala-Ser-Thr- Gly-Lys | |
| 12 | FIBA_HUMAN | Ser-Ser-His-His-Pro-Gly-Ile-Ala-Glu-Phe-Pro-Ser-Arg | |
| 13 | FIBA_HUMAN | Glu-Ser-Ser-Ser-His-His-Pro-Gly-Ile-Ala-Glu-Phe-Pro-Ser- Arg | |
| 14 | FIBA_HUMAN | Ser-Gly-Asn-Val-Ser-Pro-Gly-Thr-Arg-Arg-Glu-Tyr-His- Thr-Glu-Lys | |
| 15 | FIBA_HUMAN | Pro-Gly-Ser-Ser-Gly-Pro-Gly-Ser-Thr-Gly-Ser-Trp-Asn- Ser-Gly-Ser-Ser-Gly-Thr-Gly-Ser-Thr-Gly-Asn-Gln-Asn- Pro-Gly-Ser-Pro-Arg | |
| 16 | FIBA_HUMAN | Thr-Ser-Lys-Gly-Asp-Lys-Glu-Leu | |
| 17 | KNG1_HUMAN | Leu-Asp-Asp-Asp-Leu-Glu-His-Gln-Gly-Gly-His-Val-Leu- Asp-His-Gly-His | |
| 18 | FIBA_HUMAN | Phe-Val-Ser-Glu-Thr-Glu-Ser-Arg-Gly-Ser-Glu-Ser-Gly- Ile-Phe-Thr-Asn-Thr | |
| 19 | FIBA_HUMAN | Arg-Glu-Tyr-His-Thr-Glu-Lys-Leu-Val-Thr-Ser | |
| 20 | FIBA_HUMAN | Gly-Ser-Ser-Gly-Thr-Gly-Gly-Thr-Ala-Thr-Trp-Lys | |
| 21 | FIBA_HUMAN | Gly-Ser-Trp-Asn-Ser-Gly-Ser-Ser-Gly-Pro-Gly-Ser-Thr- Gly-Asn-Arg | |
| 22 | FIBA_HUMAN | Glu-Leu-Glu-Arg-Pro-Gly-Gly-Asn-Glu-Ile | |
| 23 | FIBA_HUMAN | Leu-Gly-Glu-Phe-Val-Ser-Glu-Thr-Glu-Ser-Arg | |
| 24 | FIBA_HUMAN | Glu-Leu-Glu-Arg-Pro-Gly-Gly-Asn-Glu | |
| 25 | FIBA_HUMAN | Asp-Glu-Ala-Gly-Ser-Glu-Ala-Asp-His-Glu-Gly-Thr-His- Ser-Thr | |
| 26 | FIBA_HUMAN | Asp-Glu-Ala-Ala-Phe-Phe-Asp-Thr-Ala-Ser-Thr-Gly-Lys | |
| 27 | FIBA_HUMAN | Met-Ala-Asp-Glu-Ala-Gly-Ser-Glu-Ala-Asp-His-Glu-Gly- Thr-His-Ser-Thr-Lys | |
| 28 | FIBB_HUMAN | Arg-Glu-Glu-Ala-Pro-Ser-Leu-Arg | |
| 29 | FIBA_HUMAN | Glu-Glu-Val-Ser-Gly-Asn-Val-Ser-Pro-Gly-Thr | |
| 30 | CASB_HUMAN | Tyr-Gln-Glu-Pro-Val-Leu-Gly-Pro-Val | |
| 31 | FIBA_HUMAN | Ser-Glu-Ser-Gly-Ile-Phe-Thr-Asn-Thr-Lys | |
| 32 | FIBA_HUMAN | His-Arg-His-Pro-Asp-Glu-Ala-Ala-Phe-Phe-Asp-Thr-Ala | |

**Table 3. The serum peptide pattern 3 for diagnostics of AIS and differential diagnosis of AIS from ICH. The sequences of diagnostic peptides, as well as the source protein identifier (*) for each peptide is given. The source protein for the given peptide is the protein which contains in its structure the given peptide. The peptides derive from the source proteins that underwent various types of degradations. The fold change (***) refers only to quantitative peptides and it shows how many times more signaling intensities present in the sera of AIS patients were higher than signaling intensities in serum samples from individuals without stroke and the patients with ICH. The listed quantitative peptides with fold change equal or higher than 50 (fold change ≥ 50) have diagnostic value and confirms the diagnosis of AIS.**

| Serial number | Source protein identifier* | Peptide sequence | |
|---|---|---|---|
| | | | Quantitative peptides Fold change*** ≥ 50 |
| 1 | FIBA_HUMAN | Val-Ser-Glu-Thr-Glu-Ser-Arg-Gly-Ser-Glu-Ser-Gly-Ile- Phe-Thr-Asn-Thr-Lys | |
| 2 | FIBA_HUMAN | Phe-Phe-Asp-Thr-Ala-Ser-Thr-Gly-Lys | |
| 3 | FIBA_HUMAN | Gly-Ser-Glu-Ser-Gly-Ile-Phe-Thr-Asn-Thr-Lys | |
| 4 | FIBA_HUMAN | Asn-Pro-Gly-Ser-Ser-Gly-Thr-Gly-Gly-Thr-Ala-Thr-Trp-Lys | |
| 5 | FIBA_HUMAN | Gln-Phe-Thr-Ser-Ser-Thr-Ser-Tyr-Asn-Arg | |
| 6 | FIBB_HUMAN | Ala-Pro-Pro-Pro-Ile-Ser-Gly-Gly-Gly-Tyr | |
| 7 | FIBA_HUMAN | Pro-Gly-Ser-Thr-Gly-Thr-Trp-Asn-Pro-Gly-Ser-Ser-Glu- Arg | |
| 8 | FIBA_HUMAN | His-Pro-Asp-Glu-Ala-Ala-Phe-Phe-Asp-Thr-Ala-Ser-Thr- Gly-Lys | |
| 9 | FIBA_HUMAN | Glu-Ser-Ser-Ser-His-His-Pro-Gly-Ile-Ala-Glu-Phe-Pro-Ser- Arg | |
| 10 | FIBA_HUMAN | Pro-Gly-Ser-Ser-Gly-Pro-Gly-Ser-Thr-Gly-Ser-Trp-Asn- Ser-Gly-Ser-Ser-Gly-Thr-Gly-Ser-Thr-Gly-Asn-Gln-Asn- Pro-Gly-Ser-Pro-Arg | |
| 11 | FIBA_HUMAN | Thr-Ser-Lys-Gly-Asp-Lys-Glu-Leu | |
| 12 | KNG1_HUMAN | Leu-Asp-Asp-Asp-Leu-Glu-His-Gln-Gly-Gly-His-Val-Leu- Asp-His-Gly-His | |
| 13 | FIBA_HUMAN | Arg-Glu-Tyr-His-Thr-Glu-Lys-Leu-Val-Thr-Ser | |
| 14 | FIBA_HUMAN | Gly-Ser-Ser-Gly-Thr-Gly-Gly-Thr-Ala-Thr-Trp-Lys | |
| 15 | FIBA_HUMAN | Gly-Ser-Trp-Asn-Ser-Gly-Ser-Ser-Gly-Pro-Gly-Ser-Thr- Gly-Asn-Arg | |
| 16 | FIBA_HUMAN | Glu-Leu-Glu-Arg-Pro-Gly-Gly-Asn-Glu-Ile | |
| 17 | FIBA_HUMAN | Leu-Gly-Glu-Phe-Val-Ser-Glu-Thr-Glu-Ser-Arg | |
| 18 | FIBA_HUMAN | Glu-Leu-Glu-Arg-Pro-Gly-Gly-Asn-Glu | |
| 19 | FIBA_HUMAN | Asp-Glu-Ala-Ala-Phe-Phe-Asp-Thr-Ala-Ser-Thr-Gly-Lys | |
| 20 | FIBB_HUMAN | Arg-Glu-Glu-Ala-Pro-Ser-Leu-Arg | |
| 21 | FIBA_HUMAN | Glu-Glu-Val-Ser-Gly-Asn-Val-Ser-Pro-Gly-Thr | |
| 22 | CASB_HUMAN | Tyr-Gln-Glu-Pro-Val-Leu-Gly-Pro-Val | |
| 23 | FIBA_HUMAN | Ser-Glu-Ser-Gly-Ile-Phe-Thr-Asn-Thr-Lys | |
| 24 | FIBA_HUMAN | His-Arg-His-Pro-Asp-Glu-Ala-Ala-Phe-Phe-Asp-Thr-Ala | |

**Table 4. The serum peptide pattern 4 for diagnostics of AIS and differential diagnosis of AIS from ICH. The sequences of diagnostic peptides, as well as the source protein identifier (*) for each peptide is given. The source protein for the given peptide is the protein which contains in its structure the given peptide. The peptides derive from the source proteins that underwent various types of degradations. The fold change (***) refers only to quantitative peptides and it shows how many times more signaling intensities present in the sera of AIS patients were higher than signaling intensities in serum samples from individuals without stroke and the patients with ICH. The listed quantitative peptides with fold change equal or higher than 100 (fold change ≥ 100) have diagnostic value and confirms the diagnosis of AIS.**

| Serial number | Source protein identifier* | Peptide sequence | |
|---|---|---|---|
| | | | Quantitative peptides Fold change*** ≥ 100 |
| 1 | FIBA_HUMAN | Val-Ser-Glu-Thr-Glu-Ser-Arg-Gly-Ser-Glu-Ser-Gly-Ile- Phe-Thr-Asn-Thr-Lys | |
| 2 | FIBA_HUMAN | Phe-Phe-Asp-Thr-Ala-Ser-Thr-Gly-Lys | |
| 3 | FIBA_HUMAN | Gly-Ser-Glu-Ser-Gly-Ile-Phe-Thr-Asn-Thr-Lys | |
| 4 | FIBA_HUMAN | Asn-Pro-Gly-Ser-Ser-Gly-Thr-Gly-Gly-Thr-Ala-Thr-Trp-Lys | |
| 5 | FIBA_HUMAN | Gln-Phe-Thr-Ser-Ser-Thr-Ser-Tyr-Asn-Arg | |
| 6 | FIBB_HUMAN | Ala-Pro-Pro-Pro-Ile-Ser-Gly-Gly-Gly-Tyr | |
| 7 | FIBA_HUMAN | Pro-Gly-Ser-Thr-Gly-Thr-Trp-Asn-Pro-Gly-Ser-Ser-Glu- Arg | |
| 8 | FIBA_HUMAN | Glu-Ser-Ser-Ser-His-His-Pro-Gly-Ile-Ala-Glu-Phe-Pro-Ser- Arg | |
| 9 | FIBA_HUMAN | Pro-Gly-Ser-Ser-Gly-Pro-Gly-Ser-Thr-Gly-Ser-Trp-Asn- Ser-Gly-Ser-Ser-Gly-Thr-Gly-Ser-Thr-Gly-Asn-Gln-Asn- Pro-Gly-Ser-Pro-Arg | |
| 10 | FIBA_HUMAN | Thr-Ser-Lys-Gly-Asp-Lys-Glu-Leu | |
| 11 | FIBA_HUMAN | Arg-Glu-Tyr-His-Thr-Glu-Lys-Leu-Val-Thr-Ser | |
| 12 | FIBA_HUMAN | Gly-Ser-Ser-Gly-Thr-Gly-Gly-Thr-Ala-Thr-Trp-Lys | |
| 13 | FIBA_HUMAN | Glu-Leu-Glu-Arg-Pro-Gly-Gly-Asn-Glu-Ile | |
| 14 | FIBB_HUMAN | Arg-Glu-Glu-Ala-Pro-Ser-Leu-Arg | |
| 15 | FIBA_HUMAN | Glu-Glu-Val-Ser-Gly-Asn-Val-Ser-Pro-Gly-Thr | |
| 16 | FIBA_HUMAN | Ser-Glu-Ser-Gly-Ile-Phe-Thr-Asn-Thr-Lys | |

**Table 5. The serum peptide pattern 5 for diagnostics of AIS and differential diagnosis of AIS from ICH. The sequences of diagnostic peptide, as well as the source protein identifier (*) for each peptide is given. The source protein for the given peptide is the protein which contains in its structure the given peptide. The peptide derive from the source protein that underwent various types of degradations. The fold change (***) refers only to quantitative peptides and it shows how many times more signaling intensities present in the sera of AIS patients were higher than signaling intensities in serum samples from individuals without stroke and the patients with ICH. The listed quantitative peptide with fold change equal or higher than 1000 (fold change ≥ 1000) have diagnostic value and confirms the diagnosis of AIS.**

| Serial number | Source protein identifier* | Peptide sequence | |
|---|---|---|---|
| | | | Quantitative peptides Fold change*** ≥ 1000 |
| 1 | FIBA_HUMAN | Val-Ser-Glu-Thr-Glu-Ser-Arg-Gly-Ser-Glu-Ser-Gly-Ile- Phe-Thr-Asn-Thr-Lys | |

Essence of the invention in detailed and the study description - approach leading to provision of the invention.

The invention differs from available approaches as we elaborated 5 complexed serum peptide patters for diagnostics of AIS and differentiation it from ICH. The first serum peptide pattern comprises of 100 quantitative and 54 qualitative peptides (Table 1), the second serum peptide pattern comprises of 32 quantitative peptides (Table 2), the third serum peptide pattern comprises of 24 quantitative peptides (Table 3), the fourth serum peptide pattern comprises of 16 quantitative peptides (Table 4), while the fifth serum peptide pattern comprises of 1 quantitative peptide (Table 5). These are the novel and not described previously serum peptides patterns that can serve for diagnostics of AIS and its differentiation from ICH.

Brief explanation of the method that provided the invention
1. Samples; a collection of serum samples from acute ischemic stroke (AIS) patients groups. 50-100 µl of serum samples from each patient are collected and stored at -80 °C immediately until the analysis.
2. Glassware qualities; mass spectrometer is a highly sensitive analytical machine to detect the minute amount of contaminants during the sample preparation. In order to not compromise the peptides signals, it is necessary to use high grades purity of water and other chemicals.
3. Buffer preparation; unexpected use of low pH buffer for peptide release from complexes with high abundant/carrier proteins. The citrate-phosphate buffer pH 3.1-3.6 was prepared in high grade of water.
4. Incubation; peptidomics samples preparation starts with the incubation by using a glass beaker between the serum samples and an acidic buffer, citrate-phosphate buffer for 3-5 minutes (gentle and vertexing necessary) in the temperature at 4-8 °C to dissociate protein-peptide interactions especially the peptides bound to the carrier proteins like albumin. Therefore, is characterized by the unexpected use of low pH buffer for peptide release from complexes with high abundant/carrier proteins.
5. Purification of peptides; single-step purification is performed by using hydrophilic-lipophilic balanced columns. Unexpectedly this column is able to deplete the highly abundant proteins without losing the peptides and simultaneously, it purifies the peptides from other contaminants in the serum. Therefore, it allows us to yield more peptides and isolate possible carrier peptides from highly abundant proteins.
6. Elution of peptides; first several washing performed with high-grade water to remove the unbound and low-binding peptides from the columns. Then the bound serum peptides are eluted from the columns using water/80% methanol/0.2% formic acid volume per volume (v/v). Further eluted peptides collected have been passed through 3kDalton molecular weight filters. Lastly, peptides of interest are lyophilized to dryness. All dried peptide samples were stored at -80 until the mass spectrometry analysis.
7. Sequencing of peptides with tandem mass spectrometry; the dried serum peptides were further subjected to sequencing with UltiMate^{™} 3000 RSLCnano liquid chromatograph (Thermo Scientific) online coupled with Orbitrap Exploris 480 mass spectrometer (MS) (Thermo Scientific). Indexed retention time (iRT) peptides were added to each sample for controlling the retention time of serum peptides for downstream quantitative analysis. Analytical peptide separation was performed by a non-linear gradient with mobile phases A and B. Serum peptides eluting from the analytical column were ionized in a nano-electrospray ion source (NSI) connected to a mass spectrometer.
8. The isolated peptides are then sequenced based on amino acid sequences with a tandem mass spectrometry tool.

Description of the following steps of the analysis - approach leading to provision of the invention - diagnosis method.

### a) Comprehensive qualitative and quantitative analysis

The invention employed multi-search engine strategies for creating the spectral library for qualitative and quantitative serum peptidomics, facilitating to screen the diagnostic peptides for the AIS. As a result, for the first time, comprehensive qualitative serum peptides were identified. Furthermore, it enabled DDA (data-dependent acquisition) to identify a total of at least 5000 serum peptides (iprophet iprobability value > 0.99%). At the same time, at least 1900 serum peptides were successfully quantified by DIA (data-independent acquisition) LC-MS/MS (Liquid Chromatography with tandem mass spectrometry) analysis of AIS samples. Therefore, high throughputs in the context of qualitative and quantitative serum peptidomics were the key for defining serum peptide patterns for AIS diagnostics, but the method that was used is not the limitation of the study.

### b) Peptide length distribution

The approach facilitates the crucial feature of serum peptidomics by screening a wide range of peptide lengths. These peptides were not generated by trypsin digestion, where the trypsin enzyme cleaves the proteins into every K (lysine) or R (arginine) site. On the contrary, the novel approach allows screening the natural and without any enzymatically digested peptides. Furthermore, the peptide length distribution is wide at 7-30 amino acid residues. Therefore, it can be easily tunable to 1-10 kilo Dalton in a range of natural serum peptides from the AIS samples.

### c) Data analysis workflow

The approach deals with quantitative analysis of high throughput (≥1900 peptides quantified) serum peptidomics. The serum peptidomics quantification workflow consists of unique steps within the Skyline, MSStats pipeline. First, the MS/MS DDA (data-dependent acquisition) data are searched in a multi-search engine strategy, including for example MS-fragger, Comet search engines (different search engines can be also used). These facilitate the creation of a comprehensive spectral library in which all serum peptide identification spectral data are summarized and later used to extract quantitative peptidomics data-independent MS/MS data (DIA). Next, the skyline extracts product ion chromatograms and integrates product ion peak group areas. The false discovery rate (FDR) of analysis is controlled by determining peak groups FDR values in mProphet module. Finally, inference of product ion peak areas to peptides and their summed peak area statistics were performed in MSstats module in a peptide-centric view. Output quantitative matrix from MSstats then subjected to the downstream multi-bioinformatics analysis such as volcano plot, dendrograms, heatmaps, functional AIS serum peptide enrichment maps, results shown in table 6.

9. Selection of the quantitative and qualitative peptides with the diagnostic value. The two thresholds were used for selection of the diagnostic peptides to define serum peptide patters. For the first serum peptide pattern (listed in Table 1 and 6) detected quantitative and qualitative peptides had to show adjusted P value (adj. pval) ≤ 0.05 (adjusted p value ≤ 0.05 is marked with "**" in table 6). In addition, in case of quantitative peptides the fold increase in serum peptide levels equal to 2 or higher was used as the second threshold for selection of diagnostic peptide to define serum peptide pattern. In table 6, the fold increase of the peptide levels is equal or higher than 2 (fold change ≥ 2) and is marked with "***" in the table 6, confirming the diagnosis of AIS.

For the second serum peptide pattern (listed in Table 2 and 7) detected quantitative peptides had to show adjusted P value (adj. pval) ≤ 0.05 (adjusted p value ≤ 0.05 is marked with "**" in table 7). The second threshold for defining the second diagnostic peptide pattern was the fold increase in serum peptide levels of these peptides that was equal or higher than 30 (fold change ≥ 30). In table 7, the fold increase of the serum peptide levels equal or higher than 30 (fold change ≥ 30) is marked with "***", confirming the diagnosis of AIS.

For the third serum peptide pattern (listed in Table 3 and 8) detected quantitative peptides had to show adjusted P value (adj. pval) ≤ 0.05 (adjusted p value ≤ 0.05 is marked with "**" in table 8). The second threshold for defining the second diagnostic peptide pattern was the fold increase in serum peptide levels of these peptides that was equal or higher than 50 (fold change ≥ 50). In table 8, the fold increase of the serum peptide levels equal or higher than 50 (fold change ≥ 50) is marked with "***", confirming the diagnosis of AIS.

For the fourth serum peptide pattern (listed in Table 4 and 9) detected quantitative peptides had to show adjusted P value (adj. pval) ≤ 0.05 (adjusted p value ≤ 0.05 is marked with "**" in table 9). The second threshold for defining the second diagnostic peptide pattern was the fold increase in serum peptide levels of these peptides that was equal or higher than 100 (fold change ≥ 100). In table 9, the fold increase of the serum peptide levels equal or higher than 100 (fold change ≥ 100) is marked with "***", confirming the diagnosis of AIS.

For the fifth serum peptide pattern (listed in Table 5 and 10) detected quantitative peptide had to show adjusted P value (adj. pval) ≤ 0.05 (adjusted p value ≤ 0.05 is marked with "**" in table 10). The second threshold for defining the second diagnostic peptide pattern was the fold increase in serum peptide levels of these peptides that was equal or higher than 1000 (fold change ≥ 1000). In table 10, the fold increase of the serum peptide levels equal or higher than 1000 (fold change ≥ 1000) is marked with "***", confirming the diagnosis of AIS.

For each of five serum peptide patterns for diagnostics of AIS the fold-increase reflects the ratio of integrated peak areas in peptides and shows how many times the serum levels of the peptide are increased in AIS patients as compared with individuals without stroke and patients with ICH.

These five serum peptide patterns (Tables 1-10) are complexed sets of biomarkers for AIS diagnostics and differentiation from ICH. Their diagnostic value was checked during the approach for the invention, and their levels were increased in serum samples of AIS patients as determined with MS/MS (tandem mass spectrometry) signaling intensities. However, MS/MS (tandem mass spectrometry) and signaling intensity measurement are not the limitations of the invention. The peptides can be identified and measured (quantified) with different methods.

Hence, in table 6, 1-100 peptides: Val-Ser-Glu-Thr-Glu-Ser-Arg-Gly-Ser-Glu-Ser-Gly-Ile-Phe-Thr-Asn-Thr-Lys, His-Arg-His-Pro-Asp-Glu-Ala-Ala-Phe-Phe-Asp-Thr-Ala-Ser-Thr, Phe-Phe-Asp-Thr-Ala-Ser-Thr-Gly-Lys, Gln-Leu-Gly-Leu-Pro-Gly-Pro-Pro-Asp-Val-Pro-Asp-His-Ala-Ala, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Ala-Ala-Thr-Val-Gly-Ser-Leu-Ala-Gly-Gln-Pro-Leu-Gln-Glu-Arg, Pro-Gly-Ser-Thr-Gly-Thr-Trp-Asn-Pro-Gly-Ser-Ser-Glu-Arg, His-Asp-Trp-Gly-His-Glu-Lys-Gln-Arg, His-Pro-Asp-Glu-Ala-Ala-Phe-Phe-Asp-Thr-Ala-Ser-Thr-Gly-Lys, Ser-Ser-His-His-Pro-Gly-Ile-Ala-Glu-Phe-Pro-Ser-Arg, Val-His-Leu-Thr-Pro-Glu-Glu-Lys-Ser-Ala-Val-Thr-Ala-Leu, Glu-Ser-Ser-Ser-His-His-Pro-Gly-Ile-Ala-Glu-Phe-Pro-Ser-Arg, Thr-Ala-Asn-Asp-Ser-Gly-Pro-Arg-Arg-Tyr-Thr-Ile-Ala-Ala-Leu, Arg-His-Asp-Trp-Gly-His-Glu-Lys-Gln-Arg, Val-Lys-Leu-Val-Asn-Glu-Val-Thr-Glu, Gln-Phe-Thr-Ser-Ser-Thr-Ser-Tyr-Asn, Ser-Gly-Asn-Val-Ser-Pro-Gly-Thr-Arg-Arg-Glu-Tyr-His-Thr-Glu-Lys, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Leu-Asp-Asp-Asp-Leu-Glu-His-Gln-Gly-Gly-His-Val-Leu-Asp-His-Gly-His, Ala-Phe-Arg-Gln-Pro-Ser-Ser-Ala, Phe-Val-Ser-Glu-Thr-Glu-Ser-Arg-Gly-Ser-Glu-Ser-Gly-Ile-Phe-Thr-Asn-Thr, Phe-Ser-Thr-Tyr-Asp-Arg-Asp-Asn-Asp-Gly-Trp, Thr-Val-Ile-Gln-Asn-Arg-Gln-Asp-Gly-Ser-Val-Asp-Phe, Arg-Glu-Tyr-His-Thr-Glu-Lys-Leu-Val-Thr-Ser, Gln-Ala-Gly-Ala-Ala-Gly-Ser-Arg-Met-Asn-Phe-Arg-Pro-Gly-Val-Leu-Ser-Ser, Ser-Pro-Gly-Ser-Gly-Asn-Ala-Arg-Pro-Asn-Asn-Pro-Asp-Trp-Gly-Thr-Phe, Ser-Asp-Ile-Ser-Ser-Thr-Arg-Gly-Phe-Pro-Ser-Val-Leu-Arg, Ile-Thr-His-Arg-Ile-His-Trp-Glu-Ser-Ala-Ser-Leu-Leu, Val-Ser-Gly-Trp-Gly-Arg-Asn-Ala-Asn-Phe, Phe-Asp-Thr-Ala-Ser-Thr-Gly-Lys-Thr-Phe-Pro-Gly-Phe, Ser-Ala-Gly-Ser-Trp-Asn-Ser-Gly-Ser-Ser-Gly-Pro-Gly-Ser-Thr-Gly-Asn-Arg-Asn, Val-His-Leu-Thr-Pro-Glu-Glu-Lys, Asp-Ala-Val-Arg-Gly-Ser-Pro-Ala-Ile-Asn-Val, Asp-Glu-Ala-Ala-Phe-Phe-Asp-Thr, Val-His-Leu-Thr-Pro-Glu-Glu-Lys-Ser-Ala-Val-Thr, Pro-Gly-Ser-Pro-Arg-Pro-Gly-Ser-Thr-Gly-Thr-Trp-Asn-Pro-Gly-Ser-Ser-Glu-Arg, Gly-Ser-Ser-Gly-Thr-Gly-Gly-Thr-Ala-Thr-Trp-Lys, Gly-Ser-Trp-Asn-Ser-Gly-Ser-Ser-Gly-Pro-Gly-Ser-Thr-Gly-Asn-Arg, Ser-Glu-Thr-Glu-Ser-Arg-Gly-Ser-Glu-Ser-Gly-Ile-Phe-Thr-Asn-Thr-Lys, Glu-Leu-Glu-Arg-Pro-Gly-Gly-Asn-Glu-Ile, Leu-Gly-Glu-Phe-Val-Ser-Glu-Thr-Glu-Ser-Arg, Ala-Leu-Glu-Glu-Tyr-Thr-Lys-Lys-Leu-Asn-Thr-Gln, Glu-Leu-Glu-Arg-Pro-Gly-Gly-Asn-Glu, Asp-Glu-Ala-Gly-Ser-Glu-Ala-Asp-His-Glu-Gly-Thr-His-Ser-Thr, Arg-His-Asp-Trp-Gly-His-Glu-Lys, Ala-Ala-Ser-Arg-Tyr-Leu-Asp-Lys-Thr-Glu-Gln, Arg-His-Pro-Asp-Glu-Ala-Ala-Phe, Pro-Asn-Ile-Leu-Arg-Leu-Glu-Ser-Glu-Glu-Thr, Ile-Glu-Gly-Val-Asp-Ala-Glu-Asp-Gly-His-Gly-Pro-Gly-Glu-Gln-Gln-Lys, Pro-Ser-Leu-Arg-Pro-Ala-Pro-Pro-Pro-Ile-Ser-Gly-Gly-Gly-Tyr, Thr-Asn-Thr-Lys-Glu-Ser-Ser-Ser-His-His-Pro-Gly-Ile-Ala-Glu-Phe-Pro-Ser-Arg-Gly, Ala-Glu-Val-Gly-Arg-Val-Gly-Tyr-Val-Ser-Gly-Trp-Gly-Arg-Asn-Ala-Asn-Phe, Asp-Glu-Ala-Ala-Phe-Phe-Asp-Thr-Ala-Ser-Thr-Gly-Lys, Lys-Val-Glu-Gln-Ala-Val-Glu-Thr-Glu-Pro-Glu-Pro-Glu-Leu-Arg-Gln-Gln-Thr, Met-Ala-Asp-Glu-Ala-Gly-Ser-Glu-Ala-Asp-His-Glu-Gly-Thr-His-Ser-Thr-Lys, Ser-Gly-Pro-Ser-Ser-Pro-Gln-Gln-Ala-Pro-Pro-Ala-Val-His, Gly-Ser-Glu-Ser-Gly-Ile-Phe-Thr-Asn-Thr-Lys-Glu-Ser-Ser-Ser-His-His-Pro-Gly-Ile-Ala-Glu-Phe-Pro-Ser-Arg-Gly, Arg-Glu-Glu-Ala-Pro-Ser-Leu-Arg, Glu-Glu-Val-Ser-Gly-Asn-Val-Ser-Pro-Gly-Thr, Glu-Asn-Glu-Gly-Phe-Thr-Val-Thr-Ala-Glu-Gly-Lys, Val-Thr-Glu-Pro-Ile-Ser-Ala-Glu-Ser-Gly-Glu-Gln-Val-Glu-Arg, Gly-Asp-Asp-Pro-Ser-Asp-Lys-Phe, Gly-Pro-Pro-Asp-Val-Pro-Asp-His-Ala, Glu-Glu-Val-Ser-Gly-Asn-Val-Ser-Pro-Gly-Thr-Arg-Arg-Glu, Tyr-His-Thr-Glu-Lys-Leu-Val, Tyr-Gln-Glu-Pro-Val-Leu-Gly-Pro-Val, Leu-Leu-Val-Arg-Tyr-Thr-Lys-Lys-Val-Pro-Gln-Val-Ser-Thr-Pro-Thr-Leu, Ile-Gln-Ile-Tyr-Lys-Lys-Leu-Arg-Thr-Ser-Ser-Phe, Ser-Val-Ser-Ala-Gly-Ser-Pro-His-Pro-Ala-Ile-Ala-Arg-Leu, His-Pro-Gly-Ile-Ala-Glu-Phe-Pro-Ser-Arg-Gly, Thr-Phe-Glu-Ser-Lys-Ser-Tyr, Ile-Glu-Gly-Val-Asp-Ala-Glu-Asp-Gly-His-Gly-Pro-Gly-Glu-Gln-Gln-Lys-Arg, Val-Leu-Val-Pro-Ala-Gly-Ser-Ala-Arg-Pro-Val-Ala-Phe, Asp-Ala-His-Lys-Ser-Glu-Val-Ala-His-Arg-Phe-Lys-Asp-Leu-Gly-Glu-Glu-Asn-Phe, Lys-Val-Lys-Glu-Lys-Leu-Lys-Ile-Asp-Ser, Glu-Phe-Val-Ser-Glu-Thr-Glu-Ser, His-Ala-Leu-Gln-Leu-Asn-Asn-Arg-Gln, Ser-Glu-Ser-Gly-Ile-Phe-Thr-Asn-Thr-Lys, Tyr-Val-Gly-Lys-Lys-Gln-Leu, Leu-Thr-Gln-Val-Lys-Glu-Ser-Leu-Ser-Ser-Tyr, Ser-Ser-His-His-Pro-Gly-Ile-Ala-Glu-Phe-Pro-Ser, Gly-Phe-Tyr-Glu-Ser-Asp-Val-Met-Gly-Arg, Gly-Gly-His-Leu-Asp-Ala-Lys-Gly-Ser-Phe, Asp-Val-Leu-Lys-Asp-Ser-Gly-Arg-Asp, His-Arg-His-Pro-Asp-Glu-Ala-Ala-Phe-Phe-Asp-Thr-Ala, Trp-Ser-Thr-Val-Lys-Asp-Lys-Phe-Ser-Glu-Phe, Arg-Gly-Ser-Glu-Ser-Gly-Ile-Phe-Thr-Asn-Thr, Glu-Glu-Ala-Pro-Ser-Leu-Arg-Pro-Ala-Pro-Pro-Pro-Ile-Ser-Gly-Gly-Gly-Tyr-Arg, Gly-Thr-Phe-Glu-Glu-Val-Ser-Gly-Asn-Val-Ser-Pro-Gly-Thr-Arg-Arg-Glu-Tyr-His-Thr-Glu, Gly-Ser-Ser-Gly-Thr-Gly-Gly-Thr-Ala-Thr-Trp-Lys-Pro-Gly-Ser-Ser, Ser-Ser-Ser-Tyr-Ser-Lys-Gln-Phe-Thr-Ser-Ser-Thr-Ser-Tyr-Asn-Arg-Gly-Asp-Ser-Thr-Phe-Glu-Ser-Lys-Ser-Tyr-Lys, Trp-Glu-Ser-Ala-Lys-Thr-Ala-Ala-Gln-Asn-Leu, Phe-Ser-Lys-Ala-Leu-Pro-Arg, Pro-Ser-Glu-Leu-Arg-Asp-Glu-His, Val-Val-Phe-Thr-Ala-Asn-Asp-Ser-Gly-Pro-Arg-Arg-Tyr-Thr-Ile-Ala, Leu-Gly-Glu-Phe-Val-Ser-Glu-Thr-Glu-Ser-Arg are quantitative peptides that were increased at least twice in AIS patients as compared with individuals without stroke and patients with ICH and the differences were statistically significant (adjusted p value ≤ 0.05 and fold change ≥ 2).

The next 54 peptides (from 101-154) in table 6: Asp-Asp-Tyr-Val-Asn-Thr-Gln-Gly-Ala-Ser-Leu, Tyr-Pro-Trp-Thr-Gln-Arg-Phe, Phe-Arg-Leu-Leu-Gly-Asn-Val-Leu, Tyr-Gln-Lys-Val-Val-Ala-Gly-Val-Ala-Asn-Ala-Leu-Ala-His-Lys-Tyr-His, Phe-Ser-Glu-Thr-Phe-Gln-Lys-Val-Lys-Glu-Lys-Leu-Lys-Ile-Asp-Ser, Val-Lys-Glu-Lys-Leu-Lys-Ile-Asp-Ser, Tyr-Glu-Lys-Thr-Tyr-Leu-Pro-Ala-Val-Asp-Glu-Lys-Leu, Thr-Gln-Gln-Pro-Gln-Gln-Asp-Glu-Met-Pro-Ser-Pro-Thr-Phe-Leu, Thr-Gly-Ile-Phe-Thr-Asp-Gln-Val-Leu, Ser-Thr-Val-Lys-Asp-Lys-Phe-Ser-Glu-Phe, Tyr-Trp-Ser-Thr-Val-Lys-Asp-Lys-Phe, Tyr-Trp-Ser-Thr-Val-Lys-Asp-Lys-Phe-Ser-Glu-Phe, His-Thr-Asp-Leu-Ser-Gly-Lys-Val, His-Thr-Asp-Leu-Ser-Gly-Lys-Val-Phe-Val-Phe, Tyr-Val-Ile-Ile-Lys-Pro-Leu-Val-Trp-Val, Ala-Val-Arg-Gly-Ser-Pro-Ala-Ile-Asn, Glu-Val-Val-Phe-Thr-Ala-Asn-Asp-Ser-Gly-Pro-Arg-Arg-Tyr-Thr-Ile-Ala, Glu-Val-Val-Phe-Thr-Ala-Asn-Asp-Ser-Gly-Pro-Arg-Arg-Tyr-Thr-Ile-Ala-Ala, Phe-Thr-Ala-Asn-Asp-Ser-Gly-Pro-Arg-Arg-Tyr-Thr-Ile-Ala-Ala, Phe-Val-Glu-Gly-Ile-Tyr-Lys-Val-Glu, Leu-Leu-Ser-Pro-Tyr-Ser-Tyr-Ser-Thr-Thr-Ala, Leu-Ser-Pro-Tyr-Ser-Tyr-Ser-Thr-Thr-Ala, Thr-Ala-Asn-Asp-Ser-Gly-Pro-Arg-Arg-Tyr-Thr-Ile-Ala-Ala-Leu-Leu-Ser-Pro-Tyr-Ser-Tyr-Ser-Thr-Thr-Ala, Val-Val-Phe-Thr-Ala-Asn-Asp-Ser-Gly-Pro-Arg-Arg-Tyr-Thr-Ile-Ala-Ala, Phe-Lys-Asp-Ser-Ala-His-Gly-Phe, Phe-Ser-Ser-Pro-His-Gly-Lys-Asp-Leu-Leu-Phe-Lys-Asp-Ser-Ala-His-Gly-Phe, Asn-Gln-Ala-Gln-Glu-His-Phe-Gly-Lys-Asp-Lys-Ser-Lys-Glu-Phe-Gln-Leu, Tyr-Ala-Val-Ala-Val-Val-Lys-Lys-Asp, Tyr-Ala-Val-Ala-Val-Val-Lys-Lys-Asp-Ser, Tyr-Val-Thr-Ala-Ile-Arg-Asn-Leu-Arg-Glu, Ser-Lys-Glu-Asp-Pro-Gln-Thr-Phe-Tyr-Tyr-Ala-Val-Ala, Tyr-Gly-Ser-Lys-Glu-Asp-Pro-Gln-Thr-Phe, Ala-Tyr-Leu-Ala-Pro-Asn-Asn-Leu-Lys-Pro-Val-Val-Ala-Glu, Ala-Tyr-Leu-Ala-Pro-Asn-Asn-Leu-Lys-Pro-Val-Val-Ala-Glu-Phe, Ala-Tyr-Leu-Ala-Pro-Asn-Asn-Leu-Lys-Pro-Val-Val-Ala-Glu-Phe-Tyr-Gly-Ser-Lys-Glu-Asp, Asp-Ala-Gly-Leu-Val-Tyr-Asp-Ala-Tyr, Asp-Ala-Tyr-Leu-Ala-Pro-Asn-Asn-Leu-Lys-Pro-Val-Val-Ala-Glu-Phe, Tyr-Leu-Ala-Pro-Asn-Asn-Leu-Lys-Pro-Val-Val-Ala-Glu, Tyr-Leu-Ala-Pro-Asn-Asn-Leu-Lys-Pro-Val-Val-Ala-Glu-Phe, Tyr-Leu-Ala-Pro-Asn-Asn-Leu-Lys-Pro-Val-Val-Ala-Glu-Phe-Tyr-Gly-Ser-Lys-Glu-Asp, Ser-Ala-Leu-Thr-Ser-Asp-Asn-His-Gly-Ala-Thr-Tyr, Ser-Ala-Leu-Thr-Ser-Asp-Asn-His-Gly-Ala-Thr-Tyr-Ala-Phe-Ser-Gly-Thr-His-Tyr, Phe-Arg-Gln-Gly-His-Asn-Ser-Val-Phe, Val-Ala-Glu-Lys-Ala-Leu-Gln-Glu-Tyr-Arg-Lys-Lys-His-Arg-Glu-Glu, Phe-Thr-Arg-Glu-Pro-Gln-Asp-Thr-Tyr-His-Tyr-Leu-Pro, Arg-Pro-His-Phe-Phe-Phe-Pro, Arg-Pro-His-Phe-Phe-Phe-Pro-Lys-Ser-Arg-Ile-Val, Phe-Leu-Arg-Asp-Thr-Lys-Thr-Leu, Val-Ile-Glu-Pro-Gln-Gly-Leu-Arg-Phe, Phe-Val-Thr-Pro-Leu-Thr-Ser-Met, Val-His-Leu-Thr-Pro-Glu-Glu-Lys-Ser, Trp-Gly-Lys-Val-Asn-Val-Asp-Glu-Val-Gly-Gly-Glu-Ala-Leu-Gly-Arg-Leu, Arg-Gly-Pro-Leu-Val-Lys-Tyr-Ser-Ser-Asp-Tyr, Ala-Ser-Glu-Gly-Gly-Phe-Thr-Ala-Thr-Gly-Gln-Arg are qualitative peptides detectable only in patients with AIS. If other peptides are detected or show ≥2-fold increase they do not correspond with AIS.

**Table 6. The serum peptide pattern 1 for diagnostics of AIS and differential diagnosis of AIS from ICH. List of AIS diagnostic peptides with amino acid sequence, adjusted p value, and fold change is given.**

| | Serial number | Protein identifier* | Peptide sequence | Adjusted p value** | Fold change*** |
|---|---|---|---|---|---|
| Quantitative peptides | 1 | FIBA_HUMAN | Val-Ser-Glu-Thr-Glu-Ser- Arg-Gly-Ser-Glu-Ser-Gly- Ile-Phe-Thr-Asn-Thr-Lys | 0.0000861 | 1765.5 |
| | 2 | FIBA_HUMAN | His-Arg-His-Pro-Asp- Glu-Ala-Ala-Phe-Phe- Asp-Thr-Ala-Ser-Thr | 0.000135 | 33.2 |
| | 3 | FIBA_HUMAN | Phe-Phe-Asp-Thr-Ala- Ser-Thr-Gly-Lys | 0.000452 | 207.9 |
| | 4 | ITIH4_HUMAN | Gln-Leu-Gly-Leu-Pro- Gly-Pro-Pro-Asp-Val- Pro-Asp-His-Ala-Ala | 0.000452 | 3.7 |
| | 5 | FIBA_HUMAN | Gly-Ser-Glu-Ser-Gly-Ile- Phe-Thr-Asn-Thr-Lys | 0.00134 | 782.3 |
| | 6 | FIBA_HUMAN | Asn-Pro-Gly-Ser-Ser- Gly-Thr-Gly-Gly-Thr-Ala- Thr-Trp-Lys | 0.00134 | 215.2 |
| | 7 | FIBA_HUMAN | Gln-Phe-Thr-Ser-Ser- Thr-Ser-Tyr-Asn-Arg | 0.00134 | 154.9 |
| | 8 | FIBB_HUMAN | Ala-Pro-Pro-Pro-Ile-Ser- Gly-Gly-Gly-Tyr | 0.00134 | 116.7 |
| | 9 | APOE_HUMAN | Ala-Ala-Thr-Val-Gly-Ser- Leu-Ala-Gly-Gln-Pro- Leu-Gln-Glu-Arg | 0.00134 | 46.9 |
| | 10 | FIBA_HUMAN | Pro-Gly-Ser-Thr-Gly-Thr- Trp-Asn-Pro-Gly-Ser- Ser-Glu-Arg | 0.001509 | 199.9 |
| | 11 | KNG1_HUMAN | His-Asp-Trp-Gly-His-Glu- Lys-Gln-Arg | 0.00162 | 32.0 |
| | 12 | FIBA_HUMAN | His-Pro-Asp-Glu-Ala-Ala- Phe-Phe-Asp-Thr-Ala- Ser-Thr-Gly-Lys | 0.003056 | 97.8 |
| | 13 | FIBA_HUMAN | Ser-Ser-His-His-Pro-Gly- Ile-Ala-Glu-Phe-Pro-Ser- Arg | 0.003239 | 38.1 |
| | 14 | HBB_HUMAN | Val-His-Leu-Thr-Pro- Glu-Glu-Lys-Ser-Ala-Val- Thr-Ala-Leu | 0.003239 | 14.2 |
| | 15 | FIBA_HUMAN | Glu-Ser-Ser-Ser-His-His- Pro-Gly-Ile-Ala-Glu-Phe- Pro-Ser-Arg | 0.003319 | 141.6 |
| | 16 | TTHY_HUMAN | Thr-Ala-Asn-Asp-Ser- Gly-Pro-Arg-Arg-Tyr- Thr-Ile-Ala-Ala-Leu | 0.003319 | 16.2 |
| | 17 | KNG1_HUMAN | Arg-His-Asp-Trp-Gly-His- Glu-Lys-Gln-Arg | 0.003319 | 6.3 |
| | 18 | ALBU_HUMAN | Val-Lys-Leu-Val-Asn- Glu-Val-Thr-Glu | 0.003319 | 5.0 |
| | 19 | FIBA_HUMAN | Gln-Phe-Thr-Ser-Ser- Thr-Ser-Tyr-Asn | 0.003536 | 6.3 |
| | 20 | FIBA_HUMAN | Ser-Gly-Asn-Val-Ser-Pro- Gly-Thr-Arg-Arg-Glu- Tyr-His-Thr-Glu-Lys | 0.003789 | 32.8 |
| | 21 | FIBA_HUMAN | Pro-Gly-Ser-Ser-Gly-Pro- Gly-Ser-Thr-Gly-Ser-Trp- Asn-Ser-Gly-Ser-Ser-Gly- Thr-Gly-Ser-Thr-Gly- Asn-Gln-Asn-Pro-Gly- Ser-Pro-Arg | 0.003798 | 206.2 |
| | 22 | FIBA_HUMAN | Thr-Ser-Lys-Gly-Asp-Lys- Glu-Leu | 0.003798 | 198.4 |
| | 23 | KNG1_HUMAN | Leu-Asp-Asp-Asp-Leu- Glu-His-Gln-Gly-Gly-His- Val-Leu-Asp-His-Gly-His | 0.003798 | 96.1 |
| | 24 | CO3_HUMAN | Ala-Phe-Arg-Gln-Pro- Ser-Ser-Ala | 0.004176 | 9.1 |
| | 25 | FIBA_ HUMAN | Phe-Val-Ser-Glu-Thr- Glu-Ser-Arg-Gly-Ser- Glu-Ser-Gly-Ile-Phe-Thr- Asn-Thr | 0.005196 | 41.2 |
| | 26 | FIBB_HUMAN | Phe-Ser-Thr-Tyr-Asp- Arg-Asp-Asn-Asp-Gly- Trp | 0.005264 | 6.2 |
| | 27 | FIBB_HUMAN | Thr-Val-Ile-Gln-Asn-Arg- Gln-Asp-Gly-Ser-Val- Asp-Phe | 0.005299 | 3.0 |
| | 28 | FIBA_HUMAN | Arg-Glu-Tyr-His-Thr-Glu- Lys-Leu-Val-Thr-Ser | 0.006342 | 125.3 |
| | 29 | ITIH4_HUMAN | Gln-Ala-Gly-Ala-Ala-Gly- Ser-Arg-Met-Asn-Phe- Arg-Pro-Gly-Val-Leu- Ser-Ser | 0.006342 | 9.8 |
| | 30 | FIBA_HUMAN | Ser-Pro-Gly-Ser-Gly- Asn-Ala-Arg-Pro-Asn- Asn-Pro-Asp-Trp-Gly- Thr-Phe | 0.006784 | 7.3 |
| | 31 | IGHM_HUMAN | Ser-Asp-Ile-Ser-Ser-Thr- Arg-Gly-Phe-Pro-Ser- Val-Leu-Arg | 0.006963 | 4.9 |
| | 32 | CO3_HUMAN | Ile-Thr-His-Arg-Ile-His- Trp-Glu-Ser-Ala-Ser-Leu- Leu | 0.008326 | 7.1 |
| | 33 | HPT_HUMAN | Val-Ser-Gly-Trp-Gly-Arg- Asn-Ala-Asn-Phe | 0.008326 | 6.4 |
| | 34 | FIBA_HUMAN | Phe-Asp-Thr-Ala-Ser- Thr-Gly-Lys-Thr-Phe- Pro-Gly-Phe | 0.008326 | 4.5 |
| | 35 | FIBA_HUMAN | Ser-Ala-Gly-Ser-Trp-Asn- Ser-Gly-Ser-Ser-Gly-Pro- Gly-Ser-Thr-Gly-Asn- Arg-Asn | 0.008442 | 6.6 |
| | 36 | HBD_HUMAN | Val-His-Leu-Thr-Pro- Glu-Glu-Lys | 0.008442 | 3.0 |
| | 37 | TTHY_HUMAN | Asp-Ala-Val-Arg-Gly-Ser- Pro-Ala-Ile-Asn-Val | 0.009255 | 6.5 |
| | 38 | FIBA_HUMAN | Asp-Glu-Ala-Ala-Phe- Phe-Asp-Thr | 0.009894 | 6.5 |
| | 39 | HBB_HUMAN | Val-His-Leu-Thr-Pro- Glu-Glu-Lys-Ser-Ala-Val- Thr | 0.010777 | 2.6 |
| | 40 | FIBA_HUMAN | Pro-Gly-Ser-Pro-Arg- Pro-Gly-Ser-Thr-Gly-Thr- Trp-Asn-Pro-Gly-Ser- Ser-Glu-Arg | 0.011021 | 20.4 |
| | 41 | FIBA_HUMAN | Gly-Ser-Ser-Gly-Thr-Gly- Gly-Thr-Ala-Thr-Trp-Lys | 0.012001 | 307.0 |
| | 42 | FIBA_HUMAN | Gly-Ser-Trp-Asn-Ser- Gly-Ser-Ser-Gly-Pro-Gly- Ser-Thr-Gly-Asn-Arg | 0.012001 | 74.0 |
| | 43 | FIBA_ HUMAN | Ser-Glu-Thr-Glu-Ser- Arg-Gly-Ser-Glu-Ser-Gly- Ile-Phe-Thr-Asn-Thr-Lys | 0.01271 | 5.5 |
| | 44 | FIBA_HUMAN | Glu-Leu-Glu-Arg-Pro- Gly-Gly-Asn-Glu-Ile | 0.015039 | 198.9 |
| | 45 | FIBA_HUMAN | Leu-Gly-Glu-Phe-Val- Ser-Glu-Thr-Glu-Ser-Arg | 0.015039 | 79.3 |
| | 46 | APOA1_HUMAN | Ala-Leu-Glu-Glu-Tyr- Thr-Lys-Lys-Leu-Asn- Thr-Gln | 0.015039 | 7.1 |
| | 47 | FIBA_HUMAN | Glu-Leu-Glu-Arg-Pro- Gly-Gly-Asn-Glu | 0.015298 | 84.8 |
| | 48 | FIBA_HUMAN | Asp-Glu-Ala-Gly-Ser- Glu-Ala-Asp-His-Glu- Gly-Thr-His-Ser-Thr | 0.015298 | 49.0 |
| | 49 | KNG1_HUMAN | Arg-His-Asp-Trp-Gly-His- Glu-Lys | 0.015298 | 10.6 |
| | 50 | CO4A_HUMAN | Ala-Ala-Ser-Arg-Tyr-Leu- Asp-Lys-Thr-Glu-Gln | 0.015298 | 5.2 |
| | 51 | FIBA_HUMAN | Arg-His-Pro-Asp-Glu- Ala-Ala-Phe | 0.015442 | 18.0 |
| | 52 | CO3_HUMAN | Pro-Asn-Ile-Leu-Arg- Leu-Glu-Ser-Glu-Glu-Thr | 0.015442 | 2.8 |
| | 53 | CFAB_HUMAN | Ile-Glu-Gly-Val-Asp-Ala- Glu-Asp-Gly-His-Gly- Pro-Gly-Glu-Gln-Gln-Lys | 0.015442 | 2.8 |
| | 54 | FIBB_HUMAN | Pro-Ser-Leu-Arg-Pro- Ala-Pro-Pro-Pro-Ile-Ser- Gly-Gly-Gly-Tyr | 0.015442 | 2.3 |
| | 55 | FIBA_HUMAN | Thr-Asn-Thr-Lys-Glu- Ser-Ser-Ser-His-His-Pro- Gly-Ile-Ala-Glu-Phe-Pro- Ser-Arg-Gly | 0.015865 | 4.4 |
| | 56 | HPT_HUMAN | Ala-Glu-Val-Gly-Arg-Val- Gly-Tyr-Val-Ser-Gly-Trp- Gly-Arg-Asn-Ala-Asn- Phe | 0.016377 | 10.3 |
| | 57 | FIBA_HUMAN | Asp-Glu-Ala-Ala-Phe- Phe-Asp-Thr-Ala-Ser- Thr-Gly-Lys | 0.016406 | 57.1 |
| | 58 | APOE_HUMAN | Lys-Val-Glu-Gln-Ala-Val- Glu-Thr-Glu-Pro-Glu- Pro-Glu-Leu-Arg-Gln- Gln-Thr | 0.016406 | 2.8 |
| | 59 | FIBA_HUMAN | Met-Ala-Asp-Glu-Ala- Gly-Ser-Glu-Ala-Asp-His- Glu-Gly-Thr-His-Ser-Thr- Lys | 0.01677 | 43.3 |
| | 60 | A0A140T9L8 | Ser-Gly-Pro-Ser-Ser-Pro- Gln-Gln-Ala-Pro-Pro- Ala-Val-His | 0.018982 | 3.4 |
| | 61 | FIBA_HUMAN | Gly-Ser-Glu-Ser-Gly-Ile- Phe-Thr-Asn-Thr-Lys- Glu-Ser-Ser-Ser-His-His- Pro-Gly-Ile-Ala-Glu-Phe- Pro-Ser-Arg-Gly | 0.019797 | 9.6 |
| | 62 | FIBB_HUMAN | Arg-Glu-Glu-Ala-Pro- Ser-Leu-Arg | 0.02059 | 350.0 |
| | 63 | FIBA_HUMAN | Glu-Glu-Val-Ser-Gly- Asn-Val-Ser-Pro-Gly-Thr | 0.020618 | 114.7 |
| | 64 | CO3_HUMAN | Glu-Asn-Glu-Gly-Phe- Thr-Val-Thr-Ala-Glu-Gly- Lys | 0.021119 | 7.0 |
| | 65 | APOL1_HUMAN | Val-Thr-Glu-Pro-Ile-Ser- Ala-Glu-Ser-Gly-Glu-Gln- Val-Glu-Arg | 0.021119 | 4.0 |
| | 66 | FIBG_HUMAN | Gly-Asp-Asp-Pro-Ser- Asp-Lys-Phe | 0.021119 | 2.8 |
| | 67 | ITIH4_HUMAN | Gly-Pro-Pro-Asp-Val- Pro-Asp-His-Ala | 0.021119 | 2.4 |
| | 68 | FIBA_HUMAN | Glu-Glu-Val-Ser-Gly- Asn-Val-Ser-Pro-Gly- Thr-Arg-Arg-Glu | 0.021679 | 2.1 |
| | 69 | FIBA_HUMAN | Tyr-His-Thr-Glu-Lys-Leu- Val | 0.023036 | 10.6 |
| | 70 | CASB_HUMAN | Tyr-Gln-Glu-Pro-Val- Leu-Gly-Pro-Val | 0.024791 | 59.5 |
| | 71 | ALBU_HUMAN | Leu-Leu-Val-Arg-Tyr- Thr-Lys-Lys-Val-Pro-Gln- Val-Ser-Thr-Pro-Thr-Leu | 0.024791 | 19.8 |
| | 72 | APOB_HUMAN | Ile-Gln-Ile-Tyr-Lys-Lys- Leu-Arg-Thr-Ser-Ser-Phe | 0.025146 | 5.1 |
| | 73 | CO4A_HUMAN | Ser-Val-Ser-Ala-Gly-Ser- Pro-His-Pro-Ala-Ile-Ala- Arg-Leu | 0.026078 | 3.2 |
| | 74 | FIBA_HUMAN | His-Pro-Gly-Ile-Ala-Glu- Phe-Pro-Ser-Arg-Gly | 0.026226 | 10.8 |
| | 75 | FIBA_HUMAN | Thr-Phe-Glu-Ser-Lys- Ser-Tyr | 0.02979 | 2.7 |
| | 76 | CFAB_HUMAN | Ile-Glu-Gly-Val-Asp-Ala- Glu-Asp-Gly-His-Gly- Pro-Gly-Glu-Gln-Gln- Lys-Arg | 0.029825 | 4.9 |
| | 77 | CO4A_HUMAN | Val-Leu-Val-Pro-Ala-Gly- Ser-Ala-Arg-Pro-Val-Ala- Phe | 0.029825 | 2.4 |
| | 78 | ALBU_HUMAN | Asp-Ala-His-Lys-Ser-Glu- Val-Ala-His-Arg-Phe-Lys- Asp-Leu-Gly-Glu-Glu- Asn-Phe | 0.030439 | 11.8 |
| | 79 | APOC1_HUMAN | Lys-Val-Lys-Glu-Lys-Leu- Lys-Ile-Asp-Ser | 0.030439 | 10.4 |
| | 80 | FIBA_ HUMAN | Glu-Phe-Val-Ser-Glu- Thr-Glu-Ser | 0.033024 | 19.3 |
| | 81 | CO4A_HUMAN | His-Ala-Leu-Gln-Leu- Asn-Asn-Arg-Gln | 0.033813 | 2.6 |
| | 82 | FIBA_HUMAN | Ser-Glu-Ser-Gly-Ile-Phe- Thr-Asn-Thr-Lys | 0.034075 | 151.8 |
| | 83 | HPT_HUMAN | Tyr-Val-Gly-Lys-Lys-Gln- Leu | 0.034075 | 5.9 |
| | 84 | APOC2_HUMAN | Leu-Thr-Gln-Val-Lys- Glu-Ser-Leu-Ser-Ser-Tyr | 0.03417 | 4.3 |
| | 85 | FIBA_HUMAN | Ser-Ser-His-His-Pro-Gly- Ile-Ala-Glu-Phe-Pro-Ser | 0.03421 | 19.0 |
| | 86 | A2MG_HUMAN | Gly-Phe-Tyr-Glu-Ser- Asp-Val-Met-Gly-Arg | 0.03421 | 7.0 |
| | 87 | HPT_HUMAN | Gly-Gly-His-Leu-Asp- Ala-Lys-Gly-Ser-Phe | 0.03421 | 2.2 |
| | 88 | APOA1_HUMAN | Asp-Val-Leu-Lys-Asp- Ser-Gly-Arg-Asp | 0.037447 | 4.6 |
| | 89 | FIBA_HUMAN | His-Arg-His-Pro-Asp- Glu-Ala-Ala-Phe-Phe- Asp-Thr-Ala | 0.038093 | 61.7 |
| | 90 | FIBA_HUMAN | Trp-Ser-Thr-Val-Lys-Asp- Lys-Phe-Ser-Glu-Phe | 0.041114 | 10.8 |
| | 91 | APOC3_HUMAN | Arg-Gly-Ser-Glu-Ser-Gly- Ile-Phe-Thr-Asn-Thr | 0.042977 | 2.0 |
| | 92 | FIBA_HUMAN | Glu-Glu-Ala-Pro-Ser- Leu-Arg-Pro-Ala-Pro- Pro-Pro-Ile-Ser-Gly-Gly- Gly-Tyr-Arg | 0.043616 | 3.8 |
| | 93 | FIBB_HUMAN | Gly-Thr-Phe-Glu-Glu- Val-Ser-Gly-Asn-Val-Ser- Pro-Gly-Thr-Arg-Arg- Glu-Tyr-His-Thr-Glu | 0.044407 | 15.3 |
| | 94 | FIBA_HUMAN | Gly-Ser-Ser-Gly-Thr-Gly- Gly-Thr-Ala-Thr-Trp-Lys- Pro-Gly-Ser-Ser | 0.045174 | 5.4 |
| | 95 | FIBA_HUMAN | Ser-Ser-Ser-Tyr-Ser-Lys- Gln-Phe-Thr-Ser-Ser- Thr-Ser-Tyr-Asn-Arg- Gly-Asp-Ser-Thr-Phe- Glu-Ser-Lys-Ser-Tyr-Lys | 0.045699 | 2.3 |
| | 96 | FIBA_HUMAN | Trp-Glu-Ser-Ala-Lys-Thr- Ala-Ala-Gln-Asn-Leu | 0.045923 | 11.2 |
| | 97 | APOC2_HUMAN | Phe-Ser-Lys-Ala-Leu- Pro-Arg | 0.045923 | 3.0 |
| | 98 | RARR2_HUMAN | Pro-Ser-Glu-Leu-Arg- Asp-Glu-His | 0.046904 | 2.9 |
| | 99 | TLN1_HUMAN | Val-Val-Phe-Thr-Ala- Asn-Asp-Ser-Gly-Pro- Arg-Arg-Tyr-Thr-Ile-Ala | 0.049165 | 4.2 |
| | 100 | TTHY_HUMAN | Leu-Gly-Glu-Phe-Val- Ser-Glu-Thr-Glu-Ser-Arg | 0.015039 | 7.1 |
| Qualitative peptides | 101 | PLMN_HUMAN | Asp-Asp-Tyr-Val-Asn- Thr-Gln-Gly-Ala-Ser-Leu | AIS only | AIS only |
| | 102 | HBD_HUMAN | Tyr-Pro-Trp-Thr-Gln- Arg-Phe | AIS only | AIS only |
| | 103 | HBD_HUMAN | Phe-Arg-Leu-Leu-Gly- Asn-Val-Leu | AIS only | AIS only |
| | 104 | HBD_HUMAN | Tyr-Gln-Lys-Val-Val-Ala- Gly-Val-Ala-Asn-Ala- Leu-Ala-His-Lys-Tyr-His | AIS only | AIS only |
| | 105 | APOC1_HUMAN | Phe-Ser-Glu-Thr-Phe- Gln-Lys-Val-Lys-Glu-Lys- Leu-Lys-Ile-Asp-Ser | AIS only | AIS only |
| | 106 | APOC1_HUMAN | Val-Lys-Glu-Lys-Leu-Lys- Ile-Asp-Ser | AIS only | AIS only |
| | 107 | APOC2_HUMAN | Tyr-Glu-Lys-Thr-Tyr-Leu- Pro-Ala-Val-Asp-Glu- Lys-Leu | AIS only | AIS only |
| | 108 | APOC2_HUMAN | Thr-Gln-Gln-Pro-Gln- Gln-Asp-Glu-Met-Pro- Ser-Pro-Thr-Phe-Leu | AIS only | AIS only |
| | 109 | APOC2_HUMAN | Thr-Gly-Ile-Phe-Thr-Asp- Gln-Val-Leu | AIS only | AIS only |
| | 110 | APOC3_HUMAN | Ser-Thr-Val-Lys-Asp-Lys- Phe-Ser-Glu-Phe | AIS only | AIS only |
| | 111 | APOC3_HUMAN | Tyr-Trp-Ser-Thr-Val-Lys- Asp-Lys-Phe | AIS only | AIS only |
| | 112 | APOC3_HUMAN | Tyr-Trp-Ser-Thr-Val-Lys- Asp-Lys-Phe-Ser-Glu- Phe | AIS only | AIS only |
| | 113 | SAMP_HUMAN | His-Thr-Asp-Leu-Ser- Gly-Lys-Val | AIS only | AIS only |
| | 114 | SAMP_HUMAN | His-Thr-Asp-Leu-Ser- Gly-Lys-Val-Phe-Val-Phe | AIS only | AIS only |
| | 115 | SAMP_HUMAN | Tyr-Val-Ile-Ile-Lys-Pro- Leu-Val-Trp-Val | AIS only | AIS only |
| | 116 | TTHY_HUMAN | Ala-Val-Arg-Gly-Ser-Pro- Ala-Ile-Asn | AIS only | AIS only |
| | 117 | TTHY_HUMAN | Glu-Val-Val-Phe-Thr- Ala-Asn-Asp-Ser-Gly- Pro-Arg-Arg-Tyr-Thr-Ile- Ala | AIS only | AIS only |
| | 118 | TTHY_HUMAN | Glu-Val-Val-Phe-Thr- Ala-Asn-Asp-Ser-Gly- Pro-Arg-Arg-Tyr-Thr-Ile- Ala-Ala | AIS only | AIS only |
| | 119 | TTHY_HUMAN | Phe-Thr-Ala-Asn-Asp- Ser-Gly-Pro-Arg-Arg- Tyr-Thr-Ile-Ala-Ala | AIS only | AIS only |
| | 120 | TTHY_HUMAN | Phe-Val-Glu-Gly-Ile-Tyr- Lys-Val-Glu | AIS only | AIS only |
| | 121 | TTHY_HUMAN | Leu-Leu-Ser-Pro-Tyr- Ser-Tyr-Ser-Thr-Thr-Ala | AIS only | AIS only |
| | 122 | TTHY_HUMAN | Leu-Ser-Pro-Tyr-Ser-Tyr- Ser-Thr-Thr-Ala | AIS only | AIS only |
| | 123 | TTHY_HUMAN | Thr-Ala-Asn-Asp-Ser- Gly-Pro-Arg-Arg-Tyr- Thr-Ile-Ala-Ala-Leu-Leu- Ser-Pro-Tyr-Ser-Tyr-Ser- Thr-Thr-Ala | AIS only | AIS only |
| | 124 | TTHY_HUMAN | Val-Val-Phe-Thr-Ala- Asn-Asp-Ser-Gly-Pro- Arg-Arg-Tyr-Thr-Ile-Ala- Ala | AIS only | AIS only |
| | 125 | TRFE_HUMAN | Phe-Lys-Asp-Ser-Ala- His-Gly-Phe | AIS only | AIS only |
| | 126 | TRFE_HUMAN | Phe-Ser-Ser-Pro-His- Gly-Lys-Asp-Leu-Leu- Phe-Lys-Asp-Ser-Ala- His-Gly-Phe | AIS only | AIS only |
| | 127 | TRFE_HUMAN | Asn-Gln-Ala-Gln-Glu- His-Phe-Gly-Lys-Asp- Lys-Ser-Lys-Glu-Phe- Gln-Leu | AIS only | AIS only |
| | 128 | TRFE_HUMAN | Tyr-Ala-Val-Ala-Val-Val- Lys-Lys-Asp | AIS only | AIS only |
| | 129 | TRFE_HUMAN | Tyr-Ala-Val-Ala-Val-Val- Lys-Lys-Asp-Ser | AIS only | AIS only |
| | 130 | TRFE_HUMAN | Tyr-Val-Thr-Ala-Ile-Arg- Asn-Leu-Arg-Glu | AIS only | AIS only |
| | 131 | TRFE_HUMAN | Ser-Lys-Glu-Asp-Pro- Gln-Thr-Phe-Tyr-Tyr- Ala-Val-Ala | AIS only | AIS only |
| | 132 | TRFE_HUMAN | Tyr-Gly-Ser-Lys-Glu-Asp- Pro-Gln-Thr-Phe | AIS only | AIS only |
| | 133 | TRFE_HUMAN | | AIS only | AIS only |
| | 134 | TRFE_HUMAN | Ala-Tyr-Leu-Ala-Pro- Asn-Asn-Leu-Lys-Pro- Val-Val-Ala-Glu-Phe | AIS only | AIS only |
| | 135 | TRFE_HUMAN | Ala-Tyr-Leu-Ala-Pro- Asn-Asn-Leu-Lys-Pro- Val-Val-Ala-Glu-Phe-Tyr- Gly-Ser-Lys-Glu-Asp | AIS only | AIS only |
| | 136 | TRFE_HUMAN | Asp-Ala-Gly-Leu-Val- Tyr-Asp-Ala-Tyr | AIS only | AIS only |
| | 137 | TRFE_HUMAN | Asp-Ala-Tyr-Leu-Ala- Pro-Asn-Asn-Leu-Lys- Pro-Val-Val-Ala-Glu-Phe | AIS only | AIS only |
| | 138 | TRFE_HUMAN | Tyr-Leu-Ala-Pro-Asn- Asn-Leu-Lys-Pro-Val- Val-Ala-Glu | AIS only | AIS only |
| | 139 | TRFE_HUMAN | Tyr-Leu-Ala-Pro-Asn- Asn-Leu-Lys-Pro-Val- Val-Ala-Glu-Phe | AIS only | AIS only |
| | 140 | TRFE_HUMAN | Tyr-Leu-Ala-Pro-Asn- Asn-Leu-Lys-Pro-Val- Val-Ala-Glu-Phe-Tyr-Gly- Ser-Lys-Glu-Asp | AIS only | AIS only |
| | 141 | HEMO_HUMAN | Ser-Ala-Leu-Thr-Ser- Asp-Asn-His-Gly-Ala- Thr-Tyr | AIS only | AIS only |
| | 142 | HEMO_HUMAN | Ser-Ala-Leu-Thr-Ser- Asp-Asn-His-Gly-Ala- Thr-Tyr-Ala-Phe-Ser- Gly-Thr-His-Tyr | AIS only | AIS only |
| | 143 | HEMO_HUMAN | Phe-Arg-Gln-Gly-His- Asn-Ser-Val-Phe | AIS only | AIS only |
| | 144 | CLUS_HUMAN | Val-Ala-Glu-Lys-Ala-Leu- Gln-Glu-Tyr-Arg-Lys-Lys- His-Arg-Glu-Glu | AIS only | AIS only |
| | 145 | CLUS_HUMAN | Phe-Thr-Arg-Glu-Pro- Gln-Asp-Thr-Tyr-His-Tyr- Leu-Pro | AIS only | AIS only |
| | 146 | CLUS_HUMAN | Arg-Pro-His-Phe-Phe- Phe-Pro | AIS only | AIS only |
| | 147 | CLUS_HUMAN | Arg-Pro-His-Phe-Phe- Phe-Pro-Lys-Ser-Arg-Ile- Val | AIS only | AIS only |
| | 148 | A1AG2_HUMAN | Phe-Leu-Arg-Asp-Thr- Lys-Thr-Leu | AIS only | AIS only |
| | 149 | ITIH2_HUMAN | Val-Ile-Glu-Pro-Gln-Gly- Leu-Arg-Phe | AIS only | AIS only |
| | 150 | ITIH1_HUMAN | Phe-Val-Thr-Pro-Leu- Thr-Ser-Met | AIS only | AIS only |
| | 151 | HBB_HUMAN | Val-His-Leu-Thr-Pro- Glu-Glu-Lys-Ser | AIS only | AIS only |
| | 152 | HBB_HUMAN | Trp-Gly-Lys-Val-Asn-Val- Asp-Glu-Val-Gly-Gly- Glu-Ala-Leu-Gly-Arg-Leu | AIS only | AIS only |
| | 153 | LG3BP_HUMAN | Arg-Gly-Pro-Leu-Val-Lys- Tyr-Ser-Ser-Asp-Tyr | AIS only | AIS only |
| | 154 | ECM1_HUMAN | Ala-Ser-Glu-Gly-Gly- Phe-Thr-Ala-Thr-Gly- Gln-Arg | AIS only | AIS only |

Hence, in table 7, the following 32 peptides: Val-Ser-Glu-Thr-Glu-Ser-Arg-Gly-Ser-Glu-Ser-Gly-Ile-Phe-Thr-Asn-Thr-Lys, His-Arg-His-Pro-Asp-Glu-Ala-Ala-Phe-Phe-Asp-Thr-Ala-Ser-Thr, Phe-Phe-Asp-Thr-Ala-Ser-Thr-Gly-Lys, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Ala-Ala-Thr-Val-Gly-Ser-Leu-Ala-Gly-Gln-Pro-Leu-Gln-Glu-Arg, Pro-Gly-Ser-Thr-Gly-Thr-Trp-Asn-Pro-Gly-Ser-Ser-Glu-Arg, His-Asp-Trp-Gly-His-Glu-Lys-Gln-Arg, His-Pro-Asp-Glu-Ala-Ala-Phe-Phe-Asp-Thr-Ala-Ser-Thr-Gly-Lys, Ser-Ser-His-His-Pro-Gly-Ile-Ala-Glu-Phe-Pro-Ser-Arg, Glu-Ser-Ser-Ser-His-His-Pro-Gly-Ile-Ala-Glu-Phe-Pro-Ser-Arg, Ser-Gly-Asn-Val-Ser-Pro-Gly-Thr-Arg-Arg-Glu-Tyr-His-Thr-Glu-Lys, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Leu-Asp-Asp-Asp-Leu-Glu-His-Gln-Gly-Gly-His-Val-Leu-Asp-His-Gly-His, Phe-Val-Ser-Glu-Thr-Glu-Ser-Arg-Gly-Ser-Glu-Ser-Gly-Ile-Phe-Thr-Asn-Thr, Arg-Glu-Tyr-His-Thr-Glu-Lys-Leu-Val-Thr-Ser, Gly-Ser-Ser-Gly-Thr-Gly-Gly-Thr-Ala-Thr-Trp-Lys, Gly-Ser-Trp-Asn-Ser-Gly-Ser-Ser-Gly-Pro-Gly-Ser-Thr-Gly-Asn-Arg, Glu-Leu-Glu-Arg-Pro-Gly-Gly-Asn-Glu-Ile, Leu-Gly-Glu-Phe-Val-Ser-Glu-Thr-Glu-Ser-Arg, Glu-Leu-Glu-Arg-Pro-Gly-Gly-Asn-Glu, Asp-Glu-Ala-Gly-Ser-Glu-Ala-Asp-His-Glu-Gly-Thr-His-Ser-Thr, Asp-Glu-Ala-Ala-Phe-Phe-Asp-Thr-Ala-Ser-Thr-Gly-Lys, Met-Ala-Asp-Glu-Ala-Gly-Ser-Glu-Ala-Asp-His-Glu-Gly-Thr-His-Ser-Thr-Lys, Arg-Glu-Glu-Ala-Pro-Ser-Leu-Arg, Glu-Glu-Val-Ser-Gly-Asn-Val-Ser-Pro-Gly-Thr, Tyr-Gln-Glu-Pro-Val-Leu-Gly-Pro-Val, Ser-Glu-Ser-Gly-Ile-Phe-Thr-Asn-Thr-Lys, His-Arg-His-Pro-Asp-Glu-Ala-Ala-Phe-Phe-Asp-Thr-Ala are quantitative peptides that were increased at least 30 folds in AIS patients as compared with individuals without stroke and patients with ICH and the differences were statistically significant (adjusted p value ≤ 0.05 and fold change ≥ 30).

**Table 7. The serum peptide pattern 2 for diagnostics of AIS and differential diagnosis of AIS from ICH. List of AIS diagnostic peptides with amino acid sequence, adjusted p value, and fold change is given.**

| | Serial number | Source protein identifier* | Peptide sequence | Adjusted P value** | Fold change*** |
|---|---|---|---|---|---|
| Quantitative peptides | 1 | FIBA_HUMAN | Val-Ser-Glu-Thr-Glu-Ser-Arg- Gly-Ser-Glu-Ser-Gly-Ile-Phe- Thr-Asn-Thr-Lys | 8.61E-05 | 1765.5 |
| | 2 | FIBA_HUMAN | His-Arg-His-Pro-Asp-Glu-Ala- Ala-Phe-Phe-Asp-Thr-Ala-Ser- Thr | 0.000135 | 33.2 |
| | 3 | FIBA_HUMAN | Phe-Phe-Asp-Thr-Ala-Ser-Thr- Gly-Lys | 0.000452 | 207.9 |
| | 4 | FIBA_HUMAN | Gly-Ser-Glu-Ser-Gly-Ile-Phe- Thr-Asn-Thr-Lys | 0.00134 | 782.3 |
| | 5 | FIBA_HUMAN | Asn-Pro-Gly-Ser-Ser-Gly-Thr- Gly-Gly-Thr-Ala-Thr-Trp-Lys | 0.00134 | 215.2 |
| | 6 | FIBA_HUMAN | Gln-Phe-Thr-Ser-Ser-Thr-Ser- Tyr-Asn-Arg | 0.00134 | 154.9 |
| | 7 | FIBB_HUMAN | Ala-Pro-Pro-Pro-Ile-Ser-Gly- Gly-Gly-Tyr | 0.00134 | 116.7 |
| | 8 | APOE_HUMAN | Ala-Ala-Thr-Val-Gly-Ser-Leu- Ala-Gly-Gln-Pro-Leu-Gln-Glu- Arg | 0.00134 | 46.9 |
| | 9 | FIBA_HUMAN | Pro-Gly-Ser-Thr-Gly-Thr-Trp- Asn-Pro-Gly-Ser-Ser-Glu-Arg | 0.001509 | 199.9 |
| | 10 | KNG1_HUMAN | His-Asp-Trp-Gly-His-Glu-Lys- Gln-Arg | 0.00162 | 32 |
| | 11 | FIBA_HUMAN | His-Pro-Asp-Glu-Ala-Ala-Phe- Phe-Asp-Thr-Ala-Ser-Thr-Gly- Lys | 0.003056 | 97.8 |
| | 12 | FIBA_HUMAN | Ser-Ser-His-His-Pro-Gly-Ile- Ala-Glu-Phe-Pro-Ser-Arg | 0.003239 | 38.1 |
| | 13 | FIBA_HUMAN | Glu-Ser-Ser-Ser-His-His-Pro- Gly-Ile-Ala-Glu-Phe-Pro-Ser- Arg | 0.003319 | 141.6 |
| | 14 | FIBA_HUMAN | Ser-Gly-Asn-Val-Ser-Pro-Gly- Thr-Arg-Arg-Glu-Tyr-His-Thr- Glu-Lys | 0.003789 | 32.8 |
| | 15 | FIBA_HUMAN | Pro-Gly-Ser-Ser-Gly-Pro-Gly- Ser-Thr-Gly-Ser-Trp-Asn-Ser- Gly-Ser-Ser-Gly-Thr-Gly-Ser- Thr-Gly-Asn-Gln-Asn-Pro-Gly- Ser-Pro-Arg | 0.003798 | 206.2 |
| | 16 | FIBA_HUMAN | Thr-Ser-Lys-Gly-Asp-Lys-Glu- Leu | 0.003798 | 198.4 |
| | 17 | KNG1_HUMAN | Leu-Asp-Asp-Asp-Leu-Glu-His- Gln-Gly-Gly-His-Val-Leu-Asp- His-Gly-His | 0.003798 | 96.1 |
| | 18 | FIBA_HUMAN | Phe-Val-Ser-Glu-Thr-Glu-Ser- Arg-Gly-Ser-Glu-Ser-Gly-Ile- Phe-Thr-Asn-Thr | 0.005196 | 41.2 |
| | 19 | FIBA_ HUMAN | Arg-Glu-Tyr-His-Thr-Glu-Lys- Leu-Val-Thr-Ser | 0.006342 | 125.3 |
| | 20 | FIBA_ HUMAN | Gly-Ser-Ser-Gly-Thr-Gly-Gly- Thr-Ala-Thr-Trp-Lys | 0.012001 | 307 |
| | 21 | FIBA_HUMAN | Gly-Ser-Trp-Asn-Ser-Gly-Ser- Ser-Gly-Pro-Gly-Ser-Thr-Gly- Asn-Arg | 0.012001 | 74 |
| | 22 | FIBA_HUMAN | Glu-Leu-Glu-Arg-Pro-Gly-Gly- Asn-Glu-Ile | 0.015039 | 198.9 |
| | 23 | FIBA_HUMAN | Leu-Gly-Glu-Phe-Val-Ser-Glu- Thr-Glu-Ser-Arg | 0.015039 | 79.3 |
| | 24 | FIBA_HUMAN | Glu-Leu-Glu-Arg-Pro-Gly-Gly- Asn-Glu | 0.015298 | 84.8 |
| | 25 | FIBA_HUMAN | Asp-Glu-Ala-Gly-Ser-Glu-Ala- Asp-His-Glu-Gly-Thr-His-Ser- Thr | 0.015298 | 49 |
| | 26 | FIBA_HUMAN | Asp-Glu-Ala-Ala-Phe-Phe-Asp- Thr-Ala-Ser-Thr-Gly-Lys | 0.016406 | 57.1 |
| | 27 | FIBA_HUMAN | Met-Ala-Asp-Glu-Ala-Gly-Ser- Glu-Ala-Asp-His-Glu-Gly-Thr- His-Ser-Thr-Lys | 0.01677 | 43.3 |
| | 28 | FIBB_HUMAN | Arg-Glu-Glu-Ala-Pro-Ser-Leu- Arg | 0.02059 | 350 |
| | 29 | FIBA_HUMAN | Glu-Glu-Val-Ser-Gly-Asn-Val- Ser-Pro-Gly-Thr | 0.020618 | 114.7 |
| | 30 | CASB_HUMAN | Tyr-Gln-Glu-Pro-Val-Leu-Gly- Pro-Val | 0.024791 | 59.5 |
| | 31 | FIBA_ HUMAN | Ser-Glu-Ser-Gly-Ile-Phe-Thr- Asn-Thr-Lys | 0.034075 | 151.8 |
| | 32 | FIBA_HUMAN | His-Arg-His-Pro-Asp-Glu-Ala- Ala-Phe-Phe-Asp-Thr-Ala | 0.038093 | 61.7 |

Hence, in table 8, the following 24 peptides: Val-Ser-Glu-Thr-Glu-Ser-Arg-Gly-Ser-Glu-Ser-Gly-Ile-Phe-Thr-Asn-Thr-Lys, Phe-Phe-Asp-Thr-Ala-Ser-Thr-Gly-Lys, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Pro-Gly-Ser-Thr-Gly-Thr-Trp-Asn-Pro-Gly-Ser-Ser-Glu-Arg, His-Pro-Asp-Glu-Ala-Ala-Phe-Phe-Asp-Thr-Ala-Ser-Thr-Gly-Lys, Glu-Ser-Ser-Ser-His-His-Pro-Gly-Ile-Ala-Glu-Phe-Pro-Ser-Arg, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Leu-Asp-Asp-Asp-Leu-Glu-His-Gln-Gly-Gly-His-Val-Leu-Asp-His-Gly-His, Arg-Glu-Tyr-His-Thr-Glu-Lys-Leu-Val-Thr-Ser, Gly-Ser-Ser-Gly-Thr-Gly-Gly-Thr-Ala-Thr-Trp-Lys, Gly-Ser-Trp-Asn-Ser-Gly-Ser-Ser-Gly-Pro-Gly-Ser-Thr-Gly-Asn-Arg, Glu-Leu-Glu-Arg-Pro-Gly-Gly-Asn-Glu-Ile, Leu-Gly-Glu-Phe-Val-Ser-Glu-Thr-Glu-Ser-Arg, Glu-Leu-Glu-Arg-Pro-Gly-Gly-Asn-Glu, Asp-Glu-Ala-Ala-Phe-Phe-Asp-Thr-Ala-Ser-Thr-Gly-Lys, Arg-Glu-Glu-Ala-Pro-Ser-Leu-Arg, Glu-Glu-Val-Ser-Gly-Asn-Val-Ser-Pro-Gly-Thr, Tyr-Gln-Glu-Pro-Val-Leu-Gly-Pro-Val, Ser-Glu-Ser-Gly-Ile-Phe-Thr-Asn-Thr-Lys, His-Arg-His-Pro-Asp-Glu-Ala-Ala-Phe- Phe-Asp-Thr-Ala are quantitative peptides that were increased at least 50 folds in AIS patients as compared with individuals without stroke and patients with ICH and the differences were statistically significant (adjusted p value ≤ 0.05 and fold change ≥ 50).

**Table 8. The serum peptide pattern 3 for diagnostics of AIS and differential diagnosis of AIS from ICH. List of AIS diagnostic peptides with amino acid sequence, adjusted p value, and fold change is given.**

| | Serial number | Source protein identifier* | Peptide sequence | Adjusted p value** | Fold change*** |
|---|---|---|---|---|---|
| Quantitative peptides | 1 | FIBA_HUMAN | Val-Ser-Glu-Thr-Glu-Ser-Arg- Gly-Ser-Glu-Ser-Gly-Ile-Phe- Thr-Asn-Thr-Lys | 8.61E-05 | 1765.5 |
| | 2 | FIBA_HUMAN | Phe-Phe-Asp-Thr-Ala-Ser- Thr-Gly-Lys | 0.000452 | 207.9 |
| | 3 | FIBA_HUMAN | Gly-Ser-Glu-Ser-Gly-Ile-Phe- Thr-Asn-Thr-Lys | 0.00134 | 782.3 |
| | 4 | FIBA_HUMAN | Asn-Pro-Gly-Ser-Ser-Gly-Thr- Gly-Gly-Thr-Ala-Thr-Trp-Lys | 0.00134 | 215.2 |
| | 5 | FIBA_HUMAN | Gln-Phe-Thr-Ser-Ser-Thr- Ser-Tyr-Asn-Arg | 0.00134 | 154.9 |
| | 6 | FIBB_HUMAN | Ala-Pro-Pro-Pro-Ile-Ser-Gly- Gly-Gly-Tyr | 0.00134 | 116.7 |
| | 7 | FIBA_HUMAN | Pro-Gly-Ser-Thr-Gly-Thr-Trp- Asn-Pro-Gly-Ser-Ser-Glu-Arg | 0.001509 | 199.9 |
| | 8 | FIBA_HUMAN | His-Pro-Asp-Glu-Ala-Ala- Phe-Phe-Asp-Thr-Ala-Ser- Thr-Gly-Lys | 0.003056 | 97.8 |
| | 9 | FIBA_HUMAN | Glu-Ser-Ser-Ser-His-His-Pro- Gly-Ile-Ala-Glu-Phe-Pro-Ser- Arg | 0.003319 | 141.6 |
| | 10 | FIBA_HUMAN | Pro-Gly-Ser-Ser-Gly-Pro-Gly- Ser-Thr-Gly-Ser-Trp-Asn-Ser- Gly-Ser-Ser-Gly-Thr-Gly-Ser- Thr-Gly-Asn-Gln-Asn-Pro- Gly-Ser-Pro-Arg | 0.003798 | 206.2 |
| | 11 | FIBA_HUMAN | Thr-Ser-Lys-Gly-Asp-Lys-Glu- Leu | 0.003798 | 198.4 |
| | 12 | KNG1_HUMAN | Leu-Asp-Asp-Asp-Leu-Glu- His-Gln-Gly-Gly-His-Val-Leu- Asp-His-Gly-His | 0.003798 | 96.1 |
| | 13 | FIBA_HUMAN | Arg-Glu-Tyr-His-Thr-Glu-Lys- Leu-Val-Thr-Ser | 0.006342 | 125.3 |
| | 14 | FIBA_HUMAN | Gly-Ser-Ser-Gly-Thr-Gly-Gly- Thr-Ala-Thr-Trp-Lys | 0.012001 | 307 |
| | 15 | FIBA_HUMAN | Gly-Ser-Trp-Asn-Ser-Gly-Ser- Ser-Gly-Pro-Gly-Ser-Thr-Gly- Asn-Arg | 0.012001 | 74 |
| | 16 | FIBA_HUMAN | Glu-Leu-Glu-Arg-Pro-Gly- Gly-Asn-Glu-Ile | 0.015039 | 198.9 |
| | 17 | FIBA_HUMAN | Leu-Gly-Glu-Phe-Val-Ser- Glu-Thr-Glu-Ser-Arg | 0.015039 | 79.3 |
| | 18 | FIBA_HUMAN | Glu-Leu-Glu-Arg-Pro-Gly- Gly-Asn-Glu | 0.015298 | 84.8 |
| | 19 | FIBA_HUMAN | Asp-Glu-Ala-Ala-Phe-Phe- Asp-Thr-Ala-Ser-Thr-Gly-Lys | 0.016406 | 57.1 |
| | 20 | FIBB_HUMAN | Arg-Glu-Glu-Ala-Pro-Ser- Leu-Arg | 0.02059 | 350 |
| | 21 | FIBA_HUMAN | Glu-Glu-Val-Ser-Gly-Asn-Val- Ser-Pro-Gly-Thr | 0.020618 | 114.7 |
| | 22 | CASB_HUMAN | Tyr-Gln-Glu-Pro-Val-Leu-Gly- Pro-Val | 0.024791 | 59.5 |
| | 23 | FIBA_HUMAN | Ser-Glu-Ser-Gly-Ile-Phe-Thr- Asn-Thr-Lys | 0.034075 | 151.8 |
| | 24 | FIBA_HUMAN | His-Arg-His-Pro-Asp-Glu-Ala- Ala-Phe-Phe-Asp-Thr-Ala | 0.038093 | 61.7 |

Hence, in table 9, the following 16 peptides: Val-Ser-Glu-Thr-Glu-Ser-Arg-Gly-Ser-Glu-Ser-Gly-Ile-Phe-Thr-Asn-Thr-Lys, Phe-Phe-Asp-Thr-Ala-Ser-Thr-Gly-Lys, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Pro-Gly-Ser-Thr-Gly-Thr-Trp-Asn-Pro-Gly-Ser-Ser-Glu-Arg, Glu-Ser-Ser-Ser-His-His-Pro-Gly-Ile-Ala-Glu-Phe-Pro-Ser-Arg, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Arg-Glu-Tyr-His-Thr-Glu-Lys-Leu-Val-Thr-Ser, Gly-Ser-Ser-Gly-Thr-Gly-Gly-Thr-Ala-Thr-Trp-Lys, Glu-Leu-Glu-Arg-Pro-Gly-Gly-Asn-Glu-Ile, Arg-Glu-Glu-Ala-Pro-Ser-Leu-Arg, Glu-Glu-Val-Ser-Gly-Asn-Val-Ser-Pro-Gly-Thr, Ser-Glu-Ser-Gly-Ile-Phe-Thr-Asn-Thr-Lys are quantitative peptides that were increased at least 100 folds in AIS patients as compared with individuals without stroke and patients with ICH and the differences were statistically significant (adjusted p value ≤ 0.05 and fold change ≥ 100).

**Table 9. The serum peptide pattern 4 for diagnostics of AIS and differential diagnosis of AIS from ICH. List of AIS diagnostic peptides with amino acid sequence, adjusted p value, and fold change is given.**

| | Serial number | Source protein identifier* | Peptide sequence | Adjusted p value** | Fold change*** |
|---|---|---|---|---|---|
| Quantitative peptides | 1 | FIBA_HUMAN | Val-Ser-Glu-Thr-Glu-Ser-Arg-Gly- Ser-Glu-Ser-Gly-Ile-Phe-Thr-Asn- Thr-Lys | 8.61E-05 | 1765.5 |
| | 2 | FIBA_HUMAN | Phe-Phe-Asp-Thr-Ala-Ser-Thr- Gly-Lys | 0.000452 | 207.9 |
| | 3 | FIBA_HUMAN | Gly-Ser-Glu-Ser-Gly-Ile-Phe-Thr- Asn-Thr-Lys | 0.00134 | 782.3 |
| | 4 | FIBA_HUMAN | Asn-Pro-Gly-Ser-Ser-Gly-Thr-Gly- Gly-Thr-Ala-Thr-Trp-Lys | 0.00134 | 215.2 |
| | 5 | FIBA_HUMAN | Gln-Phe-Thr-Ser-Ser-Thr-Ser- Tyr-Asn-Arg | 0.00134 | 154.9 |
| | 6 | FIBB_HUMAN | Ala-Pro-Pro-Pro-Ile-Ser-Gly-Gly- Gly-Tyr | 0.00134 | 116.7 |
| | 7 | FIBA_HUMAN | Pro-Gly-Ser-Thr-Gly-Thr-Trp- Asn-Pro-Gly-Ser-Ser-Glu-Arg | 0.001509 | 199.9 |
| | 8 | FIBA_HUMAN | Glu-Ser-Ser-Ser-His-His-Pro-Gly- Ile-Ala-Glu-Phe-Pro-Ser-Arg | 0.003319 | 141.6 |
| | 9 | FIBA_HUMAN | Pro-Gly-Ser-Ser-Gly-Pro-Gly-Ser- Thr-Gly-Ser-Trp-Asn-Ser-Gly-Ser- Ser-Gly-Thr-Gly-Ser-Thr-Gly-Asn- Gln-Asn-Pro-Gly-Ser-Pro-Arg | 0.003798 | 206.2 |
| | 10 | FIBA_HUMAN | Thr-Ser-Lys-Gly-Asp-Lys-Glu-Leu | 0.003798 | 198.4 |
| | 11 | FIBA_HUMAN | Arg-Glu-Tyr-His-Thr-Glu-Lys-Leu- Val-Thr-Ser | 0.006342 | 125.3 |
| | 12 | FIBA_HUMAN | Gly-Ser-Ser-Gly-Thr-Gly-Gly-Thr- Ala-Thr-Trp-Lys | 0.012001 | 307 |
| | 13 | FIBA_HUMAN | Glu-Leu-Glu-Arg-Pro-Gly-Gly- Asn-Glu-Ile | 0.015039 | 198.9 |
| | 14 | FIBB_HUMAN | Arg-Glu-Glu-Ala-Pro-Ser-Leu-Arg | 0.02059 | 350 |
| | 15 | FIBA_HUMAN | Glu-Glu-Val-Ser-Gly-Asn-Val-Ser- Pro-Gly-Thr | 0.020618 | 114.7 |
| | 16 | FIBA_HUMAN | Ser-Glu-Ser-Gly-Ile-Phe-Thr-Asn- Thr-Lys | 0.034075 | 151.8 |

Hence, in table 10, the following 1 peptide: Val-Ser-Glu-Thr-Glu-Ser-Arg-Gly-Ser-Glu-Ser-Gly-Ile-Phe-Thr-Asn-Thr-Lys was quantitative peptide that were increased at least 1000 folds in AIS patients as compared with individuals without stroke and patients with ICH and the differences were statistically significant (adjusted p value ≤ 0.05 and fold change ≥ 1000).

**Table 10. The serum peptide pattern 5 for diagnostics of AIS and differential diagnosis of AIS from ICH. List of AIS diagnostic peptides with amino acid sequence, adjusted p value, and fold change is given.**

| | Serial number | Source protein identifier* | Peptide sequence | Adjusted p value** | Fold change*** |
|---|---|---|---|---|---|
| Quantitative peptides | 1 | FIBA_HUMAN | Val-Ser-Glu-Thr-Glu-Ser-Arg- Gly-Ser-Glu-Ser-Gly-Ile-Phe- Thr-Asn-Thr-Lys | 8.61E-05 | 1765.5 |

### The advantages of invention are presented in the following tables

Table 6. The serum peptide pattern 1 for diagnostics of AIS and differential diagnosis of AIS from ICH. The peptides listed in Table 6 are qualitative and quantitative biomarkers of AIS since they distinguish patients with AIS from individuals without stroke and from patients with ICH. The fifty four qualitative markers (peptides 101-154 in table 5) are detectable only in patients with AIS, but not detectable in individuals without stroke and patients with ICG. Quantitative peptides (peptides 1-100 in table 6) are at least 2-folds increased in patients with AIS as compared with individuals without stroke and from patients with ICH. Their levels were determined with MS/MS (tandem mass spectrometry) signaling intensities. The upregulation of a particular peptide was determined by first integrating its peak area measured by a mass spectrometer across compared conditions here AIS and matched control groups (here individuals without stroke and patients with ICH). The integrated peak area reflects the number of peptide molecules in the measured sample. Next, by relative comparison, of integrated peak areas across the compared groups the fold-increase increase is calculated. Fold-increase reflects the ratio of integrated peak areas in compared conditions and is proportional to the ratio of number of the peptide molecules in compared conditions. In other words fold-increase shows how many times more the peptide is present in serum of the patients with AIS than in the individuals without stroke and patients with ICH. However, the method that we used (tandem mass spectrometry) for identification and quantification of the diagnostic peptides is not the limitation of the invention. Any other method for qualitative and quantitative peptide analysis can be used to identify and quantify these peptides.

In our analysis we have also considered the adjusted P value (adj. pval) which is the smallest significance level at which a particular comparison will be declared statistically significant as part of the multiple comparison such as AIS and respective control groups (individuals without stroke and patients with ICH). The threshold for selecting the peptides in the table 6 was as follow; adjusted P value (adj. pval) ≤ 0.05, fold-increase≥ 2 for the quantitative peptides.

Val-Ser-Glu-Thr-Glu-Ser-Arg-Gly-Ser-Glu-Ser-Gly-Ile-Phe-Thr-Asn-Thr-Lys, His-Arg-His-Pro-Asp-Glu-Ala-Ala-Phe-Phe-Asp-Thr-Ala-Ser-Thr, Phe-Phe-Asp-Thr-Ala-Ser-Thr-Gly-Lys, Gln-Leu-Gly-Leu-Pro-Gly-Pro-Pro-Asp-Val-Pro-Asp-His-Ala-Ala, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Ala-Ala-Thr-Val-Gly-Ser-Leu-Ala-Gly-Gln-Pro-Leu-Gln-Glu-Arg, Pro-Gly-Ser-Thr-Gly-Thr-Trp-Asn-Pro-Gly-Ser-Ser-Glu-Arg, His-Asp-Trp-Gly-His-Glu-Lys-Gln-Arg, His-Pro-Asp-Glu-Ala-Ala-Phe-Phe-Asp-Thr-Ala-Ser-Thr-Gly-Lys, Ser-Ser-His-His-Pro-Gly-Ile-Ala-Glu-Phe-Pro-Ser-Arg, Val-His-Leu-Thr-Pro-Glu-Glu-Lys-Ser-Ala-Val-Thr-Ala-Leu, Glu-Ser-Ser-Ser-His-His-Pro-Gly-Ile-Ala-Glu-Phe-Pro-Ser-Arg, Thr-Ala-Asn-Asp-Ser-Gly-Pro-Arg-Arg-Tyr-Thr-Ile-Ala-Ala-Leu, Arg-His-Asp-Trp-Gly-His-Glu-Lys-Gln-Arg, Val-Lys-Leu-Val-Asn-Glu-Val-Thr-Glu, Gln-Phe-Thr-Ser-Ser-Thr-Ser-Tyr-Asn, Ser-Gly-Asn-Val-Ser-Pro-Gly-Thr-Arg-Arg-Glu-Tyr-His-Thr-Glu-Lys, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Leu-Asp-Asp-Asp-Leu-Glu-His-Gln-Gly-Gly-His-Val-Leu-Asp-His-Gly-His, Ala-Phe-Arg-Gln-Pro-Ser-Ser-Ala, Phe-Val-Ser-Glu-Thr-Glu-Ser-Arg-Gly-Ser-Glu-Ser-Gly-Ile-Phe-Thr-Asn-Thr, Phe-Ser-Thr-Tyr-Asp-Arg-Asp-Asn-Asp-Gly-Trp, Thr-Val-Ile-Gln-Asn-Arg-Gln-Asp-Gly-Ser-Val-Asp-Phe, Arg-Glu-Tyr-His-Thr-Glu-Lys-Leu-Val-Thr-Ser, Gln-Ala-Gly-Ala-Ala-Gly-Ser-Arg-Met-Asn-Phe-Arg-Pro-Gly-Val-Leu-Ser-Ser, Ser-Pro-Gly-Ser-Gly-Asn-Ala-Arg-Pro-Asn-Asn-Pro-Asp-Trp-Gly-Thr-Phe, Ser-Asp-Ile-Ser-Ser-Thr-Arg-Gly-Phe-Pro-Ser-Val-Leu-Arg, Ile-Thr-His-Arg-Ile-His-Trp-Glu-Ser-Ala-Ser-Leu-Leu, Val-Ser-Gly-Trp-Gly-Arg-Asn-Ala-Asn-Phe, Phe-Asp-Thr-Ala-Ser-Thr-Gly-Lys-Thr-Phe-Pro-Gly-Phe, Ser-Ala-Gly-Ser-Trp-Asn-Ser-Gly-Ser-Ser-Gly-Pro-Gly-Ser-Thr-Gly-Asn-Arg-Asn, Val-His-Leu-Thr-Pro-Glu-Glu-Lys, Asp-Ala-Val-Arg-Gly-Ser-Pro-Ala-Ile-Asn-Val, Asp-Glu-Ala-Ala-Phe-Phe-Asp-Thr, Val-His-Leu-Thr-Pro-Glu-Glu-Lys-Ser-Ala-Val-Thr, Pro-Gly-Ser-Pro-Arg-Pro-Gly-Ser-Thr-Gly-Thr-Trp-Asn-Pro-Gly-Ser-Ser-Glu-Arg, Gly-Ser-Ser-Gly-Thr-Gly-Gly-Thr-Ala-Thr-Trp-Lys, Gly-Ser-Trp-Asn-Ser-Gly-Ser-Ser-Gly-Pro-Gly-Ser-Thr-Gly-Asn-Arg, Ser-Glu-Thr-Glu-Ser-Arg-Gly-Ser-Glu-Ser-Gly-Ile-Phe-Thr-Asn-Thr-Lys, Glu-Leu-Glu-Arg-Pro-Gly-Gly-Asn-Glu-Ile, Leu-Gly-Glu-Phe-Val-Ser-Glu-Thr-Glu-Ser-Arg, Ala-Leu-Glu-Glu-Tyr-Thr-Lys-Lys-Leu-Asn-Thr-Gln, Glu-Leu-Glu-Arg-Pro-Gly-Gly-Asn-Glu, Asp-Glu-Ala-Gly-Ser-Glu-Ala-Asp-His-Glu-Gly-Thr-His-Ser-Thr, Arg-His-Asp-Trp-Gly-His-Glu-Lys, Ala-Ala-Ser-Arg-Tyr-Leu-Asp-Lys-Thr-Glu-Gln, Arg-His-Pro-Asp-Glu-Ala-Ala-Phe, Pro-Asn-Ile-Leu-Arg-Leu-Glu-Ser-Glu-Glu-Thr, Ile-Glu-Gly-Val-Asp-Ala-Glu-Asp-Gly-His-Gly-Pro-Gly-Glu-Gln-Gln-Lys, Pro-Ser-Leu-Arg-Pro-Ala-Pro-Pro-Pro-Ile-Ser-Gly-Gly-Gly-Tyr, Thr-Asn-Thr-Lys-Glu-Ser-Ser-Ser-His-His-Pro-Gly-Ile-Ala-Glu-Phe-Pro-Ser-Arg-Gly, Ala-Glu-Val-Gly-Arg-Val-Gly-Tyr-Val-Ser-Gly-Trp-Gly-Arg-Asn-Ala-Asn-Phe, Asp-Glu-Ala-Ala-Phe-Phe-Asp-Thr-Ala-Ser-Thr-Gly-Lys, Lys-Val-Glu-Gln-Ala-Val-Glu-Thr-Glu-Pro-Glu-Pro-Glu-Leu-Arg-Gln-Gln-Thr, Met-Ala-Asp-Glu-Ala-Gly-Ser-Glu-Ala-Asp-His-Glu-Gly-Thr-His-Ser-Thr-Lys, Ser-Gly-Pro-Ser-Ser-Pro-Gln-Gln-Ala-Pro-Pro-Ala-Val-His, Gly-Ser-Glu-Ser-Gly-Ile-Phe-Thr-Asn-Thr-Lys-Glu-Ser-Ser-Ser-His-His-Pro-Gly-Ile-Ala-Glu-Phe-Pro-Ser-Arg-Gly, Arg-Glu-Glu-Ala-Pro-Ser-Leu-Arg, Glu-Glu-Val-Ser-Gly-Asn-Val-Ser-Pro-Gly-Thr, Glu-Asn-Glu-Gly-Phe-Thr-Val-Thr-Ala-Glu-Gly-Lys, Val-Thr-Glu-Pro-Ile-Ser-Ala-Glu-Ser-Gly-Glu-Gln-Val-Glu-Arg, Gly-Asp-Asp-Pro-Ser-Asp-Lys-Phe, Gly-Pro-Pro-Asp-Val-Pro-Asp-His-Ala, Glu-Glu-Val-Ser-Gly-Asn-Val-Ser-Pro-Gly-Thr-Arg-Arg-Glu, Tyr-His-Thr-Glu-Lys-Leu-Val, Tyr-Gln-Glu-Pro-Val-Leu-Gly-Pro-Val, Leu-Leu-Val-Arg-Tyr-Thr-Lys-Lys-Val-Pro-Gln-Val-Ser-Thr-Pro-Thr-Leu, Ile-Gln-Ile-Tyr-Lys-Lys-Leu-Arg-Thr-Ser-Ser-Phe, Ser-Val-Ser-Ala-Gly-Ser-Pro-His-Pro-Ala-Ile-Ala-Arg-Leu, His-Pro-Gly-Ile-Ala-Glu-Phe-Pro-Ser-Arg-Gly, Thr-Phe-Glu-Ser-Lys-Ser-Tyr, Ile-Glu-Gly-Val-Asp-Ala-Glu-Asp-Gly-His-Gly-Pro-Gly-Glu-Gln-Gln-Lys-Arg, Val-Leu-Val-Pro-Ala-Gly-Ser-Ala-Arg-Pro-Val-Ala-Phe, Asp-Ala-His-Lys-Ser-Glu-Val-Ala-His-Arg-Phe-Lys-Asp-Leu-Gly-Glu-Glu-Asn-Phe, Lys-Val-Lys-Glu-Lys-Leu-Lys-Ile-Asp-Ser, Glu-Phe-Val-Ser-Glu-Thr-Glu-Ser, His-Ala-Leu-Gln-Leu-Asn-Asn-Arg-Gln, Ser-Glu-Ser-Gly-Ile-Phe-Thr-Asn-Thr-Lys, Tyr-Val-Gly-Lys-Lys-Gln-Leu, Leu-Thr-Gln-Val-Lys-Glu-Ser-Leu-Ser-Ser-Tyr, Ser-Ser-His-His-Pro-Gly-Ile-Ala-Glu-Phe-Pro-Ser, Gly-Phe-Tyr-Glu-Ser-Asp-Val-Met-Gly-Arg, Gly-Gly-His-Leu-Asp-Ala-Lys-Gly-Ser-Phe, Asp-Val-Leu-Lys-Asp-Ser-Gly-Arg-Asp, His-Arg-His-Pro-Asp-Glu-Ala-Ala-Phe-Phe-Asp-Thr-Ala, Trp-Ser-Thr-Val-Lys-Asp-Lys-Phe-Ser-Glu-Phe, Arg-Gly-Ser-Glu-Ser-Gly-Ile-Phe-Thr-Asn-Thr, Glu-Glu-Ala-Pro-Ser-Leu-Arg-Pro-Ala-Pro-Pro-Pro-Ile-Ser-Gly-Gly-Gly-Tyr-Arg, Gly-Thr-Phe-Glu-Glu-Val-Ser-Gly-Asn-Val-Ser-Pro-Gly-Thr-Arg-Arg-Glu-Tyr-His-Thr-Glu, Gly-Ser-Ser-Gly-Thr-Gly-Gly-Thr-Ala-Thr-Trp-Lys-Pro-Gly-Ser-Ser, Ser-Ser-Ser-Tyr-Ser-Lys-Gln-Phe-Thr-Ser-Ser-Thr-Ser-Tyr-Asn-Arg-Gly-Asp-Ser-Thr-Phe-Glu-Ser-Lys-Ser-Tyr-Lys, Trp-Glu-Ser-Ala-Lys-Thr-Ala-Ala-Gln-Asn-Leu, Phe-Ser-Lys-Ala-Leu-Pro-Arg, Pro-Ser-Glu-Leu-Arg-Asp-Glu-His, Val-Val-Phe-Thr-Ala-Asn-Asp-Ser-Gly-Pro-Arg-Arg-Tyr-Thr-Ile-Ala, Leu-Gly-Glu-Phe-Val-Ser-Glu-Thr-Glu-Ser-Arg were quantified in serum samples from patients with AIS and in serum samples of the individuals without stroke and serum samples of patients with ICH and were at least 2 folds increased (≥ 2 folds) in serum samples of patients with AIS as compared with serum samples of individuals without stroke and patients with ICH . Simultaneously the serum peptide pattern 1 comprises 54 qualitative peptides Asp-Asp-Tyr-Val-Asn-Thr-Gln-Gly-Ala-Ser-Leu, Tyr-Pro-Trp-Thr-Gln-Arg-Phe, Phe-Arg-Leu-Leu-Gly-Asn-Val-Leu, Tyr-Gln-Lys-Val-Val-Ala-Gly-Val-Ala-Asn-Ala-Leu-Ala-His-Lys-Tyr-His, Phe-Ser-Glu-Thr-Phe-Gln-Lys-Val-Lys-Glu-Lys-Leu-Lys-Ile-Asp-Ser, Val-Lys-Glu-Lys-Leu-Lys-Ile-Asp-Ser, Tyr-Glu-Lys-Thr-Tyr-Leu-Pro-Ala-Val-Asp-Glu-Lys-Leu, Thr-Gln-Gln-Pro-Gln-Gln-Asp-Glu-Met-Pro-Ser-Pro-Thr-Phe-Leu, Thr-Gly-Ile-Phe-Thr-Asp-Gln-Val-Leu, Ser-Thr-Val-Lys-Asp-Lys-Phe-Ser-Glu-Phe, Tyr-Trp-Ser-Thr-Val-Lys-Asp-Lys-Phe, Tyr-Trp-Ser-Thr-Val-Lys-Asp-Lys-Phe-Ser-Glu-Phe, His-Thr-Asp-Leu-Ser-Gly-Lys-Val, His-Thr-Asp-Leu-Ser-Gly-Lys-Val-Phe-Val-Phe, Tyr-Val-Ile-Ile-Lys-Pro-Leu-Val-Trp-Val, Ala-Val-Arg-Gly-Ser-Pro-Ala-Ile-Asn, Glu-Val-Val-Phe-Thr-Ala-Asn-Asp-Ser-Gly-Pro-Arg-Arg-Tyr-Thr-Ile-Ala, Glu-Val-Val-Phe-Thr-Ala-Asn-Asp-Ser-Gly-Pro-Arg-Arg-Tyr-Thr-Ile-Ala-Ala, Phe-Thr-Ala-Asn-Asp-Ser-Gly-Pro-Arg-Arg-Tyr-Thr-Ile-Ala-Ala, Phe-Val-Glu-Gly-Ile-Tyr-Lys-Val-Glu, Leu-Leu-Ser-Pro-Tyr-Ser-Tyr-Ser-Thr-Thr-Ala, Leu-Ser-Pro-Tyr-Ser-Tyr-Ser-Thr-Thr-Ala, Thr-Ala-Asn-Asp-Ser-Gly-Pro-Arg-Arg-Tyr-Thr-Ile-Ala-Ala-Leu-Leu-Ser-Pro-Tyr-Ser-Tyr-Ser-Thr-Thr-Ala, Val-Val-Phe-Thr-Ala-Asn-Asp-Ser-Gly-Pro-Arg-Arg-Tyr-Thr-Ile-Ala-Ala, Phe-Lys-Asp-Ser-Ala-His-Gly-Phe, Phe-Ser-Ser-Pro-His-Gly-Lys-Asp-Leu-Leu-Phe-Lys-Asp-Ser-Ala-His-Gly-Phe, Asn-Gln-Ala-Gln-Glu-His-Phe-Gly-Lys-Asp-Lys-Ser-Lys-Glu-Phe-Gln-Leu, Tyr-Ala-Val-Ala-Val-Val-Lys-Lys-Asp, Tyr-Ala-Val-Ala-Val-Val-Lys-Lys-Asp-Ser, Tyr-Val-Thr-Ala-Ile-Arg-Asn-Leu-Arg-Glu, Ser-Lys-Glu-Asp-Pro-Gln-Thr-Phe-Tyr-Tyr-Ala-Val-Ala, Tyr-Gly-Ser-Lys-Glu-Asp-Pro-Gln-Thr-Phe, Ala-Tyr-Leu-Ala-Pro-Asn-Asn-Leu-Lys-Pro-Val-Val-Ala-Glu, Ala-Tyr-Leu-Ala-Pro-Asn-Asn-Leu-Lys-Pro-Val-Val-Ala-Glu-Phe, Ala-Tyr-Leu-Ala-Pro-Asn-Asn-Leu-Lys-Pro-Val-Val-Ala-Glu-Phe-Tyr-Gly-Ser-Lys-Glu-Asp, Asp-Ala-Gly-Leu-Val-Tyr-Asp-Ala-Tyr, Asp-Ala-Tyr-Leu-Ala-Pro-Asn-Asn-Leu-Lys-Pro-Val-Val-Ala-Glu-Phe, Tyr-Leu-Ala-Pro-Asn-Asn-Leu-Lys-Pro-Val-Val-Ala-Glu, Tyr-Leu-Ala-Pro-Asn-Asn-Leu-Lys-Pro-Val-Val-Ala-Glu-Phe, Tyr-Leu-Ala-Pro-Asn-Asn-Leu-Lys-Pro-Val-Val-Ala-Glu-Phe-Tyr-Gly-Ser-Lys-Glu-Asp, Ser-Ala-Leu-Thr-Ser-Asp-Asn-His-Gly-Ala-Thr-Tyr, Ser-Ala-Leu-Thr-Ser-Asp-Asn-His-Gly-Ala-Thr-Tyr-Ala-Phe-Ser-Gly-Thr-His-Tyr, Phe-Arg-Gln-Gly-His-Asn-Ser-Val-Phe, Val-Ala-Glu-Lys-Ala-Leu-Gln-Glu-Tyr-Arg-Lys-Lys-His-Arg-Glu-Glu, Phe-Thr-Arg-Glu-Pro-Gln-Asp-Thr-Tyr-His-Tyr-Leu-Pro, Arg-Pro-His-Phe-Phe-Phe-Pro, Arg-Pro-His-Phe-Phe-Phe-Pro-Lys-Ser-Arg-Ile-Val, Phe-Leu-Arg-Asp-Thr-Lys-Thr-Leu, Val-Ile-Glu-Pro-Gln-Gly-Leu-Arg-Phe, Phe-Val-Thr-Pro-Leu-Thr-Ser-Met, Val-His-Leu-Thr-Pro-Glu-Glu-Lys-Ser, Trp-Gly-Lys-Val-Asn-Val-Asp-Glu-Val-Gly-Gly-Glu-Ala-Leu-Gly-Arg-Leu, Arg-Gly-Pro-Leu-Val-Lys-Tyr-Ser-Ser-Asp-Tyr, Ala-Ser-Glu-Gly-Gly-Phe-Thr-Ala-Thr-Gly-Gln-Arg that were detectable only in serum of the patients with AIS but were not detectable in serum samples of the individuals without stroke and serum samples of patients with ICH.

Table 7. The serum peptide pattern 2 for diagnostics of and differential diagnosis of AIS from ICH. The peptides listed in Table 7 are quantitative biomarkers of AIS since they distinguish patients with AIS from individuals without stroke and from patients with ICH. The quantitative peptides listed in table 7 are at least 30-folds increased in patients with AIS as compared with individuals without stroke and from patients with ICH. Their levels were determined with MS/MS (tandem mass spectrometry) signaling intensities. The upregulation of a particular peptide was determined by first integrating its peak area measured by a mass spectrometer across compared conditions here AIS and matched control groups (here individuals without stroke and patients with ICH). The integrated peak area reflects the number of peptide molecules in the measured sample. Next, by relative comparison, of integrated peak areas across the compared groups the fold-increase increase is calculated. Fold-increase reflects the ratio of integrated peak areas in compared conditions and is proportional to the ratio of number of the peptide molecules in compared conditions. In other words fold-increase shows how many times more the peptide is present in serum of the patients with AIS than in the individuals without stroke and patients with ICH. However, the method that we used (tandem mass spectrometry) for quantification of the diagnostic peptides is not the limitation of the invention. Any other method for quantitative peptide analysis can be used to measure the increase in serum levels of these quantitative peptides in patients with AIS.

In our analysis we have also considered the adjusted P value (adj. pval) which is the smallest significance level at which a particular comparison will be declared statistically significant as part of the multiple comparison such as between patients with AIS and respective control groups (individuals without stroke and patients with ICH). The threshold for selecting the peptides in the table 7 was as follow; adjusted P value (adj. pval) ≤ 0.05, fold-increase≥ 30. In the serum peptide pattern 2 for diagnostics of AIS and differential diagnosis of AIS from ICH the following 32 peptides: Val-Ser-Glu-Thr-Glu-Ser-Arg-Gly-Ser-Glu-Ser-Gly-Ile-Phe-Thr-Asn-Thr-Lys, His-Arg-His-Pro-Asp-Glu-Ala-Ala-Phe-Phe-Asp-Thr-Ala-Ser-Thr, Phe-Phe-Asp-Thr-Ala-Ser-Thr-Gly-Lys, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Ala-Ala-Thr-Val-Gly-Ser-Leu-Ala-Gly-Gln-Pro-Leu-Gln-Glu-Arg, Pro-Gly-Ser-Thr-Gly-Thr-Trp-Asn-Pro-Gly-Ser-Ser-Glu-Arg, His-Asp-Trp-Gly-His-Glu-Lys-Gln-Arg, His-Pro-Asp-Glu-Ala-Ala-Phe-Phe-Asp-Thr-Ala-Ser-Thr-Gly-Lys, Ser-Ser-His-His-Pro-Gly-Ile-Ala-Glu-Phe-Pro-Ser-Arg, Glu-Ser-Ser-Ser-His-His-Pro-Gly-Ile-Ala-Glu-Phe-Pro-Ser-Arg, Ser-Gly-Asn-Val-Ser-Pro-Gly-Thr-Arg-Arg-Glu-Tyr-His-Thr-Glu-Lys, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Leu-Asp-Asp-Asp-Leu-Glu-His-Gln-Gly-Gly-His-Val-Leu-Asp-His-Gly-His, Phe-Val-Ser-Glu-Thr-Glu-Ser-Arg-Gly-Ser-Glu-Ser-Gly-Ile-Phe-Thr-Asn-Thr, Arg-Glu-Tyr-His-Thr-Glu-Lys-Leu-Val-Thr-Ser, Gly-Ser-Ser-Gly-Thr-Gly-Gly-Thr-Ala-Thr-Trp-Lys, Gly-Ser-Trp-Asn-Ser-Gly-Ser-Ser-Gly-Pro-Gly-Ser-Thr-Gly-Asn-Arg, Glu-Leu-Glu-Arg-Pro-Gly-Gly-Asn-Glu-Ile, Leu-Gly-Glu-Phe-Val-Ser-Glu-Thr-Glu-Ser-Arg, Glu-Leu-Glu-Arg-Pro-Gly-Gly-Asn-Glu, Asp-Glu-Ala-Gly-Ser-Glu-Ala-Asp-His-Glu-Gly-Thr-His-Ser-Thr, Asp-Glu-Ala-Ala-Phe-Phe-Asp-Thr-Ala-Ser-Thr-Gly-Lys, Met-Ala-Asp-Glu-Ala-Gly-Ser-Glu-Ala-Asp-His-Glu-Gly-Thr-His-Ser-Thr-Lys, Arg-Glu-Glu-Ala-Pro-Ser-Leu-Arg, Glu-Glu-Val-Ser-Gly-Asn-Val-Ser-Pro-Gly-Thr, Tyr-Gln-Glu-Pro-Val-Leu-Gly-Pro-Val, Ser-Glu-Ser-Gly-Ile-Phe-Thr-Asn-Thr-Lys, His-Arg-His-Pro-Asp-Glu-Ala-Ala-Phe-Phe-Asp-Thr-Ala were quantified in serum samples from patients with AIS and in serum samples of the individuals without stroke and serum samples of patients with ICH and were at least 30 folds increased (≥ 30 folds) in serum samples of patients with AIS as compared with serum samples from individuals without stroke and patients with ICH .

Table 8. The serum peptide pattern 3 for diagnostics of AIS and differential diagnosis of AIS from ICH. The peptides listed in Table 8 are quantitative biomarkers of AIS since they distinguish patients with AIS from individuals without stroke and from patients with ICH. The quantitative peptides listed in table 8 are at least 50-folds increased in patients with AIS as compared with individuals without stroke and from patients with ICH. Their levels were determined with MS/MS (tandem mass spectrometry) signaling intensities. The upregulation of a particular peptide was determined by first integrating its peak area measured by a mass spectrometer across compared conditions here AIS and matched control groups (here individuals without stroke and patients with ICH). The integrated peak area reflects the number of peptide molecules in the measured sample. Next, by relative comparison, of integrated peak areas across the compared groups the fold-increase increase is calculated. Fold-increase reflects the ratio of integrated peak areas in compared conditions and is proportional to the ratio of number of the peptide molecules in compared conditions. In other words, fold-increase shows how many times more the peptide is present in serum of the patients with AIS than in the individuals without stroke and patients with ICH. However, the method that we used (tandem mass spectrometry) for quantification of the diagnostic peptides is not the limitation of the invention. Any other method for quantitative peptide analysis can be used to measure the increase in serum levels of these quantitative peptides in patients with AIS.

In our analysis we have also considered the adjusted P value (adj. pval) which is the smallest significance level at which a particular comparison will be declared statistically significant as part of the multiple comparison such as between patients with AIS and respective control groups (individuals without stroke and patients with ICH). The threshold for selecting the peptides in the table 8 was as follow; adjusted P value (adj. pval) ≤ 0.05, fold-increase≥ 50. In the serum peptide pattern 3 for diagnostics of AIS and differential diagnosis of AIS from ICH the following 24 peptides: Val-Ser-Glu- Thr-Glu-Ser-Arg-Gly-Ser-Glu-Ser-Gly-Ile-Phe- Thr-Asn-Thr-Lys, Phe-Phe-Asp-Thr-Ala-Ser-Thr-Gly-Lys, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Pro-Gly-Ser-Thr-Gly-Thr-Trp-Asn-Pro-Gly-Ser-Ser-Glu-Arg, His-Pro-Asp-Glu-Ala-Ala-Phe-Phe-Asp-Thr-Ala-Ser-Thr-Gly-Lys, Glu-Ser-Ser-Ser-His-His-Pro-Gly-Ile-Ala-Glu-Phe-Pro-Ser-Arg, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Leu-Asp-Asp-Asp-Leu-Glu-His-Gln-Gly-Gly-His-Val-Leu-Asp-His-Gly-His, Arg-Glu-Tyr-His-Thr-Glu-Lys-Leu-Val-Thr-Ser, Gly-Ser-Ser-Gly-Thr-Gly-Gly-Thr-Ala-Thr-Trp-Lys, Gly-Ser-Trp-Asn-Ser-Gly-Ser-Ser-Gly-Pro-Gly-Ser-Thr-Gly-Asn-Arg, Glu-Leu-Glu-Arg-Pro-Gly-Gly-Asn-Glu-Ile, Leu-Gly-Glu-Phe-Val-Ser-Glu-Thr-Glu-Ser-Arg, Glu-Leu-Glu-Arg-Pro-Gly-Gly-Asn-Glu, Asp-Glu-Ala-Ala-Phe-Phe-Asp-Thr-Ala-Ser-Thr-Gly-Lys, Arg-Glu-Glu-Ala-Pro-Ser-Leu-Arg, Glu-Glu-Val-Ser-Gly-Asn-Val-Ser-Pro-Gly-Thr, Tyr-Gln-Glu-Pro-Val-Leu-Gly-Pro-Val, Ser-Glu-Ser-Gly-Ile-Phe-Thr-Asn-Thr-Lys, His-Arg-His-Pro-Asp-Glu-Ala-Ala-Phe- Phe-Asp-Thr-Ala were quantified in serum samples from patients with AIS and in serum samples of the individuals without stroke and serum samples of patients with ICH and were at least 50 folds increased (≥ 50 folds) in serum samples of patients with AIS as compared with serum samples from individuals without stroke and patients with ICH.

Table 9. The serum peptide pattern 4 for diagnostics of AIS and differential diagnosis of AIS from ICH. The peptides listed in Table 9 are quantitative biomarkers of AIS since they distinguish patients with AIS from individuals without stroke and from patients with ICH. The quantitative peptides listed in table 9 are at least 100-folds increased in patients with AIS as compared with individuals without stroke and from patients with ICH. Their levels were determined with MS/MS (tandem mass spectrometry) signaling intensities. The upregulation of a particular peptide was determined by first integrating its peak area measured by a mass spectrometer across compared conditions here AIS and matched control groups (here individuals without stroke and patients with ICH). The integrated peak area reflects the number of peptide molecules in the measured sample. Next, by relative comparison, of integrated peak areas across the compared groups the fold-increase increase is calculated. Fold-increase reflects the ratio of integrated peak areas in compared conditions and is proportional to the ratio of number of the peptide molecules in compared conditions. In other words, fold-increase shows how many times more the peptide is present in serum of the patients with AIS than in the individuals without stroke and patients with ICH. However, the method that we used (tandem mass spectrometry) for quantification of the diagnostic peptides is not the limitation of the invention. Any other method for quantitative peptide analysis can be used to measure the increase in serum levels of these quantitative peptides in patients with AIS.

In our analysis we have also considered the adjusted P value (adj. pval) which is the smallest significance level at which a particular comparison will be declared statistically significant as part of the multiple comparison such as between patients with AIS and respective control groups (individuals without stroke and patients with ICH). The threshold for selecting the peptides in the table 9 was as follow; adjusted P value (adj. pval) ≤ 0.05, fold-increase≥ 100. In the serum peptide pattern 4 for diagnostics of AIS and differential diagnosis of AIS from ICH the following 16 peptides: Val-Ser-Glu-Thr-Glu-Ser-Arg-Gly-Ser-Glu-Ser-Gly-Ile-Phe-Thr-Asn-Thr-Lys, Phe-Phe-Asp-Thr-Ala-Ser-Thr-Gly-Lys, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Pro-Gly-Ser-Thr-Gly-Thr-Trp-Asn-Pro-Gly-Ser-Ser-Glu-Arg, Glu-Ser-Ser-Ser-His-His-Pro-Gly-Ile-Ala-Glu-Phe-Pro-Ser-Arg, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Arg-Glu-Tyr-His-Thr-Glu-Lys-Leu-Val-Thr-Ser, Gly-Ser-Ser-Gly-Thr-Gly-Gly-Thr-Ala-Thr-Trp-Lys, Glu-Leu-Glu-Arg-Pro-Gly-Gly-Asn-Glu-Ile, Arg-Glu-Glu-Ala-Pro-Ser-Leu-Arg, Glu-Glu-Val-Ser-Gly-Asn-Val-Ser-Pro-Gly-Thr, Ser-Glu-Ser-Gly-Ile-Phe-Thr-Asn-Thr-Lys were quantified in serum samples from patients with AIS and in serum samples of the individuals without stroke and serum samples of patients with ICH and were at least 100 folds increased (≥ 100 folds) in serum samples of patients with AIS as compared with serum samples from individuals without stroke and patients with ICH.

Table 10. The serum peptide pattern 5 for diagnostics of AIS and differential diagnosis of AIS from ICH. The peptides listed in Table 10 are quantitative biomarkers of AIS since they distinguish patients with AIS from individuals without stroke and from patients with ICH. The quantitative peptides listed in table 10 are at least 1000-folds increased in patients with AIS as compared with individuals without stroke and from patients with ICH. Their levels were determined with MS/MS (tandem mass spectrometry) signaling intensities. The upregulation of a particular peptide was determined by first integrating its peak area measured by a mass spectrometer across compared conditions here AIS and matched control groups (here individuals without stroke and patients with ICH). The integrated peak area reflects the number of peptide molecules in the measured sample. Next, by relative comparison, of integrated peak areas across the compared groups the fold-increase increase is calculated. Fold-increase reflects the ratio of integrated peak areas in compared conditions and is proportional to the ratio of number of the peptide molecules in compared conditions. In other words, fold-increase shows how many times more the peptide is present in serum of the patients with AIS than in the individuals without stroke and patients with ICH. However, the method that we used (tandem mass spectrometry) for quantification of the diagnostic peptides is not the limitation of the invention. Any other method for quantitative peptide analysis can be used to measure the increase in serum levels of these quantitative peptides in patients with AIS.

In our analysis we have also considered the adjusted P value (adj. pval) which is the smallest significance level at which a particular comparison will be declared statistically significant as part of the multiple comparison such as between patients with AIS and respective control groups (individuals without stroke and patients with ICH). The threshold for selecting the peptides in the table 10 was as follow; adjusted P value (adj. pval) ≤ 0.05, fold-increase≥ 1000. In the serum peptide pattern 5 for diagnostics of AIS and differential diagnosis of AIS from ICH the following 1 peptide: Val-Ser-Glu-Thr-Glu-Ser-Arg-Gly-Ser-Glu-Ser-Gly-Ile-Phe-Thr-Asn-Thr-Lys was quantified in serum samples from patients with AIS and in serum samples of the individuals without stroke and serum samples of patients with ICH and were at least 1000 folds increased (≥ 1000 folds) in serum samples of patients with AIS as compared with serum samples from individuals without stroke and patients with ICH.

The following examples illustrate the present invention

### Example

### Solution and glassware quality

Prior to starting any steps of peptidomics method, all the solutions using LC-MS (Liquid Chromatography with mass spectrometry) grade water, solvents, reagents, and ultraclean glass and plasticware were prepared. Moreover, all solutions, buffers were prepared immediately before use and disposed of after one week of storage.

### Collection of serum samples

The blood samples were collected directly after the stroke patients have been admitted to the hospital by specialized medical staff. Additionally, as a control blood samples from individuals without stroke and from patients with AIS were collected. All research procedures were performed per the principles of the World Medical Association Declaration of Helsinki. Thus, the studied samples were sera from patients with AIS (n=5) diagnosed routinely with imaging diagnostics methods computed tomography (CT) or magnetic resonance imaging (MRI), individuals without stroke (n=5), and patients with ICH (n=5) diagnosed routinely with imaging diagnostics methods computed tomography (CT) or magnetic resonance imaging (MRI). The serum samples were obtained as follow: the clotted blood samples were centrifuged at 3000 ×g for 10 min at 4 °C and then the serum was collected and stored at -80 °C immediately until analysis.

### Buffer preparation

First step was to prepare all the necessary buffers on the same day of peptidomics samples preparation.

1st buffer: citrate-phosphate buffer at pH 3.1 to 3.6 - (0.131 M citric acid/0.066 M Na2HPO4, NaCl 150 mM) and adjust the pH 3.1 to 3.6 with 1M NaOH prepared in LC-MS (Liquid Chromatography with mass spectrometry) grade water.

2^{nd} solution: 0.2% formic acid/methanol volume per volume (v/v) was prepared (final proportion of formic acid in methanol was 0.2%).

3^{rd} solution: 0.2% formic acid/water volume per volume (v/v) was prepared (final proportion of formic acid in water was 0.2%).

4^{th} wash buffer: water/5% methanol/0.2% formic acid volume per volume (v/v) was prepared (final proportion of methanol and formic acid in water was 5% and 0.2%, respectively).

5^{th} elution buffer: water/80% methanol/0.2% formic acid volume per volume (v/v) was prepared (final proportion of methanol and formic acid in water was 80% and 0.2%, respectively).

### Sample preparation

100 µL serum was taken from each sample and incubated with 2.9 ml of citrate-phosphate buffer- at pH 3.1-3.6 (with optimal results for pH 3.3) for 3-5 minutes (gentle and manual vertexing necessary to dissociate protein-peptide interactions) in the temperature below 4 to 8 °C e.g. on the ice. All the above mixtures were mixed e.g. centrifuged at 5000 ×g for 5 minutes at 4 °C. The supernatant was collected and diluted to 1:1 with 3 ml 0.2% FA in LC-MS (Liquid Chromatography with mass spectrometry) water volume per volume (v/v).

### One step purification of peptides

For the purification of peptides, all the diluted samples (~6ml each) were subjected to the Oasis cartridges (hydrophilic-lipophilic balanced columns, 30 mg; Waters), and all the detailed steps are as follows.
i) Cartridge's condition: cartridge was conditioned with 0.2% formic acid/methanol volume per volume (v/v) (in this solution final proportion of formic acid in methanol was 0.2%), 1ml/cartridge.
ii) Equilibration of cartridge: the cartridge was equilibrated with 0.2% formic acid/water volume per volume (v/v) (in this solution final proportion of formic acid in water was 0.2%), 1ml/cartridge.
iii) Sample loading: 6 ml of a sample that this step was the supernatant collected after centrifugation was loaded on the cartridges (hydrophilic-lipophilic balanced columns).
iv) Washing steps: cartridge was washed three times with water containing 5% methanol/0.2% formic acid volume per volume (v/v) (in this solution final proportion of methanol and formic acid in water was 5% and 0.2%, respectively), volume for each wash was 1ml/cartridge.
v) Elution: the bound material (peptides) were eluted with 1ml water containing 80% methanol/0.2% formic acid volume per volume (v/v) (in this solution final proportion of methanol and formic acid in water was 80% and 0.2%, respectively) and then diluted to water/40% methanol/0.2% formic acid volume per volume (v/v) (in this solution final proportion of methanol and formic acid in water was 40% and 0.2%, respectively).

### Peptides preparation for mass spectrometry analysis

The serum peptides sample obtained after peptides purification were spun through 3 kilo Dalton/kDa molecular weight cutoff (MWCO) ultrafiltration devices (AmiconUltra2Centrifugal Filters, Ultracel-3k, 2 mL: Millipore). Briefly, Amicon-Ultracel-3 kilo Dalton/kDa centrifugal filter was rinsed before use (the 3kDa ultrafiltration device was rinsed with 40% methanol with LC-MS (Liquid Chromatography with mass spectrometry) grade water volume per volume (v/v), 1ml water/column, and centrifuged at 4000 x g, 30 min, 4 °C). Next, 3kDa ultrafiltration device was inserted into the new collecting tube. Lastly, eluted and diluted peptide solution (~2ml) was pipetted into the 3kDa ultrafiltration device, taking care not to touch the membrane with the pipette tip. Then the 3kDa ultrafiltration device was span at 4000 x g, 120-135 min, 4 °C. The filtrate (peptides) was collected and then lyophilized to dryness. All dried peptide samples were stored at -80 until the mass spectrometry analysis.

### Sequencing of peptides with tandem mass spectrometry

The serum peptides obtained from sera of AIS patients and individuals without stroke and patients with ICH were further subjected to the LC-MS/MS (Liquid Chromatography with tandem mass spectrometry) analysis. The eluted and dried serum peptide samples were resuspended in 30 µl of loading buffer composed of 0.08% trifluoroacetic acid (TFA) in water with 2.5% acetonitrile (ACN). Indexed retention time (iRT) peptides (Biognosys) were added according to the manufacturer's guidelines. Then 6 µl of the dissolved sample were injected into UltiMate^{™} 3000 RSLCnano liquid chromatograph (Thermo Scientific) online coupled with Orbitrap Exploris 480 mass spectrometer (MS) (Thermo Scientific). µ-precolumn C18 trap cartridges (300 µm i.d.) and 5 mm length packed with C18 PepMap100 sorbent with PepMap 5 µm sorbent (P/N: 160454, Thermo Fisher Scientific) were used to concentrate and desalt serum peptides using a 5 µl/min flow of Loading buffer. Peptides were then eluted on 75 µm ID and 150 mm length fused-silica analytical column packed with PepMap 2 µm sorbent (P/N: 164534, Thermo Scientific). Analytical peptide separation was performed by a non-linear increase of a mobile phase B (0.1% formic acid (FA) in ACN) in a mobile phase A (0.1% FA in water). A non-linear gradient started at 2.5% B linearly increasing up to 35% B in 80 min, followed by a linear increase up to 60% B in the next 15 min with a flow rate of 300 nl/min. Serum peptides eluting from the column were ionized in a nano-electrospray ion source (NSI) and were introduced to Exploris 480 (Thermo Scientific).

### Mass spectrometry measurements for qualitative analysis

DDA (data-dependent acquisition) method has been used for qualitative peptidomics analysis. The data-dependent acquisition method was prepared in our laboratory. Briefly, Exploris 480 acquired the data in data-dependent peptide mode. The full scan was operated in profile mode with 120000 resolution, scanning the precursor range from m/z 350 Th to m/z 1650 Th. Normalized AGC (Automatic Gain Control) target was set to 300% with 100 msec maximum injection time. Each full scan was followed by fragmentation of the top 20 the most intense precursor ions and acquisition if their MS/MS spectra. The dynamic mass exclusion was set to 20 sec after the first precursor ion fragmentation. Precursor isotopologues were excluded, and mass tolerance was set to 10 ppm. Minimum precursor ion intensity was set to 3.0e3, and only precursor charge states of +1 to +6 were included in the experiment. The precursor isolation window was set to 1.6 Th. Normalized collision energy type with fixed collision energy mode was selected. The collision energy was set to 30%. Orbitrap resolution was set to 60000. Normalized AGC (Automatic Gain Control) target was set to 100% with an automatic setting of maximum injection time, and data type was centroid.

### Mass spectrometry measurements for quantitative analysis

DIA (data-independent acquisition) method has been used for quantitative peptidomics analysis. LC (Liquid Chromatography) separation parameters were kept identical to DDA (data-dependent acquisition) acquisition. Orbitrap Exploris 480 mass spectrometer operated in positive polarity data-independent mode (DIA) accompanied by a full-scan in profile mode with 60000 resolution. The full-scan range was set from m/z 350 Th up to m/z 1450 Th, and the normalized AGC (Automatic Gain Control) target was set to 300% with 100 msec maximum injection time. Each DIA (data-independent acquisition) cycle was accompanied by the acquisition of 62 precursor windows/scan events. DIA (data-independent acquisition) precursor range was set from m/z 350 Th up to m/z 1100 Th with 12 Th window width and 1 Th window overlap. Normalized collision energy type with fixed collision energy mode was selected to fragment precursors included within each isolation window. The collision energy was set to 30% and Orbitrap resolution in DIA (data-independent acquisition) mode was set to 30000. Normalized AGC (Automatic Gain Control) target was set to 1000% with automatic setting of maximum injection time while data type was profile.

### Data analysis for qualitative peptidomics

First, multi-search engine strategy has been applied for comprehensive qualitative analysis of serum peptidome. DDA (data-dependent acquisition) data were centroided and converted to mzML and mzXML format using MSconvert (version: 3.0.19094). Converted MS data were searched using MSFragger 3.4 embedded in Fragpipe (v.15) suite and Comet (Release 2020.01, revision 2) embedded in TPP 5.2.0 against *Homo sapiens* SwissProt+TrEMBL reference database concatenated with indexed retention time (iRT) peptide sequence (Biognosys), decoy reverse target sequences and contaminants. The search database was constructed in Fragpipe (v.15) suite. The following search settings were used for MSFragger: precursor mass tolerance was set to +-8 ppm and fragment mass tolerance to 10 ppm. Enzyme digestion was set to non-specific and peptide length was set from 7 to 45 amino acids. Peptide mass range was set from 200 to 5000 Da. Variable modifications were set to: methionine oxidation, protein N-term acetylation, and cysteinylation of cysteine. Data were mass recalibrated, and automatic parameter optimization setting was used to tune fragment mass tolerance. Output file format was set to pep.XML. The following search settings were used for Comet: precursor mass tolerance was set to 8 ppm and fragment mass tolerance to 8 ppm. The rest of the settings were identical to MSFragger. The search results (pep.XML files) were processed with PeptideProphet and iProphet as part of the Trans-Proteomic Pipeline (TPP) 5.2.0. Resulting recalculated pep.XML files with peptide iprobabilities (iPROB values) were further processed in Skyline-daily (64-bit, 20.1.9.234).

### Data analysis for quantitative peptidomics

A spectral library was built from recalculated pep.XML files in Skyline-daily (64-bit, 20.1.9.234). In "Peptide settings" a "Library" tab was selected. An option of "Build" was selected and in the newly popped-up window the "Cut-off score" was set to 0.99 (considers only peptides with false discovery rate (FDR) < 0.01 or 0.99 < iPROB value) and as an indexed retention time standard peptides were selected "Biognosys-11 (iRT-C18)". All recalculated pep.XML files were loaded and a process of building-up the spectral library was initiated. Next, in the "Digestion" tab in "Background proteome" section the "add" option was selected. In the newly popped up window the background proteome was created from the .FASTA file that was previously used as a reference search library (SwissProt+TrEMBL reference protein database concatenated with indexed retention time peptide sequence (Biognosys), decoy reverse target sequences and contaminants). Next, in the "Library" tab, "Libraries" section the newly built spectral library was selected and the "Explore" button was clicked to explore the library. In the library window an "Associate proteins" option was checked and the "Add all" button was clicked. All peptides including non-unique were added to the document. Skyline automatically generated a retention time calculator based on the indexed retention time peptide retention time values observed in recalculated pep.XML files.

The Skyline file was saved on the PC hard disc drive along with newly built spectral library (.blib format).

### Data extraction for quantitative peptidomics

The Skyline file saved in the previous step was continued to set the extraction settings properly. Peptide peak areas reflecting the peptide quantity in sample (quantitative values) were extracted from DIA (data-independent acquisition) data in Skyline-daily (64-bit, 20.1.9.234) based on the peptide-product ion pairs (transitions) listed in the spectral library generated in the previous step. In the "Peptide settings" tab, the "Max. missed cleavages" was set to 0. Next, in the "Filter" sub-tab a maximum peptide length was set from 4 to 200 amino acids. The "Exclude N terminal AAs" option was set to zero. A function of the "Auto-select all matching peptides" was selected. In the "Modifications" sub-tab, no modifications were selected. In the "Library" sub-tab, in the "Libraries" window, a spectral library that was created in the previous step was selected. Other settings in the tab were left default. In "Transition settings" tab, the "Prediction" sub-tab was left default. In the "Filter" sub-tab +1, +2, +3, +5, +6 peptide precursor charges were specified. Ion charges were set to +1 and +2. Ion type was set to y and b. Product ion selection was set as follows: in the "From:" window the "ion 4" and in the "To:" window the "last ion" options were selected. The "Auto select all matching transition" option was selected at the bottom of the sub-tab and in the "Special ions:" menu the "N-terminal to Proline" option was selected. In the "Library" sub-tab, the "Ion match tolerance" window was set to 0.05 m/z and only peptides that have at least 4 product ions were kept in analysis. In addition, if more product ions were available per peptide, then the 6 most intense were picked-up from the filtered product ions. A function of the "From filtered ion charges and types" was chosen. In the "Instrument" sub-tab we included product ions from m/z 350 up to m/z 1100. The "Method match tolerance m/z" window was set to 0.055 m/z mass tolerance. MS1 filtering was set to "none" in the full scan sub-tab and in MS/MS filtering sub-section, the "Acquisition method" window the "DIA" option was selected and in the "Product mass analyzer" the "Orbitrap" option was selected. In the "Isolation scheme:" the "Add" option was selected and in the "Edit isolation scheme" pop-up window an option of the "Prespecified isolation windows" was selected. The "Import" isolation windows from DIA (data-independent acquisition).raw file option was then selected and the isolation scheme was read from a DIA (data-independent acquisition) raw file. Back in the "Full-Scan" sub-tab in the "Resolving power:" section the resolving power was set to 30000 at 200 m/z. In the retention time filtering sub-section "Use only scans within 5 minutes of MS/MS IDs" was specified. Under the "Refine" sub-tab, the "Document" section of the "Advanced" window the "Min transitions per precursor" option was set to 4. Empty proteins were removed from the document. An equal number of reverse sequence decoys via the "Add Decoys" function in the "Refine" sub-tab was added. In the "Add Decoy Peptides" pop-up window a reverse sequence decoy generation method to create decoy peptides was selected. Following, DIA (data-independent acquisition) ".raw files" were imported. mProphet model to reintegrate peptide product ion peak boundaries in product ion chromatograms was trained as follows: in the "Refine" sub-tab the "Reintegrate" function was selected. In the "Reintegrate" pop-up window in the "Peak scoring model:" the "Add" option was selected. Following, in the "Edit Peak Scoring Model" mProphet model was activated. mProphet model was trained using targets and decoys. The "Score rows" with negative "Weight" and/or "Percentage Contribution" (red highlighted) were removed, and the mProphet model was then re-trained. New mProphet peak scoring model was then selected in the "Edit peak scoring Model" window and applied for reintegration of new peak boundaries. "Export report" function to export a summary of all dependencies required for MSstats 4.0.1. was used to generate quantitative report for downstream analysis.

### Statistical data analysis for quantitative peptidomics

DIA (data-independent acquisition) statistical data analysis performed in MSstats R-module. Statistical analysis of Skyline extracted quantitative DIA (data-independent acquisition) data was performed in R (version 4.0.0) package MSstats 4.0.1. The protein column was combined with the peptide sequence column to preserve analysis at peptide level, this formatting prevents summing peptide intensities originating from a single protein in MSstats. Extracted peakgroups were reduced by filtering on mProphet q-value < 0.01 cut-off. "SkylinetoMSstatsFormat" function was set to keep proteins with one feature and to transform Skyline output to MSstats compatible input. Further, peptide intensities were log2 transformed and quantile normalized. Differential serum peptide quantitation across AIS and individuals without stroke and patients with ICH were performed pairwise via mixed-effect models implemented in MSstats "groupComparison" function. p-values were adjusted using the Benjamini-Hochberg method and output result matrix was exported for downstream analyses.

### Serum peptide patterns for diagnostics of acute ischemic stroke in humans and differential diagnosis of acute ischemic stroke from intracranial hemorrhagic stroke

The invention reveals the accurate, multi-biomarker peptides with the biological mechanism for acute ischemic stroke (AIS). In precisely, AIS is mainly occurring due to blockage of blood flows and the potential multi-peptide biomarkers have a particular mechanism around acute phase response. Therefore, this blockage is responsible for the sudden loss of blood circulation due to thrombotic or embolic occlusion an area of the brain, resulting lack of oxygen and other nutrients so cells die and it stimulates the oxygen-carrying molecules toward the hypoxia generated cells due to blockage. The patient's body stimulates the most natural response i.e. to induce the production of plasminogen for degradation of clots and making the normal blood flow. However, these blockages are simultaneously triggering the immune response, which is reflected in our analysis. In the inclusion, our peptidomics approach reveals the peptides are identified and quantified as the response of AIS having clinical conditions corresponding to oxidative stress, inflammation, neurologic deficit, energy failure, and additional acute phase reactants that aggravate tissue damage. In the following list of biomarker peptides candidates indicate developing point-of-care diagnostic (POCD) kits.

The invention provides four serum peptide patterns for diagnostics of AIS and differential diagnosis of AIS from ICH. These peptide patters are lists of unique serum biomarker peptides specific to AIS. Selection of up-regulated peptides in AIS stroke compared to respective control groups (individuals without stroke and patients with ICH) (Table 6-10). Peptides are biomarkers of AIS since they distinguish it from individuals without stroke and patients with ICH groups based on their increased level determined from corresponding MS/MS (tandem mass spectrometry) signaling intensities. The upregulation of a particular peptide was determined by first integrating its peak area measured by a mass spectrometer across compared conditions here AIS and matched control groups (here individuals without stroke and patients with ICH). The integrated peak area reflects the number of peptide molecules in the measured sample. Next, by relative comparison, of integrated peak areas across the compared groups the fold-increase increase is calculated. Fold-increase reflects the ratio of integrated peak areas in compared conditions and is proportional to the ratio of number of the peptide molecules in compared conditions. In other words, fold-increase shows how many times more the peptide is present in serum of the patients with AIS than in the individuals without stroke and patients with ICH. However, the method that we used (tandem mass spectrometry) for identification and quantification of the diagnostic peptides is not the limitation of the invention. Any other method for qualitative and quantitative peptide analysis can be used to identify and quantify these peptides.

In our analysis we have also considered the adjusted P value (adj. pval) which is the smallest significance level at which a particular comparison will be declared statistically significant as part of the multiple comparison such as AIS and respective control groups (individuals without stroke and patients with ICH). The threshold for selecting the peptides in the table 6 was as follow; adjusted P value (adj. pval) ≤ 0.05, fold-increase≥ 2 for the quantitative peptides. Val-Ser-Glu-Thr-Glu-Ser-Arg-Gly-Ser-Glu-Ser-Gly-Ile-Phe-Thr-Asn-Thr-Lys, His-Arg-His-Pro-Asp-Glu-Ala-Ala-Phe-Phe-Asp-Thr-Ala-Ser-Thr, Phe-Phe-Asp-Thr-Ala-Ser-Thr-Gly-Lys, Gln-Leu-Gly-Leu-Pro-Gly-Pro-Pro-Asp-Val-Pro-Asp-His-Ala-Ala, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Ala-Ala-Thr-Val-Gly-Ser-Leu-Ala-Gly-Gln-Pro-Leu-Gln-Glu-Arg, Pro-Gly-Ser-Thr-Gly-Thr-Trp-Asn-Pro-Gly-Ser-Ser-Glu-Arg, His-Asp-Trp-Gly-His-Glu-Lys-Gln-Arg, His-Pro-Asp-Glu-Ala-Ala-Phe-Phe-Asp-Thr-Ala-Ser-Thr-Gly-Lys, Ser-Ser-His-His-Pro-Gly-Ile-Ala-Glu-Phe-Pro-Ser-Arg, Val-His-Leu-Thr-Pro-Glu-Glu-Lys-Ser-Ala-Val-Thr-Ala-Leu, Glu-Ser-Ser-Ser-His-His-Pro-Gly-Ile-Ala-Glu-Phe-Pro-Ser-Arg, Thr-Ala-Asn-Asp-Ser-Gly-Pro-Arg-Arg-Tyr-Thr-Ile-Ala-Ala-Leu, Arg-His-Asp-Trp-Gly-His-Glu-Lys-Gln-Arg, Val-Lys-Leu-Val-Asn-Glu-Val-Thr-Glu, Gln-Phe-Thr-Ser-Ser-Thr-Ser-Tyr-Asn, Ser-Gly-Asn-Val-Ser-Pro-Gly-Thr-Arg-Arg-Glu-Tyr-His-Thr-Glu-Lys, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Leu-Asp-Asp-Asp-Leu-Glu-His-Gln-Gly-Gly-His-Val-Leu-Asp-His-Gly-His, Ala-Phe-Arg-Gln-Pro-Ser-Ser-Ala, Phe-Val-Ser-Glu-Thr-Glu-Ser-Arg-Gly-Ser-Glu-Ser-Gly-Ile-Phe-Thr-Asn-Thr, Phe-Ser-Thr-Tyr-Asp-Arg-Asp-Asn-Asp-Gly-Trp, Thr-Val-Ile-Gln-Asn-Arg-Gln-Asp-Gly-Ser-Val-Asp-Phe, Arg-Glu-Tyr-His-Thr-Glu-Lys-Leu-Val-Thr-Ser, Gln-Ala-Gly-Ala-Ala-Gly-Ser-Arg-Met-Asn-Phe-Arg-Pro-Gly-Val-Leu-Ser-Ser, Ser-Pro-Gly-Ser-Gly-Asn-Ala-Arg-Pro-Asn-Asn-Pro-Asp-Trp-Gly-Thr-Phe, Ser-Asp-Ile-Ser-Ser-Thr-Arg-Gly-Phe-Pro-Ser-Val-Leu-Arg, Ile-Thr-His-Arg-Ile-His-Trp-Glu-Ser-Ala-Ser-Leu-Leu, Val-Ser-Gly-Trp-Gly-Arg-Asn-Ala-Asn-Phe, Phe-Asp-Thr-Ala-Ser-Thr-Gly-Lys-Thr-Phe-Pro-Gly-Phe, Ser-Ala-Gly-Ser-Trp-Asn-Ser-Gly-Ser-Ser-Gly-Pro-Gly-Ser-Thr-Gly-Asn-Arg-Asn, Val-His-Leu-Thr-Pro-Glu-Glu-Lys, Asp-Ala-Val-Arg-Gly-Ser-Pro-Ala-Ile-Asn-Val, Asp-Glu-Ala-Ala-Phe-Phe-Asp-Thr, Val-His-Leu-Thr-Pro-Glu-Glu-Lys-Ser-Ala-Val-Thr, Pro-Gly-Ser-Pro-Arg-Pro-Gly-Ser-Thr-Gly-Thr-Trp-Asn-Pro-Gly-Ser-Ser-Glu-Arg, Gly-Ser-Ser-Gly-Thr-Gly-Gly-Thr-Ala-Thr-Trp-Lys, Gly-Ser-Trp-Asn-Ser-Gly-Ser-Ser-Gly-Pro-Gly-Ser-Thr-Gly-Asn-Arg, Ser-Glu-Thr-Glu-Ser-Arg-Gly-Ser-Glu-Ser-Gly-Ile-Phe-Thr-Asn-Thr-Lys, Glu-Leu-Glu-Arg-Pro-Gly-Gly-Asn-Glu-Ile, Leu-Gly-Glu-Phe-Val-Ser-Glu-Thr-Glu-Ser-Arg, Ala-Leu-Glu-Glu-Tyr-Thr-Lys-Lys-Leu-Asn-Thr-Gln, Glu-Leu-Glu-Arg-Pro-Gly-Gly-Asn-Glu, Asp-Glu-Ala-Gly-Ser-Glu-Ala-Asp-His-Glu-Gly-Thr-His-Ser-Thr, Arg-His-Asp-Trp-Gly-His-Glu-Lys, Ala-Ala-Ser-Arg-Tyr-Leu-Asp-Lys-Thr-Glu-Gln, Arg-His-Pro-Asp-Glu-Ala-Ala-Phe, Pro-Asn-Ile-Leu-Arg-Leu-Glu-Ser-Glu-Glu-Thr, Ile-Glu-Gly-Val-Asp-Ala-Glu-Asp-Gly-His-Gly-Pro-Gly-Glu-Gln-Gln-Lys, Pro-Ser-Leu-Arg-Pro-Ala-Pro-Pro-Pro-Ile-Ser-Gly-Gly-Gly-Tyr, Thr-Asn-Thr-Lys-Glu-Ser-Ser-Ser-His-His-Pro-Gly-Ile-Ala-Glu-Phe-Pro-Ser-Arg-Gly, Ala-Glu-Val-Gly-Arg-Val-Gly-Tyr-Val-Ser-Gly-Trp-Gly-Arg-Asn-Ala-Asn-Phe, Asp-Glu-Ala-Ala-Phe-Phe-Asp-Thr-Ala-Ser-Thr-Gly-Lys, Lys-Val-Glu-Gln-Ala-Val-Glu-Thr-Glu-Pro-Glu-Pro-Glu-Leu-Arg-Gln-Gln-Thr, Met-Ala-Asp-Glu-Ala-Gly-Ser-Glu-Ala-Asp-His-Glu-Gly-Thr-His-Ser-Thr-Lys, Ser-Gly-Pro-Ser-Ser-Pro-Gln-Gln-Ala-Pro-Pro-Ala-Val-His, Gly-Ser-Glu-Ser-Gly-Ile-Phe-Thr-Asn-Thr-Lys-Glu-Ser-Ser-Ser-His-His-Pro-Gly-Ile-Ala-Glu-Phe-Pro-Ser-Arg-Gly, Arg-Glu-Glu-Ala-Pro-Ser-Leu-Arg, Glu-Glu-Val-Ser-Gly-Asn-Val-Ser-Pro-Gly-Thr, Glu-Asn-Glu-Gly-Phe-Thr-Val-Thr-Ala-Glu-Gly-Lys, Val-Thr-Glu-Pro-Ile-Ser-Ala-Glu-Ser-Gly-Glu-Gln-Val-Glu-Arg, Gly-Asp-Asp-Pro-Ser-Asp-Lys-Phe, Gly-Pro-Pro-Asp-Val-Pro-Asp-His-Ala, Glu-Glu-Val-Ser-Gly-Asn-Val-Ser-Pro-Gly-Thr-Arg-Arg-Glu, Tyr-His-Thr-Glu-Lys-Leu-Val, Tyr-Gln-Glu-Pro-Val-Leu-Gly-Pro-Val, Leu-Leu-Val-Arg-Tyr-Thr-Lys-Lys-Val-Pro-Gln-Val-Ser-Thr-Pro-Thr-Leu, Ile-Gln-Ile-Tyr-Lys-Lys-Leu-Arg-Thr-Ser-Ser-Phe, Ser-Val-Ser-Ala-Gly-Ser-Pro-His-Pro-Ala-Ile-Ala-Arg-Leu, His-Pro-Gly-Ile-Ala-Glu-Phe-Pro-Ser-Arg-Gly, Thr-Phe-Glu-Ser-Lys-Ser-Tyr, Ile-Glu-Gly-Val-Asp-Ala-Glu-Asp-Gly-His-Gly-Pro-Gly-Glu-Gln-Gln-Lys-Arg, Val-Leu-Val-Pro-Ala-Gly-Ser-Ala-Arg-Pro-Val-Ala-Phe, Asp-Ala-His-Lys-Ser-Glu-Val-Ala-His-Arg-Phe-Lys-Asp-Leu-Gly-Glu-Glu-Asn-Phe, Lys-Val-Lys-Glu-Lys-Leu-Lys-Ile-Asp-Ser, Glu-Phe-Val-Ser-Glu-Thr-Glu-Ser, His-Ala-Leu-Gln-Leu-Asn-Asn-Arg-Gln, Ser-Glu-Ser-Gly-Ile-Phe-Thr-Asn-Thr-Lys, Tyr-Val-Gly-Lys-Lys-Gln-Leu, Leu-Thr-Gln-Val-Lys-Glu-Ser-Leu-Ser-Ser-Tyr, Ser-Ser-His-His-Pro-Gly-Ile-Ala-Glu-Phe-Pro-Ser, Gly-Phe-Tyr-Glu-Ser-Asp-Val-Met-Gly-Arg, Gly-Gly-His-Leu-Asp-Ala-Lys-Gly-Ser-Phe, Asp-Val-Leu-Lys-Asp-Ser-Gly-Arg-Asp, His-Arg-His-Pro-Asp-Glu-Ala-Ala-Phe-Phe-Asp-Thr-Ala, Trp-Ser-Thr-Val-Lys-Asp-Lys-Phe-Ser-Glu-Phe, Arg-Gly-Ser-Glu-Ser-Gly-Ile-Phe-Thr-Asn-Thr, Glu-Glu-Ala-Pro-Ser-Leu-Arg-Pro-Ala-Pro-Pro-Pro-Ile-Ser-Gly-Gly-Gly-Tyr-Arg, Gly-Thr-Phe-Glu-Glu-Val-Ser-Gly-Asn-Val-Ser-Pro-Gly-Thr-Arg-Arg-Glu-Tyr-His-Thr-Glu, Gly-Ser-Ser-Gly-Thr-Gly-Gly-Thr-Ala-Thr-Trp-Lys-Pro-Gly-Ser-Ser, Ser-Ser-Ser-Tyr-Ser-Lys-Gln-Phe-Thr-Ser-Ser-Thr-Ser-Tyr-Asn-Arg-Gly-Asp-Ser-Thr-Phe-Glu-Ser-Lys-Ser-Tyr-Lys, Trp-Glu-Ser-Ala-Lys-Thr-Ala-Ala-Gln-Asn-Leu, Phe-Ser-Lys-Ala-Leu-Pro-Arg, Pro-Ser-Glu-Leu-Arg-Asp-Glu-His, Val-Val-Phe-Thr-Ala-Asn-Asp-Ser-Gly-Pro-Arg-Arg-Tyr-Thr-Ile-Ala, Leu-Gly-Glu-Phe-Val-Ser-Glu-Thr-Glu-Ser-Arg were quantified in serum samples from patients with AIS and in serum samples of the individuals without stroke and serum samples of patients with ICH and were at least 2 folds increased (≥ 2 folds) in serum samples of patients with AIS as compared with serum samples of individuals without stroke and patients with ICH (Table 6).

Simultaneously the serum peptide pattern 1 comprises 54 qualitative peptides Asp-Asp-Tyr-Val-Asn-Thr-Gln-Gly-Ala-Ser-Leu, Tyr-Pro-Trp-Thr-Gln-Arg-Phe, Phe-Arg-Leu-Leu-Gly-Asn-Val-Leu, Tyr-Gln-Lys-Val-Val-Ala-Gly-Val-Ala-Asn-Ala-Leu-Ala-His-Lys-Tyr-His, Phe-Ser-Glu-Thr-Phe-Gln-Lys-Val-Lys-Glu-Lys-Leu-Lys-Ile-Asp-Ser, Val-Lys-Glu-Lys-Leu-Lys-Ile-Asp-Ser, Tyr-Glu-Lys-Thr-Tyr-Leu-Pro-Ala-Val-Asp-Glu-Lys-Leu, Thr-Gln-Gln-Pro-Gln-Gln-Asp-Glu-Met-Pro-Ser-Pro-Thr-Phe-Leu, Thr-Gly-Ile-Phe-Thr-Asp-Gln-Val-Leu, Ser-Thr-Val-Lys-Asp-Lys-Phe-Ser-Glu-Phe, Tyr-Trp-Ser-Thr-Val-Lys-Asp-Lys-Phe, Tyr-Trp-Ser-Thr-Val-Lys-Asp-Lys-Phe-Ser-Glu-Phe, His-Thr-Asp-Leu-Ser-Gly-Lys-Val, His-Thr-Asp-Leu-Ser-Gly-Lys-Val-Phe-Val-Phe, Tyr-Val-Ile-Ile-Lys-Pro-Leu-Val-Trp-Val, Ala-Val-Arg-Gly-Ser-Pro-Ala-Ile-Asn, Glu-Val-Val-Phe-Thr-Ala-Asn-Asp-Ser-Gly-Pro-Arg-Arg-Tyr-Thr-Ile-Ala, Glu-Val-Val-Phe-Thr-Ala-Asn-Asp-Ser-Gly-Pro-Arg-Arg-Tyr-Thr-Ile-Ala-Ala, Phe-Thr-Ala-Asn-Asp-Ser-Gly-Pro-Arg-Arg-Tyr-Thr-Ile-Ala-Ala, Phe-Val-Glu-Gly-Ile-Tyr-Lys-Val-Glu, Leu-Leu-Ser-Pro-Tyr-Ser-Tyr-Ser-Thr-Thr-Ala, Leu-Ser-Pro-Tyr-Ser-Tyr-Ser-Thr-Thr-Ala, Thr-Ala-Asn-Asp-Ser-Gly-Pro-Arg-Arg-Tyr-Thr-Ile-Ala-Ala-Leu-Leu-Ser-Pro-Tyr-Ser-Tyr-Ser-Thr-Thr-Ala, Val-Val-Phe-Thr-Ala-Asn-Asp-Ser-Gly-Pro-Arg-Arg-Tyr-Thr-Ile-Ala-Ala, Phe-Lys-Asp-Ser-Ala-His-Gly-Phe, Phe-Ser-Ser-Pro-His-Gly-Lys-Asp-Leu-Leu-Phe-Lys-Asp-Ser-Ala-His-Gly-Phe, Asn-Gln-Ala-Gln-Glu-His-Phe-Gly-Lys-Asp-Lys-Ser-Lys-Glu-Phe-Gln-Leu, Tyr-Ala-Val-Ala-Val-Val-Lys-Lys-Asp, Tyr-Ala-Val-Ala-Val-Val-Lys-Lys-Asp-Ser, Tyr-Val-Thr-Ala-Ile-Arg-Asn-Leu-Arg-Glu, Ser-Lys-Glu-Asp-Pro-Gln-Thr-Phe-Tyr-Tyr-Ala-Val-Ala, Tyr-Gly-Ser-Lys-Glu-Asp-Pro-Gln-Thr-Phe, Ala-Tyr-Leu-Ala-Pro-Asn-Asn-Leu-Lys-Pro-Val-Val-Ala-Glu, Ala-Tyr-Leu-Ala-Pro-Asn-Asn-Leu-Lys-Pro-Val-Val-Ala-Glu-Phe, Ala-Tyr-Leu-Ala-Pro-Asn-Asn-Leu-Lys-Pro-Val-Val-Ala-Glu-Phe-Tyr-Gly-Ser-Lys-Glu-Asp, Asp-Ala-Gly-Leu-Val-Tyr-Asp-Ala-Tyr, Asp-Ala-Tyr-Leu-Ala-Pro-Asn-Asn-Leu-Lys-Pro-Val-Val-Ala-Glu-Phe, Tyr-Leu-Ala-Pro-Asn-Asn-Leu-Lys-Pro-Val-Val-Ala-Glu, Tyr-Leu-Ala-Pro-Asn-Asn-Leu-Lys-Pro-Val-Val-Ala-Glu-Phe, Tyr-Leu-Ala-Pro-Asn-Asn-Leu-Lys-Pro-Val-Val-Ala-Glu-Phe-Tyr-Gly-Ser-Lys-Glu-Asp, Ser-Ala-Leu-Thr-Ser-Asp-Asn-His-Gly-Ala-Thr-Tyr, Ser-Ala-Leu-Thr-Ser-Asp-Asn-His-Gly-Ala-Thr-Tyr-Ala-Phe-Ser-Gly-Thr-His-Tyr, Phe-Arg-Gln-Gly-His-Asn-Ser-Val-Phe, Val-Ala-Glu-Lys-Ala-Leu-Gln-Glu-Tyr-Arg-Lys-Lys-His-Arg-Glu-Glu, Phe-Thr-Arg-Glu-Pro-Gln-Asp-Thr-Tyr-His-Tyr-Leu-Pro, Arg-Pro-His-Phe-Phe-Phe-Pro, Arg-Pro-His-Phe-Phe-Phe-Pro-Lys-Ser-Arg-Ile-Val, Phe-Leu-Arg-Asp-Thr-Lys-Thr-Leu, Val-Ile-Glu-Pro-Gln-Gly-Leu-Arg-Phe, Phe-Val-Thr-Pro-Leu-Thr-Ser-Met, Val-His-Leu-Thr-Pro-Glu-Glu-Lys-Ser, Trp-Gly-Lys-Val-Asn-Val-Asp-Glu-Val-Gly-Gly-Glu-Ala-Leu-Gly-Arg-Leu, Arg-Gly-Pro-Leu-Val-Lys-Tyr-Ser-Ser-Asp-Tyr, Ala-Ser-Glu-Gly-Gly-Phe-Thr-Ala-Thr-Gly-Gln-Arg that were detectable only in serum of the patients with AIS but were not detectable in serum samples of the individuals without stroke and serum samples of patients with ICH (Table 6).

As grade of the fold increase of the 100 quantitative peptides corresponds with their predictive significance for AIS diagnostics, we also defined the serum peptide pattern 2 (a panel of 32 peptides), the serum peptide pattern 3 (a panel of 24 peptides), the serum peptide pattern 4 (a panel of 16 peptides) and the serum peptide pattern 5 (a panel of 1 peptide) which can be used alone for AIS diagnostics or differentiation of AIS from ICH when their fold increase in serum of patients with AIS is equal or higher than 30, 50, 100 and 1000 folds, respectively as compared with serum levels of these peptides in patients without stroke and in patients with ICH.

The serum peptide pattern 2 for diagnostics of and differential diagnosis of AIS from ICH. The peptides listed in Table 7 are quantitative biomarkers of AIS since they distinguish patients with AIS from individuals without stroke and from patients with ICH. The quantitative peptides listed in table 7 are at least 30-folds increased in patients with AIS as compared with individuals without stroke and from patients with ICH. Their levels were determined with MS/MS (tandem mass spectrometry) signaling intensities. The upregulation of a particular peptide was determined by first integrating its peak area measured by a mass spectrometer across compared conditions here AIS and matched control groups (here individuals without stroke and patients with ICH). The integrated peak area reflects the number of peptide molecules in the measured sample. Next, by relative comparison, of integrated peak areas across the compared groups the fold-increase increase is calculated. Fold-increase reflects the ratio of integrated peak areas in compared conditions and is proportional to the ratio of number of the peptide molecules in compared conditions. In other words fold-increase shows how many times more the peptide is present in serum of the patients with AIS than in the individuals without stroke and patients with ICH. However, the method that we used (tandem mass spectrometry) for quantification of the diagnostic peptides is not the limitation of the invention. Any other method for quantitative peptide analysis can be used to measure the increase in serum levels of these quantitative peptides in patients with AIS.

In our analysis we have also considered the adjusted P value (adj. pval) which is the smallest significance level at which a particular comparison will be declared statistically significant as part of the multiple comparison such as between patients with AIS and respective control groups (individuals without stroke and patients with ICH). The threshold for selecting the peptides in the table 7 was as follow; adjusted P value (adj. pval) ≤ 0.05, fold-increase≥ 30. In the serum peptide pattern 2 for diagnostics of AIS and differential diagnosis of AIS from ICH the following 32 peptides: Val-Ser-Glu-Thr-Glu-Ser-Arg-Gly-Ser-Glu-Ser-Gly-Ile-Phe-Thr-Asn-Thr-Lys, His-Arg-His-Pro-Asp-Glu-Ala-Ala-Phe-Phe-Asp-Thr-Ala-Ser-Thr, Phe-Phe-Asp-Thr-Ala-Ser-Thr-Gly-Lys, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Ala-Ala-Thr-Val-Gly-Ser-Leu-Ala-Gly-Gln-Pro-Leu-Gln-Glu-Arg, Pro-Gly-Ser-Thr-Gly-Thr-Trp-Asn-Pro-Gly-Ser-Ser-Glu-Arg, His-Asp-Trp-Gly-His-Glu-Lys-Gln-Arg, His-Pro-Asp-Glu-Ala-Ala-Phe-Phe-Asp-Thr-Ala-Ser-Thr-Gly-Lys, Ser-Ser-His-His-Pro-Gly-Ile-Ala-Glu-Phe-Pro-Ser-Arg, Glu-Ser-Ser-Ser-His-His-Pro-Gly-Ile-Ala-Glu-Phe-Pro-Ser-Arg, Ser-Gly-Asn-Val-Ser-Pro-Gly-Thr-Arg-Arg-Glu-Tyr-His-Thr-Glu-Lys, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Leu-Asp-Asp-Asp-Leu-Glu-His-Gln-Gly-Gly-His-Val-Leu-Asp-His-Gly-His, Phe-Val-Ser-Glu-Thr-Glu-Ser-Arg-Gly-Ser-Glu-Ser-Gly-Ile-Phe-Thr-Asn-Thr, Arg-Glu-Tyr-His-Thr-Glu-Lys-Leu-Val-Thr-Ser, Gly-Ser-Ser-Gly-Thr-Gly-Gly-Thr-Ala-Thr-Trp-Lys, Gly-Ser-Trp-Asn-Ser-Gly-Ser-Ser-Gly-Pro-Gly-Ser-Thr-Gly-Asn-Arg, Glu-Leu-Glu-Arg-Pro-Gly-Gly-Asn-Glu-Ile, Leu-Gly-Glu-Phe-Val-Ser-Glu-Thr-Glu-Ser-Arg, Glu-Leu-Glu-Arg-Pro-Gly-Gly-Asn-Glu, Asp-Glu-Ala-Gly-Ser-Glu-Ala-Asp-His-Glu-Gly-Thr-His-Ser-Thr, Asp-Glu-Ala-Ala-Phe-Phe-Asp-Thr-Ala-Ser-Thr-Gly-Lys, Met-Ala-Asp-Glu-Ala-Gly-Ser-Glu-Ala-Asp-His-Glu-Gly-Thr-His-Ser-Thr-Lys, Arg-Glu-Glu-Ala-Pro-Ser-Leu-Arg, Glu-Glu-Val-Ser-Gly-Asn-Val-Ser-Pro-Gly-Thr, Tyr-Gln-Glu-Pro-Val-Leu-Gly-Pro-Val, Ser-Glu-Ser-Gly-Ile-Phe-Thr-Asn-Thr-Lys, His-Arg-His-Pro-Asp-Glu-Ala-Ala-Phe-Phe-Asp-Thr-Ala were quantified in serum samples from patients with AIS and in serum samples of the individuals without stroke and serum samples of patients with ICH and were at least 30 folds increased (≥ 30 folds) in serum samples of patients with AIS as compared with serum samples from individuals without stroke and patients with ICH .

The serum peptide pattern 3 for diagnostics of AIS and differential diagnosis of AIS from ICH. The peptides listed in Table 8 are quantitative biomarkers of AIS since they distinguish patients with AIS from individuals without stroke and from patients with ICH. The quantitative peptides listed in table 8 are at least 50-folds increased in patients with AIS as compared with individuals without stroke and from patients with ICH. Their levels were determined with MS/MS (tandem mass spectrometry) signaling intensities. The upregulation of a particular peptide was determined by first integrating its peak area measured by a mass spectrometer across compared conditions here AIS and matched control groups (here individuals without stroke and patients with ICH). The integrated peak area reflects the number of peptide molecules in the measured sample. Next, by relative comparison, of integrated peak areas across the compared groups the fold-increase increase is calculated. Fold-increase reflects the ratio of integrated peak areas in compared conditions and is proportional to the ratio of number of the peptide molecules in compared conditions. In other words, fold-increase shows how many times more the peptide is present in serum of the patients with AIS than in the individuals without stroke and patients with ICH. However, the method that we used (tandem mass spectrometry) for quantification of the diagnostic peptides is not the limitation of the invention. Any other method for quantitative peptide analysis can be used to measure the increase in serum levels of these quantitative peptides in patients with AIS.

In our analysis we have also considered the adjusted P value (adj. pval) which is the smallest significance level at which a particular comparison will be declared statistically significant as part of the multiple comparison such as between patients with AIS and respective control groups (individuals without stroke and patients with ICH). The threshold for selecting the peptides in the table 8 was as follow; adjusted P value (adj. pval) ≤ 0.05, fold-increase≥ 50. In the serum peptide pattern 3 for diagnostics of AIS and differential diagnosis of AIS from ICH the following 24 peptides: Val-Ser-Glu- Thr-Glu-Ser-Arg-Gly-Ser-Glu-Ser-Gly-Ile-Phe- Thr-Asn-Thr-Lys, Phe-Phe-Asp-Thr-Ala-Ser-Thr-Gly-Lys, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Pro-Gly-Ser-Thr-Gly-Thr-Trp-Asn-Pro-Gly-Ser-Ser-Glu-Arg, His-Pro-Asp-Glu-Ala-Ala-Phe-Phe-Asp-Thr-Ala-Ser-Thr-Gly-Lys, Glu-Ser-Ser-Ser-His-His-Pro-Gly-Ile-Ala-Glu-Phe-Pro-Ser-Arg, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Leu-Asp-Asp-Asp-Leu-Glu-His-Gln-Gly-Gly-His-Val-Leu-Asp-His-Gly-His, Arg-Glu-Tyr-His-Thr-Glu-Lys-Leu-Val-Thr-Ser, Gly-Ser-Ser-Gly-Thr-Gly-Gly-Thr-Ala-Thr-Trp-Lys, Gly-Ser-Trp-Asn-Ser-Gly-Ser-Ser-Gly-Pro-Gly-Ser-Thr-Gly-Asn-Arg, Glu-Leu-Glu-Arg-Pro-Gly-Gly-Asn-Glu-Ile, Leu-Gly-Glu-Phe-Val-Ser-Glu-Thr-Glu-Ser-Arg, Glu-Leu-Glu-Arg-Pro-Gly-Gly-Asn-Glu, Asp-Glu-Ala-Ala-Phe-Phe-Asp-Thr-Ala-Ser-Thr-Gly-Lys, Arg-Glu-Glu-Ala-Pro-Ser-Leu-Arg, Glu-Glu-Val-Ser-Gly-Asn-Val-Ser-Pro-Gly-Thr, Tyr-Gln-Glu-Pro-Val-Leu-Gly-Pro-Val, Ser-Glu-Ser-Gly-Ile-Phe-Thr-Asn-Thr-Lys, His-Arg-His-Pro-Asp-Glu-Ala-Ala-Phe- Phe-Asp-Thr-Ala were quantified in serum samples from patients with AIS and in serum samples of the individuals without stroke and serum samples of patients with ICH and were at least 50 folds increased (≥ 50 folds) in serum samples of patients with AIS as compared with serum samples from individuals without stroke and patients with ICH.

The serum peptide pattern 4 for diagnostics of AIS and differential diagnosis of AIS from ICH. The peptides listed in Table 9 are quantitative biomarkers of AIS since they distinguish patients with AIS from individuals without stroke and from patients with ICH. The quantitative peptides listed in table 9 are at least 100-folds increased in patients with AIS as compared with individuals without stroke and from patients with ICH. Their levels were determined with MS/MS (tandem mass spectrometry) signaling intensities. The upregulation of a particular peptide was determined by first integrating its peak area measured by a mass spectrometer across compared conditions here AIS and matched control groups (here individuals without stroke and patients with ICH). The integrated peak area reflects the number of peptide molecules in the measured sample. Next, by relative comparison, of integrated peak areas across the compared groups the fold-increase increase is calculated. Fold-increase reflects the ratio of integrated peak areas in compared conditions and is proportional to the ratio of number of the peptide molecules in compared conditions. In other words fold-increase shows how many times more the peptide is present in serum of the patients with AIS than in the individuals without stroke and patients with ICH. However, the method that we used (tandem mass spectrometry) for quantification of the diagnostic peptides is not the limitation of the invention. Any other method for quantitative peptide analysis can be used to measure the increase in serum levels of these quantitative peptides in patients with AIS.

In our analysis we have also considered the adjusted P value (adj. pval) which is the smallest significance level at which a particular comparison will be declared statistically significant as part of the multiple comparison such as between patients with AIS and respective control groups (individuals without stroke and patients with ICH). The threshold for selecting the peptides in the table 9 was as follow; adjusted P value (adj. pval) ≤ 0.05, fold-increase≥ 100. In the serum peptide pattern 4 for diagnostics of AIS and differential diagnosis of AIS from ICH the following 16 peptides: Val-Ser-Glu-Thr-Glu-Ser-Arg-Gly-Ser-Glu-Ser-Gly-Ile-Phe-Thr-Asn-Thr-Lys, Phe-Phe-Asp-Thr-Ala-Ser-Thr-Gly-Lys, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Pro-Gly-Ser-Thr-Gly-Thr-Trp-Asn-Pro-Gly-Ser-Ser-Glu-Arg, Glu-Ser-Ser-Ser-His-His-Pro-Gly-Ile-Ala-Glu-Phe-Pro-Ser-Arg, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Arg-Glu-Tyr-His-Thr-Glu-Lys-Leu-Val-Thr-Ser, Gly-Ser-Ser-Gly-Thr-Gly-Gly-Thr-Ala-Thr-Trp-Lys, Glu-Leu-Glu-Arg-Pro-Gly-Gly-Asn-Glu-Ile, Arg-Glu-Glu-Ala-Pro-Ser-Leu-Arg, Glu-Glu-Val-Ser-Gly-Asn-Val-Ser-Pro-Gly-Thr, Ser-Glu-Ser-Gly-Ile-Phe-Thr-Asn-Thr-Lys were quantified in serum samples from patients with AIS and in serum samples of the individuals without stroke and serum samples of patients with ICH and were at least 100 folds increased (≥ 100 folds) in serum samples of patients with AIS as compared with serum samples from individuals without stroke and patients with ICH.

The serum peptide pattern 5 for diagnostics of AIS and differential diagnosis of AIS from ICH. The peptides listed in Table 10 are quantitative biomarkers of AIS since they distinguish patients with AIS from individuals without stroke and from patients with ICH. The quantitative peptides listed in table 10 are at least 1000-folds increased in patients with AIS as compared with individuals without stroke and from patients with ICH. Their levels were determined with MS/MS (tandem mass spectrometry) signaling intensities. The upregulation of a particular peptide was determined by first integrating its peak area measured by a mass spectrometer across compared conditions here AIS and matched control groups (here individuals without stroke and patients with ICH). The integrated peak area reflects the number of peptide molecules in the measured sample. Next, by relative comparison, of integrated peak areas across the compared groups the fold-increase increase is calculated. Fold-increase reflects the ratio of integrated peak areas in compared conditions and is proportional to the ratio of number of the peptide molecules in compared conditions. In other words, fold-increase shows how many times more the peptide is present in serum of the patients with AIS than in the individuals without stroke and patients with ICH. However, the method that we used (tandem mass spectrometry) for quantification of the diagnostic peptides is not the limitation of the invention. Any other method for quantitative peptide analysis can be used to measure the increase in serum levels of these quantitative peptides in patients with AIS.

In our analysis we have also considered the adjusted P value (adj. pval) which is the smallest significance level at which a particular comparison will be declared statistically significant as part of the multiple comparison such as between patients with AIS and respective control groups (individuals without stroke and patients with ICH). The threshold for selecting the peptides in the table 10 was as follow; adjusted P value (adj. pval) ≤ 0.05, fold-increase≥ 1000. In the serum peptide pattern 5 for diagnostics of AIS and differential diagnosis of AIS from ICH the following 1 peptide: Val-Ser-Glu-Thr-Glu-Ser-Arg-Gly-Ser-Glu-Ser-Gly-Ile-Phe-Thr-Asn-Thr-Lys was quantified in serum samples from patients with AIS and in serum samples of the individuals without stroke and serum samples of patients with ICH and were at least 1000 folds increased (≥ 1000 folds) in serum samples of patients with AIS as compared with serum samples from individuals without stroke and patients with ICH.

The invention provides 5 serum peptide patterns (Tables 6-10) that are sets of unique biomarker serum peptides specific to AIS. As a result, it will enhance confidence of accurate diagnosis because considering a single biomarker for stroke might be too risky to rely on an accurate diagnosis. In addition, our peptidomics analysis indicates that the unique and potential peptide multi-biomarkers AIS are derived from several proteins such as

The following peptides derive from Fibrinogen alpha chain [Cleaved into: Fibrinopeptide A; Fibrinogen alpha chain] (FGA) (FIBA_HUMAN), these peptides are:
Val-Ser-Glu-Thr-Glu-Ser-Arg-Gly-Ser-Glu-Ser-Gly-Ile-Phe-Thr-Asn-Thr-Lys
His-Arg-His-Pro-Asp-Glu-Ala-Ala-Phe-Phe-Asp-Thr-Ala-Ser-Thr
Phe-Phe-Asp-Thr-Ala-Ser-Thr-Gly-Lys
Gly-Ser-Glu-Ser-Gly-Ile-Phe-Thr-Asn-Thr-Lys
Asn-Pro-Gly-Ser-Ser-Gly-Thr-Gly-Gly-Thr-Ala-Thr-Trp-Lys
Gln-Phe-Thr-Ser-Ser-Thr-Ser-Tyr-Asn-Arg
Pro-Gly-Ser-Thr-Gly-Thr-Trp-Asn-Pro-Gly-Ser-Ser-Glu-Arg
His-Pro-Asp-Glu-Ala-Ala-Phe-Phe-Asp-Thr-Ala-Ser-Thr-Gly-Lys
Ser-Ser-His-His-Pro-Gly-Ile-Ala-Glu-Phe-Pro-Ser-Arg
Glu-Ser-Ser-Ser-His-His-Pro-Gly-Ile-Ala-Glu-Phe-Pro-Ser-Arg
Gln-Phe-Thr-Ser-Ser-Thr-Ser-Tyr-Asn
Ser-Gly-Asn-Val-Ser-Pro-Gly-Thr-Arg-Arg-Glu-Tyr-His-Thr-Glu-Lys
Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg
Thr-Ser-Lys-Gly-Asp-Lys-Glu-Leu
Phe-Val-Ser-Glu-Thr-Glu-Ser-Arg-Gly-Ser-Glu-Ser-Gly-Ile-Phe-Thr-Asn-Thr
Arg-Glu-Tyr-His-Thr-Glu-Lys-Leu-Val-Thr-Ser
Ser-Pro-Gly-Ser-Gly-Asn-Ala-Arg-Pro-Asn-Asn-Pro-Asp-Trp-Gly-Thr-Phe
Phe-Asp-Thr-Ala-Ser-Thr-Gly-Lys-Thr-Phe-Pro-Gly-Phe
Ser-Ala-Gly-Ser-Trp-Asn-Ser-Gly-Ser-Ser-Gly-Pro-Gly-Ser-Thr-Gly-Asn-Arg-Asn
Asp-Glu-Ala-Ala-Phe-Phe-Asp-Thr
Pro-Gly-Ser-Pro-Arg-Pro-Gly-Ser-Thr-Gly-Thr-Trp-Asn-Pro-Gly-Ser-Ser-Glu-Arg
Gly-Ser-Ser-Gly-Thr-Gly-Gly-Thr-Ala-Thr-Trp-Lys
Gly-Ser-Trp-Asn-Ser-Gly-Ser-Ser-Gly-Pro-Gly-Ser-Thr-Gly-Asn-Arg
Ser-Glu-Thr-Glu-Ser-Arg-Gly-Ser-Glu-Ser-Gly-Ile-Phe-Thr-Asn-Thr-Lys
Glu-Leu-Glu-Arg-Pro-Gly-Gly-Asn-Glu-Ile
Leu-Gly-Glu-Phe-Val-Ser-Glu-Thr-Glu-Ser-Arg
Glu-Leu-Glu-Arg-Pro-Gly-Gly-Asn-Glu
Asp-Glu-Ala-Gly-Ser-Glu-Ala-Asp-His-Glu-Gly-Thr-His-Ser-Thr
Arg-His-Pro-Asp-Glu-Ala-Ala-Phe
Thr-Asn-Thr-Lys-Glu-Ser-Ser-Ser-His-His-Pro-Gly-Ile-Ala-Glu-Phe-Pro-Ser-Arg-Gly
Asp-Glu-Ala-Ala-Phe-Phe-Asp-Thr-Ala-Ser-Thr-Gly-Lys
Met-Ala-Asp-Glu-Ala-Gly-Ser-Glu-Ala-Asp-His-Glu-Gly-Thr-His-Ser-Thr-Lys
Gly-Ser-Glu-Ser-Gly-Ile-Phe-Thr-Asn-Thr-Lys-Glu-Ser-Ser-Ser-His-His-Pro-Gly-Ile-Ala-Glu-Phe-Pro-Ser-Arg-Gly
Glu-Glu-Val-Ser-Gly-Asn-Val-Ser-Pro-Gly-Thr
Glu-Glu-Val-Ser-Gly-Asn-Val-Ser-Pro-Gly-Thr-Arg-Arg-Glu
Tyr-His-Thr-Glu-Lys-Leu-Val
His-Pro-Gly-Ile-Ala-Glu-Phe-Pro-Ser-Arg-Gly
Thr-Phe-Glu-Ser-Lys-Ser-Tyr
Glu-Phe-Val-Ser-Glu-Thr-Glu-Ser
Ser-Glu-Ser-Gly-Ile-Phe-Thr-Asn-Thr-Lys
Ser-Ser-His-His-Pro-Gly-Ile-Ala-Glu-Phe-Pro-Ser
His-Arg-His-Pro-Asp-Glu-Ala-Ala-Phe-Phe-Asp-Thr-Ala
Trp-Ser-Thr-Val-Lys-Asp-Lys-Phe-Ser-Glu-Phe
Glu-Glu-Ala-Pro-Ser-Leu-Arg-Pro-Ala-Pro-Pro-Pro-Ile-Ser-Gly-Gly-Gly-Tyr-Arg
Gly-Ser-Ser-Gly-Thr-Gly-Gly-Thr-Ala-Thr-Trp-Lys-Pro-Gly-Ser-Ser
Ser-Ser-Ser-Tyr-Ser-Lys-Gln-Phe-Thr-Ser-Ser-Thr-Ser-Tyr-Asn-Arg-Gly-Asp-Ser-Thr-Phe-Glu-Ser-Lys-Ser-Tyr-Lys
Trp-Glu-Ser-Ala-Lys-Thr-Ala-Ala-Gln-Asn-Leu

The following peptides derive from Inter-alpha-trypsin inhibitor heavy chain H4 (ITI heavy chain H4) (ITI-HC4) (Inter-alpha-inhibitor heavy chain 4) (Inter-alpha-trypsin inhibitor family heavy chain-related protein) (IHRP) (Plasma kallikrein sensitive glycoprotein 120) (Gp120) (PK-120) [Cleaved into: 70 kDa inter-alpha-trypsin inhibitor heavy chain H4; 35 kDa inter-alpha-trypsin inhibitor heavy chain H4] (ITIH4) (ITIH4_HUMAN), these peptides are:
Gln-Leu-Gly-Leu-Pro-Gly-Pro-Pro-Asp-Val-Pro-Asp-His-Ala-Ala
Gln-Ala-Gly-Ala-Ala-Gly-Ser-Arg-Met-Asn-Phe-Arg-Pro-Gly-Val-Leu-Ser-Ser
Gly-Pro-Pro-Asp-Val-Pro-Asp-His-Ala

The following peptides derive from Fibrinogen beta chain [Cleaved into: Fibrinopeptide B; Fibrinogen beta chain] (FGB) (FIBB_HUMAN), these peptides are:
Ala-Pro-Pro-Pro-Ile-Ser-Gly-Gly-Gly-Tyr
Phe-Ser-Thr-Tyr-Asp-Arg-Asp-Asn-Asp-Gly-Trp
Thr-Val-Ile-Gln-Asn-Arg-Gln-Asp-Gly-Ser-Val-Asp-Phe
Pro-Ser-Leu-Arg-Pro-Ala-Pro-Pro-Pro-Ile-Ser-Gly-Gly-Gly-Tyr
Arg-Glu-Glu-Ala-Pro-Ser-Leu-Arg
Gly-Thr-Phe-Glu-Glu-Val-Ser-Gly-Asn-Val-Ser-Pro-Gly-Thr-Arg-Arg-Glu-Tyr-His-Thr-Glu

The following peptides derive from Apolipoprotein E (Apo-E) (APOE) (APOE_HUMAN), these peptides are:
Ala-Ala-Thr-Val-Gly-Ser-Leu-Ala-Gly-Gln-Pro-Leu-Gln-Glu-Arg
Lys-Val-Glu-Gln-Ala-Val-Glu-Thr-Glu-Pro-Glu-Pro-Glu-Leu-Arg-Gln-Gln-Thr

The following peptides derive from Kininogen-1 (Alpha-2-thiol proteinase inhibitor) (Fitzgerald factor) (High molecular weight kininogen) (HMWK) (Williams-Fitzgerald-Flaujeac factor) [Cleaved into: Kininogen-1 heavy chain; T-kinin (Ile-Ser-Bradykinin); Bradykinin (Kallidin I); Lysyl-bradykinin (Kallidin II); Kininogen-1 light chain; Low molecular weight growth-promoting factor] (KNG1) (KNG1_HUMAN), these peptides are:
His-Asp-Trp-Gly-His-Glu-Lys-Gln-Arg
Arg-His-Asp-Trp-Gly-His-Glu-Lys-Gln-Arg
Leu-Asp-Asp-Asp-Leu-Glu-His-Gln-Gly-Gly-His-Val-Leu-Asp-His-Gly-His
Arg-His-Asp-Trp-Gly-His-Glu-Lys

The following peptides derive from Hemoglobin subunit beta (Beta-globin) (Hemoglobin beta chain) [Cleaved into: LVV-hemorphin-7; Spinorphin] (HBB) (HBB_HUMAN), these peptides are:
Val-His-Leu-Thr-Pro-Glu-Glu-Lys-Ser-Ala-Val-Thr-Ala-Leu
Val-His-Leu-Thr-Pro-Glu-Glu-Lys-Ser-Ala-Val-Thr
Val-His-Leu-Thr-Pro-Glu-Glu-Lys-Ser
Trp-Gly-Lys-Val-Asn-Val-Asp-Glu-Val-Gly-Gly-Glu-Ala-Leu-Gly-Arg-Leu

The following peptides derive from Transthyretin (ATTR) (Prealbumin) (TBPA) (TTR) (TTHY_HUMAN), these peptides are:
Thr-Ala-Asn-Asp-Ser-Gly-Pro-Arg-Arg-Tyr-Thr-Ile-Ala-Ala-Leu
Asp-Ala-Val-Arg-Gly-Ser-Pro-Ala-Ile-Asn-Val
Leu-Gly-Glu-Phe-Val-Ser-Glu-Thr-Glu-Ser-Arg
Ala-Val-Arg-Gly-Ser-Pro-Ala-Ile-Asn
Glu-Val-Val-Phe-Thr-Ala-Asn-Asp-Ser-Gly-Pro-Arg-Arg-Tyr-Thr-Ile-Ala
Glu-Val-Val-Phe-Thr-Ala-Asn-Asp-Ser-Gly-Pro-Arg-Arg-Tyr-Thr-Ile-Ala-Ala
Phe-Thr-Ala-Asn-Asp-Ser-Gly-Pro-Arg-Arg-Tyr-Thr-Ile-Ala-Ala
Phe-Val-Glu-Gly-Ile-Tyr-Lys-Val-Glu
Leu-Leu-Ser-Pro-Tyr-Ser-Tyr-Ser-Thr-Thr-Ala
Leu-Ser-Pro-Tyr-Ser-Tyr-Ser-Thr-Thr-Ala
Thr-Ala-Asn-Asp-Ser-Gly-Pro-Arg-Arg-Tyr-Thr-Ile-Ala-Ala-Leu-Leu-Ser-Pro-Tyr-Ser-Tyr-Ser-Thr-Thr-Ala
Val-Val-Phe-Thr-Ala-Asn-Asp-Ser-Gly-Pro-Arg-Arg-Tyr-Thr-Ile-Ala-Ala

The following peptides derive from Albumin (ALB) (ALBU _HUMAN), these peptides are:
Val-Lys-Leu-Val-Asn-Glu-Val-Thr-Glu
Leu-Leu-Val-Arg-Tyr-Thr-Lys-Lys-Val-Pro-Gln-Val-Ser-Thr-Pro-Thr-Leu
Asp-Ala-His-Lys-Ser-Glu-Val-Ala-His-Arg-Phe-Lys-Asp-Leu-Gly-Glu-Glu-Asn-Phe

The following peptides derive from Complement C3 (C3 and PZP-like alpha-2-macroglobulin domain-containing protein 1) [Cleaved into: Complement C3 beta chain; C3-beta-c (C3bc); Complement C3 alpha chain; C3a anaphylatoxin; Acylation stimulating protein (ASP) (C3adesArg); Complement C3b alpha' chain; Complement C3c alpha' chain fragment 1; Complement C3dg fragment; Complement C3g fragment; Complement C3d fragment; Complement C3f fragment; Complement C3c alpha' chain fragment 2] (C3) (CO3_HUMAN), these peptides are:
Ala-Phe-Arg-Gln-Pro-Ser-Ser-Ala
Ile-Thr-His-Arg-Ile-His-Trp-Glu-Ser-Ala-Ser-Leu-Leu
Pro-Asn-Ile-Leu-Arg-Leu-Glu-Ser-Glu-Glu-Thr
Glu-Asn-Glu-Gly-Phe-Thr-Val-Thr-Ala-Glu-Gly-Lys

The following peptide derive from Immunoglobulin heavy constant mu (Ig mu chain C region) (Ig mu chain C region BOT) (Ig mu chain C region GAL) (Ig mu chain C region OU) (IGHM) (IGHM_HUMAN), this peptide is:
Ser-Asp-Ile-Ser-Ser-Thr-Arg-Gly-Phe-Pro-Ser-Val-Leu-Arg

The following peptides derive from Haptoglobin (Zonulin) [Cleaved into: Haptoglobin alpha chain; Haptoglobin beta chain] (HP) (HPT _HUMAN), these peptides are:
Val-Ser-Gly-Trp-Gly-Arg-Asn-Ala-Asn-Phe
Ala-Glu-Val-Gly-Arg-Val-Gly-Tyr-Val-Ser-Gly-Trp-Gly-Arg-Asn-Ala-Asn-Phe
Tyr-Val-Gly-Lys-Lys-Gln-Leu
Gly-Gly-His-Leu-Asp-Ala-Lys-Gly-Ser-Phe

The following peptides derive from Hemoglobin subunit delta (Delta-globin) (Hemoglobin delta chain) (HBD) (HBD_HUMAN), these peptides are:
Val-His-Leu-Thr-Pro-Glu-Glu-Lys
Tyr-Pro-Trp-Thr-Gln-Arg-Phe
Phe-Arg-Leu-Leu-Gly-Asn-Val-Leu
Tyr-Gln-Lys-Val-Val-Ala-Gly-Val-Ala-Asn-Ala-Leu-Ala-His-Lys-Tyr-His

The following peptides derive from Apolipoprotein A-I (Apo-AI) (ApoA-I) (Apolipoprotein A1) [Cleaved into: Proapolipoprotein A-I (ProapoA-I); Truncated apolipoprotein A-I (Apolipoprotein A-I(1-242))] (APOA1) (APOA1_HUMAN), these peptides are:
Ala-Leu-Glu-Glu-Tyr-Thr-Lys-Lys-Leu-Asn-Thr-Gln
Asp-Val-Leu-Lys-Asp-Ser-Gly-Arg-Asp

The following peptides derive from Complement C4-A (Acidic complement C4) (C3 and PZP-like alpha-2-macroglobulin domain-containing protein 2) [Cleaved into: Complement C4 beta chain; Complement C4-A alpha chain; C4a anaphylatoxin; C4b-A; C4d-A; Complement C4 gamma chain] (C4A) (CO4A_HUMAN), these peptides are:
Ala-Ala-Ser-Arg-Tyr-Leu-Asp-Lys-Thr-Glu-Gln
Ser-Val-Ser-Ala-Gly-Ser-Pro-His-Pro-Ala-Ile-Ala-Arg-Leu
Val-Leu-Val-Pro-Ala-Gly-Ser-Ala-Arg-Pro-Val-Ala-Phe
His-Ala-Leu-Gln-Leu-Asn-Asn-Arg-Gln

The following peptides derive from Complement factor B (EC 3.4.21.47) (C3/C5 convertase) (Glycine-rich beta glycoprotein) (GBG) (PBF2) (Properdin factor B) [Cleaved into: Complement factor B Ba fragment; Complement factor B Bb fragment] (CFB) (CFAB_HUMAN), these peptides are:
Ile-Glu-Gly-Val-Asp-Ala-Glu-Asp-Gly-His-Gly-Pro-Gly-Glu-Gln-Gln-Lys
Ile-Glu-Gly-Val-Asp-Ala-Glu-Asp-Gly-His-Gly-Pro-Gly-Glu-Gln-Gln-Lys-Arg

The following peptide derive from C6orf25 (Megakaryocyte and platelet inhibitory receptor G6b) (MPIG6B) (A0A140T9L8), this peptide is
Ser-Gly-Pro-Ser-Ser-Pro-Gln-Gln-Ala-Pro-Pro-Ala-Val-His

The following peptide derive from Apolipoprotein L1 (Apolipoprotein L) (Apo-L) (ApoL) (Apolipoprotein L-I) (ApoL-I) (APOL1) (APOL1_HUMAN), this peptide is
Val-Thr-Glu-Pro-Ile-Ser-Ala-Glu-Ser-Gly-Glu-Gln-Val-Glu-Arg

The following peptide derive from Fibrinogen gamma chain (FGG) (FIBG_HUMAN), this peptide is
Gly-Asp-Asp-Pro-Ser-Asp-Lys-Phe

The following peptide derive from Beta-casein [Cleaved into: Casoparan; Antioxidant peptide; Casohypotensin] (CSN2) (CASB_HUMAN), this peptide is
Tyr-Gln-Glu-Pro-Val-Leu-Gly-Pro-Val

The following peptide derive from Apolipoprotein B-100 (Apo B-100) [Cleaved into: Apolipoprotein B-48 (Apo B-48)] (APOB) (APOB_HUMAN), this peptide is
Ile-Gln-Ile-Tyr-Lys-Lys-Leu-Arg-Thr-Ser-Ser-Phe

The following peptides derive from Apolipoprotein C-I (Apo-CI) (ApoC-I) (Apolipoprotein C1) [Cleaved into: Truncated apolipoprotein C-I] (APOC1) (APOC1_HUMAN), these peptides are:
Lys-Val-Lys-Glu-Lys-Leu-Lys-Ile-Asp-Ser
Phe-Ser-Glu-Thr-Phe-Gln-Lys-Val-Lys-Glu-Lys-Leu-Lys-Ile-Asp-Ser
Val-Lys-Glu-Lys-Leu-Lys-Ile-Asp-Ser

The following peptides derive from Apolipoprotein C-II (Apo-CII) (ApoC-II) (Apolipoprotein C2) [Cleaved into: Proapolipoprotein C-II (ProapoC-II)] (APOC2) (APOC2 _HUMAN), these peptides are:
Leu-Thr-Gln-Val-Lys-Glu-Ser-Leu-Ser-Ser-Tyr
Phe-Ser-Lys-Ala-Leu-Pro-Arg
Tyr-Glu-Lys-Thr-Tyr-Leu-Pro-Ala-Val-Asp-Glu-Lys-Leu
Thr-Gln-Gln-Pro-Gln-Gln-Asp-Glu-Met-Pro-Ser-Pro-Thr-Phe-Leu
Thr-Gly-Ile-Phe-Thr-Asp-Gln-Val-Leu

The following peptide derive from Alpha-2-macroglobulin (Alpha-2-M) (C3 and PZP-like alpha-2-macroglobulin domain-containing protein 5) (A2M) (A2MG_HUMAN), this peptide is
Gly-Phe-Tyr-Glu-Ser-Asp-Val-Met-Gly-Arg

The following peptides derive from Apolipoprotein C-III (Apo-CIII) (ApoC-III) (Apolipoprotein C3) (APOC3) (APOC3_HUMAN), these peptides are:
Arg-Gly-Ser-Glu-Ser-Gly-Ile-Phe-Thr-Asn-Thr
Ser-Thr-Val-Lys-Asp-Lys-Phe-Ser-Glu-Phe
Tyr-Trp-Ser-Thr-Val-Lys-Asp-Lys-Phe
Tyr-Trp-Ser-Thr-Val-Lys-Asp-Lys-Phe-Ser-Glu-Phe

The following peptide derive from Retinoic acid receptor responder protein 2 (Chemerin) (RAR-responsive protein TIG2) (Tazarotene-induced gene 2 protein) (RARRES2) (RARR2 _HUMAN), this peptide is
Pro-Ser-Glu-Leu-Arg-Asp-Glu-His

The following peptide derive from Talin-1 (TLN1) (TLN1_HUMAN), this peptide is
Val-Val-Phe-Thr-Ala-Asn-Asp-Ser-Gly-Pro-Arg-Arg-Tyr-Thr-Ile-Ala

The following peptide derive from Plasminogen (EC 3.4.21.7) [Cleaved into: Plasmin heavy chain A; Activation peptide; Angiostatin; Plasmin heavy chain A, short form; Plasmin light chain B] (PLG) (PLMN_HUMAN), this peptide is
Asp-Asp-Tyr-Val-Asn-Thr-Gln-Gly-Ala-Ser-Leu

The following peptides derive from Serum amyloid P-component (SAP) (9.5S alpha-1-glycoprotein) [Cleaved into: Serum amyloid P-component(1-203)] (APCS) (SAMP_HUMAN), these peptides are:
His-Thr-Asp-Leu-Ser-Gly-Lys-Val
His-Thr-Asp-Leu-Ser-Gly-Lys-Val-Phe-Val-Phe
Tyr-Val-Ile-Ile-Lys-Pro-Leu-Val-Trp-Val

The following peptides derive from Serotransferrin (Transferrin) (Beta-1 metal-binding globulin) (Siderophilin) (TF) (TRFE_HUMAN), these peptides are:
Phe-Lys-Asp-Ser-Ala-His-Gly-Phe
Phe-Ser-Ser-Pro-His-Gly-Lys-Asp-Leu-Leu-Phe-Lys-Asp-Ser-Ala-His-Gly-Phe
Asn-Gln-Ala-Gln-Glu-His-Phe-Gly-Lys-Asp-Lys-Ser-Lys-Glu-Phe-Gln-Leu
Tyr-Ala-Val-Ala-Val-Val-Lys-Lys-Asp
Tyr-Ala-Val-Ala-Val-Val-Lys-Lys-Asp-Ser
Tyr-Val-Thr-Ala-Ile-Arg-Asn-Leu-Arg-Glu
Ser-Lys-Glu-Asp-Pro-Gln-Thr-Phe-Tyr-Tyr-Ala-Val-Ala
Tyr-Gly-Ser-Lys-Glu-Asp-Pro-Gln-Thr-Phe
Ala-Tyr-Leu-Ala-Pro-Asn-Asn-Leu-Lys-Pro-Val-Val-Ala-Glu
Ala-Tyr-Leu-Ala-Pro-Asn-Asn-Leu-Lys-Pro-Val-Val-Ala-Glu-Phe
Ala-Tyr-Leu-Ala-Pro-Asn-Asn-Leu-Lys-Pro-Val-Val-Ala-Glu-Phe-Tyr-Gly-Ser-Lys-Glu-Asp
Asp-Ala-Gly-Leu-Val-Tyr-Asp-Ala-Tyr
Asp-Ala-Tyr-Leu-Ala-Pro-Asn-Asn-Leu-Lys-Pro-Val-Val-Ala-Glu-Phe
Tyr-Leu-Ala-Pro-Asn-Asn-Leu-Lys-Pro-Val-Val-Ala-Glu
Tyr-Leu-Ala-Pro-Asn-Asn-Leu-Lys-Pro-Val-Val-Ala-Glu-Phe
Tyr-Leu-Ala-Pro-Asn-Asn-Leu-Lys-Pro-Val-Val-Ala-Glu-Phe-Tyr-Gly-Ser-Lys-Glu-Asp

The following peptides derive from Hemopexin (Beta-1B-glycoprotein) (HPX) (HEMO _HUMAN), these peptides are:
Ser-Ala-Leu-Thr-Ser-Asp-Asn-His-Gly-Ala-Thr-Tyr
Ser-Ala-Leu-Thr-Ser-Asp-Asn-His-Gly-Ala-Thr-Tyr-Ala-Phe-Ser-Gly-Thr-His-Tyr
Phe-Arg-Gln-Gly-His-Asn-Ser-Val-Phe

The following peptides derive from Clusterin (Aging-associated gene 4 protein) (Apolipoprotein J) (Apo-J) (Complement cytolysis inhibitor) (CLI) (Complement-associated protein SP-40,40) (Ku70-binding protein 1) (NA1/NA2) (Sulfated glycoprotein 2) (SGP-2) (Testosterone-repressed prostate message 2) (TRPM-2) [Cleaved into: Clusterin beta chain (ApoJalpha) (Complement cytolysis inhibitor a chain); Clusterin alpha chain (ApoJbeta) (Complement cytolysis inhibitor b chain)] (CLU) (CLUS_HUMAN), these peptides are:
Val-Ala-Glu-Lys-Ala-Leu-Gln-Glu-Tyr-Arg-Lys-Lys-His-Arg-Glu-Glu
Phe-Thr-Arg-Glu-Pro-Gln-Asp-Thr-Tyr-His-Tyr-Leu-Pro
Arg-Pro-His-Phe-Phe-Phe-Pro
Arg-Pro-His-Phe-Phe-Phe-Pro-Lys-Ser-Arg-Ile-Val

The following peptide derive from Alpha-1-acid glycoprotein 2 (AGP 2) (Orosomucoid-2) (OMD 2) (ORM2) (A1AG2_HUMAN), this peptide is
Phe-Leu-Arg-Asp-Thr-Lys-Thr-Leu

The following peptide derive from Inter-alpha-trypsin inhibitor heavy chain H2 (ITI heavy chain H2) (ITI-HC2) (Inter-alpha-inhibitor heavy chain 2) (Inter-alpha-trypsin inhibitor complex component II) (Serum-derived hyaluronan-associated protein) (SHAP) (ITIH2) (ITIH2 _HUMAN), this peptide is
Val-Ile-Glu-Pro-Gln-Gly-Leu-Arg-Phe

The following peptide derive from Inter-alpha-trypsin inhibitor heavy chain H1 (ITI heavy chain H1) (ITI-HC1) (Inter-alpha-inhibitor heavy chain 1) (Inter-alpha-trypsin inhibitor complex component III) (Serum-derived hyaluronan-associated protein) (SHAP) (ITIH1) (ITIH1_HUMAN), this peptide is
Phe-Val-Thr-Pro-Leu-Thr-Ser-Met

The following peptide derive from Galectin-3-binding protein (Basement membrane autoantigen p105) (Lectin galactoside-binding soluble 3-binding protein) (Mac-2-binding protein) (MAC2BP) (Mac-2 BP) (Tumor-associated antigen 90K) (LGALS3BP) (LG3BP _HUMAN), this peptide is
Arg-Gly-Pro-Leu-Val-Lys-Tyr-Ser-Ser-Asp-Tyr

The following peptide derive from Extracellular matrix protein 1 (Secretory component p85) (ECM1) (ECM1_HUMAN), this peptide is
Ala-Ser-Glu-Gly-Gly-Phe-Thr-Ala-Thr-Gly-Gln-Arg

TTR (Transthyretin), it has a wide range of functions such as thyroid hormone transport, neuroprotective target in cerebral ischemia, decreasing inflammation, secondary tissue damage and promoting post-injury repair. TTR levels (up or down) are connected to with the AIS outcomes. The past report clearly assessed the prognostic value of serum TTR in terms of short and long-term functional outcomes in [11] ischemic stroke patients. Thus, the peptides that are quantified in our analysis which is unique to AIS only and might serve as a diagnostic marker. ORM2 (Alpha-1-acid glycoprotein 2), Alpha-1-Acid Glycoprotein high abundant plasma, acute-phase proteins. It involves various stroke protecting mechanisms such as immunomodulatory, Inflammation-sensitive/anti-inflammatory, and strengthening the barrier function of capillary and transporting proteins. The plaque formation (focal thickening of the intima-media layer >1.2 mm) or embolic occlusion directly leads to the blockage of the blood flow and subsequently induction of inflammation that leads to development and progression of the ischemia. In the past it has reported that the combination of high Alpha-1-Acid Glycoprotein and carotid plaque substantially increase the risk of stroke. Moreover, in line with our study, the a previous study showed, the elevated levels of orosomucoid are associated with increased occurrence of carotid plaque and [12] increased incidence of ischemic stroke. Therefore, the peptides released from Alpha-1-acid glycoprotein 2 might benefit of a realistic and sensitive early diagnostic marker for AIS. In our peptidomics analysis, it clearly indicated that the levels of TF (Serotransferrin) & HPX (Hemopexin) were higher in the AIS. This finding are in line with past reports that showed Intraplaque hemorrhage/ atherosclerotic tissues from patients comparatively possessed higher levels of inflammatory macrophage subsets, however, lower levels of STF (serotransferrin) and [13] HPX (hemopexin). HPX is heme-binding protein, and it plays a protective role against oxidative stress and supports the inflammatory properties of high-density lipoprotein. Our results In AIS the levels of HPX peptides suggest that the higher level is the function of counteract [14] heme-driven macrophage-mediated inflammation. Serotransferin is acute phase response protein and iron binding transport protein from sites of absorption and heme degradation. In the AIS the upregulated peptides levels are beneficial to abnormal immune response, inflammation, malnutrition and abnormal in copper and iron metabolism. FGG (Fibrinogen gamma chain), Fibrinogen is the main protein for hemostatic system, coagulation factor and an acute-phase inflammatory marker. FGG is one of the common variant of Fibrinogen for ischemic stroke. Fibrinogen γ' has dual functions; it acts as antithrombotic (via thrombin and platelet binding to fibrinogen) and [15] prothrombotic (via the factor XIII B subunit). In our peptidomics analysis we have seen peptides from FGG are identified and quantified in only AIS which, [16] is in line with the previous report. Therefore, peptides from these proteins will be the most prominent marker for AIS patients. PLG (Plasminogen), physiologically, it is a common understanding of plasminogen levels in the circulation and it's activation upon the blockage due to the thrombi for the degradation or [17] thrombotic occlusions. Therefore, plasminogen dependent proteolysis either natural system or stroke treatment with tPA are beneficial and indicative for stroke. In our peptidomics analysis we have noticed the elevated levels of plasminogen peptides from AIS stroke which follows and fits the physiological function and impact on ischemic stroke outcome. As these peptides are unique to AIS, realistic, early marker peptides to AIS. This marker will also provide the hint to AIS immediately treatment with tissue-type plasminogen activator (t-PA). Moreover, finding a AIS-specific increase in serum plasminogen peptides levels in the early diagnosis of ischemic stroke is a very interesting observation that should prompt further research in the point of care diagnosis field. CLU (Clusterin) is one of the highly expressed ubiquitous glycoprotein in brain and undergoes local upregulation upon acute stroke. In our findings, the peptides from clusterin are upregulated particularly in the AIS which is in line with previous reports were they measured secretory clusterin isoforms and they found the [18] higher levels in blood as well as in urine of patients. Clusterin is also act as a cytoprotecting chaperone and it concentration is elevated in acute phase response such as oxidative stress, proteotoxicity and also early following ischemic damage. Abnormal lipid parameters such as total Cholesterol (TC), Low and high Density Lipoprotein (LDL/HDL) cholesterol are the most probable [19] risk factors for ischemic stroke (. Apolipoproteins are components mainly derived from these lipoproteins and have an important regulatory role in cholesterol transport. Therefore, the abnormal/elevated levels of apolipoproteins are the relevance of ischemic stroke pathophysiology. In our serum peptidomics analysis, we have noticed that several peptides from a various class of apolipoproteins (APOA2 (Apolipoprotein A-II), APOC3 (Apolipoprotein C-III), APOC1 (Apolipoprotein C-I), APOF (Apolipoprotein F)) are uniquely present in the AIS. In the future, upon the confirmation of these peptides for AIS can be beneficial to discriminate the patients with other kinds of stroke. It is well known that blockage or plaque/clot is the major reason to induce the AIS. The clots in the blood vessels lead to the enlargement and restriction or block completely the blood flow which causes the also lack of oxygen to these cells. Hence the marker we found specific to AIS having a role in oxygen transportation. HBB (Hemoglobin subunit beta), HBD (Hemoglobin subunit delta), HBA2 (Hemoglobin subunit alpha-2) peptides were upregulated in AIS which indicate that this is another physiological mechanism to take place during the AIS and cells tries to maintenance the blood as optimal oxygen levels so these oxygen transport molecules were upregulated. ECM1(Extracellular matrix protein 1) Involved in endochondral bone formation as negative regulator of bone mineralization. Stimulates the proliferation of endothelial cells and promotes angiogenesis. Inhibits MMP9 proteolytic activity. Inflammation mediated responses are found at the site of plaque and most predominantly during AIS. APCS (Serum amyloid P-component/SAP) is a member of the pentraxin family that may play a role in innate immunity. The first report showed the association of Serum amyloid P-component and CVD and [20] elevated SAP may indicate the presence of ischemia. This is a reason, in our analysis, the peptides from Serum amyloid P-component proteins were predominantly visible in AIS. This also has associated with fibrinogen and universal constituent of amyloid deposits indicates the good biomarker peptides for AIS. It may also function as a calcium-dependent lectin. Inter-alpha-trypsin inhibitor heavy chains are acute-phase proteins, and they are secreted into the blood primarily by the liver. In our peptidomics analysis reveals that Inter-alpha-trypsin inhibitor heavy chain H1, H2 (ITIH1/H2) particularly upregulated in AIS patients. These proteins are the act as inflammatory responsive proteins and that also play a role in extracellular matrix stabilization. Moreover, ITIH1 contains a potential peptide that could stimulate a broad spectrum of phagocytotic cells. LGALS3BP (Galectin-3-binding protein) is a cysteine-rich, macrophage scavenger receptor family protein. This protein also closely associated with the inflammatory response which is the feature of AIS. In the previous pilot study reported that the levels of plasma galectin-3 binding protein were significantly elevated, post-stroke delirium patients. Thus, it is in line with the previous report, and peptides from this protein which is unique to AIS could be potential diagnostic marker peptides. It may also stimulate host defense against viruses and tumor cells.

Peptides are a microenvironment feature and have unrestricted access to facilities the pass-through cellular membrane, endothelial cell barrier, penetrate into tissue, blood-brain barrier, junctions, etc. Therefore, serum peptides are most accurate to the physiological state of health than proteomics i.e., phenotypic. Even minute changes and unique signatures can be reflected in the peptidome (vitamins, cytokines, growth hormones are the peptides). This high susceptibility of serum peptidome to any pathophysiological disorders can be desirably reflected in peptidome is an advantage in biomarker discovery. To our knowledge, this is the first approach employed for serum peptidomics biomarker discovery of acute ischemic stroke (AIS). Furthermore, our workflows, including (sample preparation, MS measurements, and data analysis performance) are sensitive, and precise quantification facilitates the employing of our novel approach in various clinical applications including the diagnosis of acute ischemic stroke (AIS).

### References

[1] J. M. Wardlaw, V. Murray, E. Berge, and G. J. del Zoppo, "Thrombolysis for acute ischaemic stroke," Cochrane Database Syst. Rev., no. 7, 2014, doi: 10.1002/14651858.CD000213.pub3.
[2] A. A. Rabinstein, "Treatment of Acute Ischemic Stroke," Contin. Minneap. Minn, vol. 23, no. 1, Cerebrovascular Disease, pp. 62-81, Feb. 2017, doi: 10.1212/CON.0000000000000420.
[3] A. I. Qureshi et al., "Acute Ischemic Stroke and COVID-19: An Analysis of 27 676 Patients," Stroke, vol. 52, no. 3, Art. no. 3, Mar. 2021, doi: 10.1161/STROKEAHA.120.031786.
[4] P. Belani et al., "COVID-19 Is an Independent Risk Factor for Acute Ischemic Stroke," AJNR Am. J. Neuroradiol., vol. 41, no. 8, pp. 1361-1364, Aug. 2020, doi: 10.3174/ajnr.A6650.
[5] R. Richter et al., "Composition of the peptide fraction in human blood plasma: database of circulating human peptides," J. Chromatogr. B. Biomed. Sci. App., vol. 726, no. 1-2, Art. no. 1-2, Apr. 1999, doi: 10.1016/s0378-4347(99)00012-2.
[6] H. Ay, "Biomarkers for stroke diagnosis," WO2014121252A1, Aug. 07, 2014 Accessed: Dec. 17, 2022. [Online]. Available:
   https://patents.google.com/patent/WO2014121252A1/en
[7] T. Garcia-Berrocoso and J. Montaner Vilallonga, "Biomarkers for the Prognosis of Ischemic Stroke," May 01, 2014 Accessed: Dec. 17, 2022. [Online]. Available: https://patentscope.wipo.int/search/en/detail.jsf?docId=WO2014064202
[8] P. Broberg, T. Fehniger, C. Lindberg, and G. MARKO-VARGA, "Peptides as biomarkers of copd," WO2006118522A1, Nov. 09, 2006 Accessed: Dec. 17, 2022. [Online]. Available:
   https://patents.google.com/patent/WO2006118522A1/en?oq=PCT%2fSE2006%2f00050 7
[9] K. I. VADAKKAN, "Biomarkers for rapid detection of an occurrence of a stroke event," WO2014110674A1, Jul. 24, 2014 Accessed: Dec. 17, 2022. [Online]. Available:
   https://patents.google.com/patent/WO2014110674A1/en?oq=PCT%2fCA2014%2f05002 3
[10] J. M. Villalonga, A. S. ORIOL, and L. R. PASCUAL, "Biomarkers for stroke prognosis," WO2021009287A1, Jan. 21, 2021 Accessed: Dec. 17, 2022. [Online]. Available:
   https://patents.google.com/patent/WO2021009287A1/en?oq=PCT%2fEP2020%2f07015 2
[11] W. Ambrosius, S. Michalak, R. Kazmierski, N. Andrzejewska, and W. Kozubski, "Predictive value of serum transthyretin for outcome in acute ischemic stroke," PLOS ONE, vol. 12, no. 6, p. e0179806, Jun. 2017, doi: 10.1371/journal.pone.0179806.
[12] J. Berntsson, G. Östling, M. Persson, J. G. Smith, B. Hedblad, and G. Engström, "Orosomucoid, Carotid Plaque, and Incidence of Stroke," Stroke, vol. 47, no. 7, pp. 1858-1863, Jul. 2016, doi: 10.1161/STROKEAHA.116.013374.
[13] X.-M. Yuan, L. J. Ward, C. Forssell, N. Siraj, and W. Li, "Carotid Atheroma From Men Has Significantly Higher Levels of Inflammation and Iron Metabolism Enabled by Macrophages," Stroke, vol. 49, no. 2, pp. 419-425, Feb. 2018, doi: 10.1161/STROKEAHA.117.018724.
[14] F. Vinchi et al., "Hemopexin therapy reverts heme-induced proinflammatory phenotypic switching of macrophages in a mouse model of sickle cell disease," Blood, vol. 127, no. 4, pp. 473-486, Jan. 2016, doi: 10.1182/blood-2015-08-663245.
[15] M. W. Mosesson, "Fibrinogen and fibrin structure and functions," J. Thromb. Haemost. JTH, vol. 3, no. 8, pp. 1894-1904, Aug. 2005, doi: 10.1111/j.1538-7836.2005.01365.x.
[16] E. Y. L. Cheung et al., "Fibrinogen gamma' in ischemic stroke: a case-control study," Stroke, vol. 39, no. 3, pp. 1033-1035, Mar. 2008, doi: 10.1161/STROKEAHA.107.495499.
[17] R. L. Medcalf, "Plasminogen and stroke: more is better," J. Thromb. Haemost. JTH, vol. 14, no. 9, pp. 1819-1821, Sep. 2016, doi: 10.1111/jth.13399.
[18] V. Guido et al., "Serum and urine clusterin levels are elevated in stroke patients," J. Neurol. Sci., vol. 357, pp. e379-e380, Oct. 2015, doi: 10.1016/j.jns.2015.08.1352.
[19] T. Kurth, B. M. Everett, J. E. Buring, C. S. Kase, P. M. Ridker, and J. M. Gaziano, "Lipid levels and the risk of ischemic stroke in women," Neurology, vol. 68, no. 8, pp. 556-562, Feb. 2007, doi: 10.1212/01.wnl.0000254472.41810.0d.
[20] W. Koenig, "Serum amyloid P component and cardiovascular disease: is there a sensible link?," Arterioscler. Thromb. Vasc. Biol., vol. 27, no. 4, pp. 698-700, Apr. 2007, doi: 10.1161/01.ATV.0000259353.77404.c0.
[21] J. Lee, A. Park, S. Mun, H.J. Kim, H. Son, H. Choi, D. Kim, S.J. Lee, J.G. Kim, H.G. Kang, "Proteomics-Based Identification of Diagnostic Biomarkers Related to Risk Factors and Pathogenesis of Ischemic Stroke," Diagnostics (Basel, Switzerland) vol. 10, no. 5, pp. 340-354, May 2020, doi:10.3390/diagnostics10050340.

## Claims

1. An *in vitro* method of diagnosis of acute ischemic stroke (AIS) in human and differential diagnosis from intracranial hemorrhagic stroke comprising detection of the following 100 quantitative serum peptides: Val-Ser-Glu- Thr-Glu-Ser-Arg-Gly-Ser-Glu-Ser-Gly-Ile-Phe-Thr-Asn-Thr-Lys, His-Arg-His-Pro-Asp-Glu-Ala-Ala-Phe-Phe-Asp-Thr-Ala-Ser-Thr, Phe-Phe-Asp-Thr-Ala-Ser-Thr-Gly-Lys, Gln-Leu-Gly-Leu-Pro-Gly-Pro-Pro-Asp-Val-Pro-Asp-His-Ala-Ala, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Ala-Ala-Thr-Val-Gly-Ser-Leu-Ala-Gly-Gln-Pro-Leu-Gln-Glu-Arg, Pro-Gly-Ser-Thr-Gly-Thr-Trp-Asn-Pro-Gly-Ser-Ser-Glu-Arg, His-Asp-Trp-Gly-His-Glu-Lys-Gln-Arg, His-Pro-Asp-Glu-Ala-Ala-Phe-Phe-Asp-Thr-Ala-Ser-Thr-Gly-Lys, Ser-Ser-His-His-Pro-Gly-Ile-Ala-Glu-Phe-Pro-Ser-Arg, Val-His-Leu-Thr-Pro-Glu-Glu-Lys-Ser-Ala-Val-Thr-Ala-Leu, Glu-Ser-Ser-Ser-His-His-Pro-Gly-Ile-Ala-Glu-Phe-Pro-Ser-Arg, Thr-Ala-Asn-Asp-Ser-Gly-Pro-Arg-Arg-Tyr-Thr-Ile-Ala-Ala-Leu, Arg-His-Asp-Trp-Gly-His-Glu-Lys-Gln-Arg, Val-Lys-Leu-Val-Asn-Glu-Val-Thr-Glu, Gln-Phe-Thr-Ser-Ser-Thr-Ser-Tyr-Asn, Ser-Gly-Asn-Val-Ser-Pro-Gly-Thr-Arg-Arg-Glu-Tyr-His-Thr-Glu-Lys, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Leu-Asp-Asp-Asp-Leu-Glu-His-Gln-Gly-Gly-His-Val-Leu-Asp-His-Gly-His, Ala-Phe-Arg-Gln-Pro-Ser-Ser-Ala, Phe-Val-Ser-Glu-Thr-Glu-Ser-Arg-Gly-Ser-Glu-Ser-Gly-lle-Phe-Thr-Asn-Thr, Phe-Ser-Thr-Tyr-Asp-Arg-Asp-Asn-Asp-Gly-Trp, Thr-Val-Ile-Gln-Asn-Arg-Gln-Asp-Gly-Ser-Val-Asp-Phe, Arg-Glu-Tyr-His-Thr-Glu-Lys-Leu-Val-Thr-Ser, Gln-Ala-Gly-Ala-Ala-Gly-Ser-Arg-Met-Asn-Phe-Arg-Pro-Gly-Val-Leu-Ser-Ser, Ser-Pro-Gly-Ser-Gly-Asn-Ala-Arg-Pro-Asn-Asn-Pro-Asp-Trp-Gly-Thr-Phe, Ser-Asp-Ile-Ser-Ser-Thr-Arg-Gly-Phe-Pro-Ser-Val-Leu-Arg, Ile-Thr-His-Arg-Ile-His-Trp-Glu-Ser-Ala-Ser-Leu-Leu, Val-Ser-Gly-Trp-Gly-Arg-Asn-Ala-Asn-Phe, Phe-Asp-Thr-Ala-Ser-Thr-Gly-Lys-Thr-Phe-Pro-Gly-Phe, Ser-Ala-Gly-Ser-Trp-Asn-Ser-Gly-Ser-Ser-Gly-Pro-Gly-Ser-Thr-Gly-Asn-Arg-Asn, Val-His-Leu-Thr-Pro-Glu-Glu-Lys, Asp-Ala-Val-Arg-Gly-Ser-Pro-Ala-Ile-Asn-Val, Asp-Glu-Ala-Ala-Phe-Phe-Asp-Thr, Val-His-Leu-Thr-Pro-Glu-Glu-Lys-Ser-Ala-Val-Thr, Pro-Gly-Ser-Pro-Arg-Pro-Gly-Ser-Thr-Gly-Thr-Trp-Asn-Pro-Gly-Ser-Ser-Glu-Arg, Gly-Ser-Ser-Gly-Thr-Gly-Gly-Thr-Ala-Thr-Trp-Lys, Gly-Ser-Trp-Asn-Ser-Gly-Ser-Ser-Gly-Pro-Gly-Ser-Thr-Gly-Asn-Arg, Ser-Glu-Thr-Glu-Ser-Arg-Gly-Ser-Glu-Ser-Gly-Ile-Phe-Thr-Asn-Thr-Lys, Glu-Leu-Glu-Arg-Pro-Gly-Gly-Asn-Glu-Ile, Leu-Gly-Glu-Phe-Val-Ser-Glu-Thr-Glu-Ser-Arg, Ala-Leu-Glu-Glu-Tyr-Thr-Lys-Lys-Leu-Asn-Thr-Gln, Glu-Leu-Glu-Arg-Pro-Gly-Gly-Asn-Glu, Asp-Glu-Ala-Gly-Ser-Glu-Ala-Asp-His-Glu-Gly-Thr-His-Ser-Thr, Arg-His-Asp-Trp-Gly-His-Glu-Lys, Ala-Ala-Ser-Arg-Tyr-Leu-Asp-Lys-Thr-Glu-Gln, Arg-His-Pro-Asp-Glu-Ala-Ala-Phe, Pro-Asn-Ile-Leu-Arg-Leu-Glu-Ser-Glu-Glu-Thr, Ile-Glu-Gly-Val-Asp-Ala-Glu-Asp-Gly-His-Gly-Pro-Gly-Glu-Gln-Gln-Lys, Pro-Ser-Leu-Arg-Pro-Ala-Pro-Pro-Pro-Ile-Ser-Gly-Gly-Gly-Tyr, Thr-Asn-Thr-Lys-Glu-Ser-Ser-Ser-His-His-Pro-Gly-Ile-Ala-Glu-Phe-Pro-Ser-Arg-Gly, Ala-Glu-Val-Gly-Arg-Val-Gly-Tyr-Val-Ser-Gly-Trp-Gly-Arg-Asn-Ala-Asn-Phe, Asp-Glu-Ala-Ala-Phe-Phe-Asp- Thr-Ala-Ser- Thr-Gly-Lys, Lys-Val-Glu-Gln-Ala-Val-Glu-Thr-Glu-Pro-Glu-Pro-Glu-Leu-Arg-Gln-Gln-Thr, Met-Ala-Asp-Glu-Ala-Gly-Ser-Glu-Ala-Asp-His-Glu-Gly-Thr-His-Ser-Thr-Lys, Ser-Gly-Pro-Ser-Ser-Pro-Gln-Gln-Ala-Pro-Pro-Ala-Val-His, Gly-Ser-Glu-Ser-Gly-Ile-Phe-Thr-Asn-Thr-Lys-Glu-Ser-Ser-Ser-His-His-Pro-Gly-Ile-Ala-Glu-Phe-Pro-Ser-Arg-Gly, Arg-Glu-Glu-Ala-Pro-Ser-Leu-Arg, Glu-Glu-Val-Ser-Gly-Asn-Val-Ser-Pro-Gly-Thr, Glu-Asn-Glu-Gly-Phe-Thr-Val-Thr-Ala-Glu-Gly-Lys, Val-Thr-Glu-Pro-Ile-Ser-Ala-Glu-Ser-Gly-Glu-Gln-Val-Glu-Arg, Gly-Asp-Asp-Pro-Ser-Asp-Lys-Phe, Gly-Pro-Pro-Asp-Val-Pro-Asp-His-Ala, Glu-Glu-Val-Ser-Gly-Asn-Val-Ser-Pro-Gly-Thr-Arg-Arg-Glu, Tyr-His-Thr-Glu-Lys-Leu-Val, Tyr-Gln-Glu-Pro-Val-Leu-Gly-Pro-Val, Leu-Leu-Val-Arg-Tyr-Thr-Lys-Lys-Val-Pro-Gln-Val-Ser-Thr-Pro-Thr-Leu, Ile-Gln-Ile-Tyr-Lys-Lys-Leu-Arg-Thr-Ser-Ser-Phe, Ser-Val-Ser-Ala-Gly-Ser-Pro-His-Pro-Ala-Ile-Ala-Arg-Leu, His-Pro-Gly-Ile-Ala-Glu-Phe-Pro-Ser-Arg-Gly, Thr-Phe-Glu-Ser-Lys-Ser-Tyr, Ile-Glu-Gly-Val-Asp-Ala-Glu-Asp-Gly-His-Gly-Pro-Gly-Glu-Gln-Gln-Lys-Arg, Val-Leu-Val-Pro-Ala-Gly-Ser-Ala-Arg-Pro-Val-Ala-Phe, Asp-Ala-His-Lys-Ser-Glu-Val-Ala-His-Arg-Phe-Lys-Asp-Leu-Gly-Glu-Glu-Asn-Phe, Lys-Val-Lys-Glu-Lys-Leu-Lys-Ile-Asp-Ser, Glu-Phe-Val-Ser-Glu-Thr-Glu-Ser, His-Ala-Leu-Gln-Leu-Asn-Asn-Arg-Gln, Ser-Glu-Ser-Gly-Ile-Phe-Thr-Asn-Thr-Lys, Tyr-Val-Gly-Lys-Lys-Gln-Leu, Leu-Thr-Gln-Val-Lys-Glu-Ser-Leu-Ser-Ser-Tyr, Ser-Ser-His-His-Pro-Gly-Ile-Ala-Glu-Phe-Pro-Ser, Gly-Phe-Tyr-Glu-Ser-Asp-Val-Met-Gly-Arg, Gly-Gly-His-Leu-Asp-Ala-Lys-Gly-Ser-Phe, Asp-Val-Leu-Lys-Asp-Ser-Gly-Arg-Asp, His-Arg-His-Pro-Asp-Glu-Ala-Ala-Phe-Phe-Asp-Thr-Ala, Trp-Ser-Thr-Val-Lys-Asp-Lys-Phe-Ser-Glu-Phe, Arg-Gly-Ser-Glu-Ser-Gly-Ile-Phe-Thr-Asn-Thr, Glu-Glu-Ala-Pro-Ser-Leu-Arg-Pro-Ala-Pro-Pro-Pro-Ile-Ser-Gly-Gly-Gly-Tyr-Arg, Gly-Thr-Phe-Glu-Glu-Val-Ser-Gly-Asn-Val-Ser-Pro-Gly-Thr-Arg-Arg-Glu-Tyr-His-Thr-Glu, Gly-Ser-Ser-Gly-Thr-Gly-Gly-Thr-Ala-Thr-Trp-Lys-Pro-Gly-Ser-Ser, Ser-Ser-Ser-Tyr-Ser-Lys-Gln-Phe-Thr-Ser-Ser-Thr-Ser-Tyr-Asn-Arg-Gly-Asp-Ser-Thr-Phe-Glu-Ser-Lys-Ser-Tyr-Lys, Trp-Glu-Ser-Ala-Lys-Thr-Ala-Ala-Gln-Asn-Leu, Phe-Ser-Lys-Ala-Leu-Pro-Arg, Pro-Ser-Glu-Leu-Arg-Asp-Glu-His, Val-Val-Phe- Thr-Ala-Asn-Asp-Ser-Gly-Pro-Arg-Arg- Tyr- Thr-Ile-Ala, Leu-Gly-Glu-Phe-Val-Ser-Glu-Thr-Glu-Ser-Arg wherein the serum levels of the mentioned 100 peptides in sera of patients with AIS are increased 2 folds or more as compared with individuals without stroke and patients with intracranial hemorrhagic stroke and a list of the following 54 qualitative peptides: Asp-Asp-Tyr-Val-Asn-Thr-Gln-Gly-Ala-Ser-Leu, Tyr-Pro-Trp-Thr-Gln-Arg-Phe, Phe-Arg-Leu-Leu-Gly-Asn-Val-Leu, Tyr-Gln-Lys-Val-Val-Ala-Gly-Val-Ala-Asn-Ala-Leu-Ala-His-Lys-Tyr-His, Phe-Ser-Glu-Thr-Phe-Gln-Lys-Val-Lys-Glu-Lys-Leu-Lys-Ile-Asp-Ser, Val-Lys-Glu-Lys-Leu-Lys-Ile-Asp-Ser, Tyr-Glu-Lys-Thr-Tyr-Leu-Pro-Ala-Val-Asp-Glu-Lys-Leu, Thr-Gln-Gln-Pro-Gln-Gln-Asp-Glu-Met-Pro-Ser-Pro-Thr-Phe-Leu, Thr-Gly-Ile-Phe-Thr-Asp-Gln-Val-Leu, Ser- Thr-Val-Lys-Asp-Lys-Phe-Ser-Glu-Phe, Tyr-Trp-Ser-Thr-Val-Lys-Asp-Lys-Phe, Tyr- Trp-Ser- Thr-Val-Lys-Asp-Lys-Phe-Ser-Glu-Phe, His-Thr-Asp-Leu-Ser-Gly-Lys-Val, His-Thr-Asp-Leu-Ser-Gly-Lys-Val-Phe-Val-Phe, Tyr-Val-Ile-Ile-Lys-Pro-Leu-Val-Trp-Val, Ala-Val-Arg-Gly-Ser-Pro-Ala-Ile-Asn, Glu-Val-Val-Phe-Thr-Ala-Asn-Asp-Ser-Gly-Pro-Arg-Arg-Tyr-Thr-Ile-Ala, Glu-Val-Val-Phe-Thr-Ala-Asn-Asp-Ser-Gly-Pro-Arg-Arg-Tyr-Thr-Ile-Ala-Ala, Phe-Thr-Ala-Asn-Asp-Ser-Gly-Pro-Arg-Arg-Tyr-Thr-Ile-Ala-Ala, Phe-Val-Glu-Gly-Ile-Tyr-Lys-Val-Glu, Leu-Leu-Ser-Pro-Tyr-Ser-Tyr-Ser-Thr-Thr-Ala, Leu-Ser-Pro-Tyr-Ser-Tyr-Ser-Thr-Thr-Ala, Thr-Ala-Asn-Asp-Ser-Gly-Pro-Arg-Arg-Tyr-Thr-Ile-Ala-Ala-Leu-Leu-Ser-Pro-Tyr-Ser-Tyr-Ser-Thr-Thr-Ala, Val-Val-Phe-Thr-Ala-Asn-Asp-Ser-Gly-Pro-Arg-Arg-Tyr-Thr-Ile-Ala-Ala, Phe-Lys-Asp-Ser-Ala-His-Gly-Phe, Phe-Ser-Ser-Pro-His-Gly-Lys-Asp-Leu-Leu-Phe-Lys-Asp-Ser-Ala-His-Gly-Phe, Asn-Gln-Ala-Gln-Glu-His-Phe-Gly-Lys-Asp-Lys-Ser-Lys-Glu-Phe-Gln-Leu, Tyr-Ala-Val-Ala-Val-Val-Lys-Lys-Asp, Tyr-Ala-Val-Ala-Val-Val-Lys-Lys-Asp-Ser, Tyr-Val-Thr-Ala-Ile-Arg-Asn-Leu-Arg-Glu, Ser-Lys-Glu-Asp-Pro-Gln-Thr-Phe-Tyr-Tyr-Ala-Val-Ala, Tyr-Gly-Ser-Lys-Glu-Asp-Pro-Gln-Thr-Phe, Ala-Tyr-Leu-Ala-Pro-Asn-Asn-Leu-Lys-Pro-Val-Val-Ala-Glu, Ala- Tyr-Leu-Ala-Pro-Asn-Asn-Leu-Lys-Pro- Val-Val-Ala-Glu-Phe, Ala-Tyr-Leu-Ala-Pro-Asn-Asn-Leu-Lys-Pro-Val-Val-Ala-Glu-Phe-Tyr-Gly-Ser-Lys-Glu-Asp, Asp-Ala-Gly-Leu-Val-Tyr-Asp-Ala-Tyr, Asp-Ala-Tyr-Leu-Ala-Pro-Asn-Asn-Leu-Lys-Pro-Val-Val-Ala-Glu-Phe, Tyr-Leu-Ala-Pro-Asn-Asn-Leu-Lys-Pro-Val-Val-Ala-Glu, Tyr-Leu-Ala-Pro-Asn-Asn-Leu-Lys-Pro-Val-Val-Ala-Glu-Phe, Tyr-Leu-Ala-Pro-Asn-Asn-Leu-Lys-Pro-Val-Val-Ala-Glu-Phe-Tyr-Gly-Ser-Lys-Glu-Asp, Ser-Ala-Leu-Thr-Ser-Asp-Asn-His-Gly-Ala-Thr-Tyr, Ser-Ala-Leu-Thr-Ser-Asp-Asn-His-Gly-Ala-Thr-Tyr-Ala-Phe-Ser-Gly-Thr-His-Tyr, Phe-Arg-Gln-Gly-His-Asn-Ser-Val-Phe, Val-Ala-Glu-Lys-Ala-Leu-Gln-Glu-Tyr-Arg-Lys-Lys-His-Arg-Glu-Glu, Phe-Thr-Arg-Glu-Pro-Gln-Asp-Thr-Tyr-His-Tyr-Leu-Pro, Arg-Pro-His-Phe-Phe-Phe-Pro, Arg-Pro-His-Phe-Phe-Phe-Pro-Lys-Ser-Arg-Ile-Val, Phe-Leu-Arg-Asp-Thr-Lys-Thr-Leu, Val-Ile-Glu-Pro-Gln-Gly-Leu-Arg-Phe, Phe-Val-Thr-Pro-Leu-Thr-Ser-Met, Val-His-Leu-Thr-Pro-Glu-Glu-Lys-Ser, Trp-Gly-Lys-Val-Asn-Val-Asp-Glu-Val-Gly-Gly-Glu-Ala-Leu-Gly-Arg-Leu, Arg-Gly-Pro-Leu-Val-Lys-Tyr-Ser-Ser-Asp-Tyr, Ala-Ser-Glu-Gly-Gly-Phe-Thr-Ala-Thr-Gly-Gln-Arg wherein mentioned 54 peptides are detectable in sera of the patients with AIS as compared individuals without stroke and in sera of patients with intracranial hemorrhagic stroke.

2. The method according to claim 1, wherein sera levels of the following 32 quantitative peptides: Val-Ser-Glu- Thr-Glu-Ser-Arg-Gly-Ser-Glu-Ser-Gly- Ile-Phe- Thr-Asn-Thr-Lys, His-Arg-His-Pro-Asp-Glu-Ala-Ala-Phe-Phe-Asp-Thr-Ala-Ser-Thr, Phe-Phe-Asp-Thr-Ala-Ser-Thr-Gly-Lys, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Ala-Ala-Thr-Val-Gly-Ser-Leu-Ala-Gly-Gln-Pro-Leu-Gln-Glu-Arg, Pro-Gly-Ser-Thr-Gly-Thr-Trp-Asn-Pro-Gly-Ser-Ser-Glu-Arg, His-Asp-Trp-Gly-His-Glu-Lys-Gln-Arg, His-Pro-Asp-Glu-Ala-Ala-Phe-Phe-Asp-Thr-Ala-Ser-Thr-Gly-Lys, Ser-Ser-His-His-Pro-Gly-Ile-Ala-Glu-Phe-Pro-Ser-Arg, Glu-Ser-Ser-Ser-His-His-Pro-Gly-Ile-Ala-Glu-Phe-Pro-Ser-Arg, Ser-Gly-Asn-Val-Ser-Pro-Gly-Thr-Arg-Arg-Glu-Tyr-His-Thr-Glu-Lys, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Leu-Asp-Asp-Asp-Leu-Glu-His-Gln-Gly-Gly-His-Val-Leu-Asp-His-Gly-His, Phe-Val-Ser-Glu-Thr-Glu-Ser-Arg-Gly-Ser-Glu-Ser-Gly-Ile-Phe-Thr-Asn-Thr, Arg-Glu-Tyr-His-Thr-Glu-Lys-Leu-Val-Thr-Ser, Gly-Ser-Ser-Gly-Thr-Gly-Gly-Thr-Ala-Thr-Trp-Lys, Gly-Ser-Trp-Asn-Ser-Gly-Ser-Ser-Gly-Pro-Gly-Ser-Thr-Gly-Asn-Arg, Glu-Leu-Glu-Arg-Pro-Gly-Gly-Asn-Glu-Ile, Leu-Gly-Glu-Phe-Val-Ser-Glu-Thr-Glu-Ser-Arg, Glu-Leu-Glu-Arg-Pro-Gly-Gly-Asn-Glu, Asp-Glu-Ala-Gly-Ser-Glu-Ala-Asp-His-Glu-Gly-Thr-His-Ser-Thr, Asp-Glu-Ala-Ala-Phe-Phe-Asp-Thr-Ala-Ser-Thr-Gly-Lys, Met-Ala-Asp-Glu-Ala-Gly-Ser-Glu-Ala-Asp-His-Glu-Gly-Thr-His-Ser-Thr-Lys, Arg-Glu-Glu-Ala-Pro-Ser-Leu-Arg, Glu-Glu-Val-Ser-Gly-Asn-Val-Ser-Pro-Gly-Thr, Tyr-Gln-Glu-Pro-Val-Leu-Gly-Pro-Val, Ser-Glu-Ser-Gly-Ile-Phe- Thr-Asn- Thr-Lys, His-Arg-His-Pro-Asp-Glu-Ala-Ala-Phe-Phe-Asp-Thr-Ala are increased 30 folds or more in sera of AIS patients as compared with individuals without stroke and patients with intracranial hemorrhagic stroke.

3. The methodaccording to claim 1 or 2, wherein sera levels of the following 24 quantitative peptides: Val-Ser-Glu- Thr-Glu-Ser-Arg-Gly-Ser-Glu-Ser-Gly- Ile-Phe-Thr-Asn-Thr-Lys, Phe-Phe-Asp-Thr-Ala-Ser-Thr-Gly-Lys, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly- Tyr, Pro-Gly-Ser-Thr-Gly-Thr-Trp-Asn-Pro-Gly-Ser-Ser-Glu-Arg, His-Pro-Asp-Glu-Ala-Ala-Phe-Phe-Asp-Thr-Ala-Ser-Thr-Gly-Lys, Glu-Ser-Ser-Ser-His-His-Pro-Gly-Ile-Ala-Glu-Phe-Pro-Ser-Arg, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Leu-Asp-Asp-Asp-Leu-Glu-His-Gln-Gly-Gly-His-Val-Leu-Asp-His-Gly-His, Arg-Glu-Tyr-His-Thr-Glu-Lys-Leu-Val-Thr-Ser, Gly-Ser-Ser-Gly-Thr-Gly-Gly-Thr-Ala-Thr-Trp-Lys, Gly-Ser-Trp-Asn-Ser-Gly-Ser-Ser-Gly-Pro-Gly-Ser-Thr-Gly-Asn-Arg, Glu-Leu-Glu-Arg-Pro-Gly-Gly-Asn-Glu-Ile, Leu-Gly-Glu-Phe-Val-Ser-Glu-Thr-Glu-Ser-Arg, Glu-Leu-Glu-Arg-Pro-Gly-Gly-Asn-Glu, Asp-Glu-Ala-Ala-Phe-Phe-Asp-Thr-Ala-Ser-Thr-Gly-Lys, Arg-Glu-Glu-Ala-Pro-Ser-Leu-Arg, Glu-Glu-Val-Ser-Gly-Asn-Val-Ser-Pro-Gly-Thr, Tyr-Gln-Glu-Pro-Val-Leu-Gly-Pro-Val, Ser-Glu-Ser-Gly-Ile-Phe-Thr-Asn-Thr-Lys, His-Arg-His-Pro-Asp-Glu-Ala-Ala-Phe- Phe-Asp-Thr-Ala are increased 50 folds or more in sera of AIS patients as compared with individuals without stroke and patients with intracranial hemorrhagic stroke.

4. The method according to any of claim 1-3, wherein sera levels of following 16 quantitative peptides: Val-Ser-Glu- Thr-Glu-Ser-Arg-Gly-Ser-Glu-Ser-Gly- Ile-Phe-Thr-Asn-Thr-Lys, Phe-Phe-Asp-Thr-Ala-Ser-Thr-Gly-Lys, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly- Tyr, Pro-Gly-Ser-Thr-Gly-Thr-Trp-Asn-Pro-Gly-Ser-Ser-Glu-Arg, Glu-Ser-Ser-Ser-His-His-Pro-Gly-Ile-Ala-Glu-Phe-Pro-Ser-Arg, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Arg-Glu-Tyr-His-Thr-Glu-Lys-Leu-Val-Thr-Ser, Gly-Ser-Ser-Gly-Thr-Gly-Gly-Thr-Ala-Thr-Trp-Lys, Glu-Leu-Glu-Arg-Pro-Gly-Gly-Asn-Glu-Ile, Arg-Glu-Glu-Ala-Pro-Ser-Leu-Arg, Glu-Glu-Val-Ser-Gly-Asn-Val-Ser-Pro-Gly-Thr, Ser-Glu-Ser-Gly-Ile-Phe-Thr-Asn-Thr-Lys are increased 100 folds or more in sera of AIS patients as compared with individuals without stroke and patients with intracranial hemorrhagic stroke.

5. The method according to any of claim 1-4, wherein serum level of the following 1 quantitative peptide: Val-Ser-Glu- Thr-Glu-Ser-Arg-Gly-Ser-Glu-Ser-Gly- Ile-Phe- Thr-Asn-Thr-Lys is increased 1000 folds or more in sera of AIS patients as compared with individuals without stroke and patients with intracranial hemorrhagic stroke.

6. The method according to any of claim 1-5, wherein peptides are detected and measured in obtained serum samples.

7. The method according to any of claims 1-6, wherein the serum peptide level is measured with tandem mass spectrometry.

## Patentansprüche

1. Ein *in-vitro-*Verfahren zur Diagnose von akutem ischämischem Schlaganfall (AIS) beim Menschen und zur Differenzialdiagnose von intrakraniellem hämorrhagischem Schlaganfall, das den Nachweis der folgenden 100 quantitativen Serumpeptide beinhaltet: Val-Ser-Glu- Thr-Glu-Ser-Arg-Gly-Ser-Glu-Ser-Gly-Ile-Phe-Thr-Asn-Thr-Lys, His-Arg-His-Pro-Asp-Glu-Ala-Ala-Phe-Phe-Asp-Thr-Ala-Ser-Thr, Phe-Phe-Asp-Thr-Ala-Ser-Thr-Gly-Lys, Gln-Leu-Gly-Leu-Pro-Gly-Pro-Pro-Asp-Val-Pro-Asp-His-Ala-Ala, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Ala-Ala-Thr-Val-Gly-Ser-Leu-Ala-Gly-Gln-Pro-Leu-Gln-Glu-Arg, Pro-Gly-Ser-Thr-Gly-Thr-Trp-Asn-Pro-Gly-Ser-Ser-Glu-Arg, His-Asp-Trp-Gly-His-Glu-Lys-Gln-Arg, His-Pro-Asp-Glu-Ala-Ala-Phe-Phe-Asp-Thr-Ala-Ser-Thr-Gly-Lys, Ser-Ser-His-His-Pro-Gly-Ile-Ala-Glu-Phe-Pro-Ser-Arg, Val-His-Leu-Thr-Pro-Glu-Glu-Lys-Ser-Ala-Val-Thr-Ala-Leu, Glu-Ser-Ser-Ser-His-His-Pro-Gly-Ile-Ala-Glu-Phe-Pro-Ser-Arg, Thr-Ala-Asn-Asp-Ser-Gly-Pro-Arg-Arg-Tyr-Thr-Ile-Ala-Ala-Leu, Arg-His-Asp-Trp-Gly-His-Glu-Lys-Gln-Arg, Val-Lys-Leu-Val-Asn-Glu-Val-Thr-Glu, Gln-Phe-Thr-Ser-Ser-Thr-Ser-Tyr-Asn, Ser-Gly-Asn-Val-Ser-Pro-Gly-Thr-Arg-Arg-Glu-Tyr-His-Thr-Glu-Lys, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Leu-Asp-Asp-Asp-Leu-Glu-His-Gln-Gly-Gly-His-Val-Leu-Asp-His-Gly-His, Ala-Phe-Arg-Gln-Pro-Ser-Ser-Ala, Phe-Val-Ser-Glu-Thr-Glu-Ser-Arg-Gly-Ser-Glu-Ser-Gly-Ile-Phe-Thr-Asn-Thr, Phe-Ser-Thr-Tyr-Asp-Arg-Asp-Asn-Asp-Gly-Trp, Thr-Val-Ile-Gln-Asn-Arg-Gln-Asp-Gly-Ser-Val-Asp-Phe, Arg-Glu-Tyr-His-Thr-Glu-Lys-Leu-Val-Thr-Ser, Gln-Ala-Gly-Ala-Ala-Gly-Ser-Arg-Met-Asn-Phe-Arg-Pro-Gly-Val-Leu-Ser-Ser, Ser-Pro-Gly-Ser-Gly-Asn-Ala-Arg-Pro-Asn-Asn-Pro-Asp-Trp-Gly-Thr-Phe, Ser-Asp-Ile-Ser-Ser-Thr-Arg-Gly-Phe-Pro-Ser-Val-Leu-Arg, Ile-Thr-His-Arg-Ile-His-Trp-Glu-Ser-Ala-Ser-Leu-Leu, Val-Ser-Gly-Trp-Gly-Arg-Asn-Ala-Asn-Phe, Phe-Asp-Thr-Ala-Ser-Thr-Gly-Lys-Thr-Phe-Pro-Gly-Phe, Ser-Ala-Gly-Ser-Trp-Asn-Ser-Gly-Ser-Ser-Gly-Pro-Gly-Ser-Thr-Gly-Asn-Arg-Asn, Val-His-Leu-Thr-Pro-Glu-Glu-Lys, Asp-Ala-Val-Arg-Gly-Ser-Pro-Ala-Ile-Asn-Val, Asp-Glu-Ala-Ala-Phe-Phe-Asp-Thr, Val-His-Leu-Thr-Pro-Glu-Glu-Lys-Ser-Ala-Val-Thr, Pro-Gly-Ser-Pro-Arg-Pro-Gly-Ser-Thr-Gly-Thr-Trp-Asn-Pro-Gly-Ser-Ser-Glu-Arg, Gly-Ser-Ser-Gly-Thr-Gly-Gly-Thr-Ala-Thr-Trp-Lys, Gly-Ser-Trp-Asn-Ser-Gly-Ser-Ser-Gly-Pro-Gly-Ser-Thr-Gly-Asn-Arg, Ser-Glu-Thr-Glu-Ser-Arg-Gly-Ser-Glu-Ser-Gly-Ile-Phe-Thr-Asn-Thr-Lys, Glu-Leu-Glu-Arg-Pro-Gly-Gly-Asn-Glu-Ile, Leu-Gly-Glu-Phe-Val-Ser-Glu-Thr-Glu-Ser-Arg, Ala-Leu-Glu-Glu-Tyr-Thr-Lys-Lys-Leu-Asn-Thr-Gln, Glu-Leu-Glu-Arg-Pro-Gly-Gly-Asn-Glu, Asp-Glu-Ala-Gly-Ser-Glu-Ala-Asp-His-Glu-Gly-Thr-His-Ser-Thr, Arg-His-Asp-Trp-Gly-His-Glu-Lys, Ala-Ala-Ser-Arg-Tyr-Leu-Asp-Lys-Thr-Glu-Gln, Arg-His-Pro-Asp-Glu-Ala-Ala-Phe, Pro-Asn-Ile-Leu-Arg-Leu-Glu-Ser-Glu-Glu-Thr, Ile-Glu-Gly-Val-Asp-Ala-Glu-Asp-Gly-His-Gly-Pro-Gly-Glu-Gln-Gln-Lys, Pro-Ser-Leu-Arg-Pro-Ala-Pro-Pro-Pro-Ile-Ser-Gly-Gly-Gly-Tyr, Thr-Asn-Thr-Lys-Glu-Ser-Ser-Ser-His-His-Pro-Gly-Ile-Ala-Glu-Phe-Pro-Ser-Arg-Gly, Ala-Glu-Val-Gly-Arg-Val-Gly-Tyr-Val-Ser-Gly-Trp-Gly-Arg-Asn-Ala-Asn-Phe, Asp-Glu-Ala-Ala-Phe-Phe-Asp-Thr-Ala-Ser-Thr-Gly-Lys, Lys-Val-Glu-Gln-Ala-Val-Glu-Thr-Glu-Pro-Glu-Pro-Glu-Leu-Arg-Gln-Gln-Thr, Met-Ala-Asp-Glu-Ala-Gly-Ser-Glu-Ala-Asp-His-Glu-Gly-Thr-His-Ser-Thr-Lys, Ser-Gly-Pro-Ser-Ser-Pro-Gln-Gln-Ala-Pro-Pro-Ala-Val-His, Gly-Ser-Glu-Ser-Gly-Ile-Phe-Thr-Asn-Thr-Lys-Glu-Ser-Ser-Ser-His-His-Pro-Gly-Ile-Ala-Glu-Phe-Pro-Ser-Arg-Gly, Arg-Glu-Glu-Ala-Pro-Ser-Leu-Arg, Glu-Glu-Val-Ser-Gly-Asn-Val-Ser-Pro-Gly-Thr, Glu-Asn-Glu-Gly-Phe-Thr-Val-Thr-Ala-Glu-Gly-Lys, Val-Thr-Glu-Pro-Ile-Ser-Ala-Glu-Ser-Gly-Glu-Gln-Val-Glu-Arg, Gly-Asp-Asp-Pro-Ser-Asp-Lys-Phe, Gly-Pro-Pro-Asp-Val-Pro-Asp-His-Ala, Glu-Glu-Val-Ser-Gly-Asn-Val-Ser-Pro-Gly-Thr-Arg-Arg-Glu, Tyr-His-Thr-Glu-Lys-Leu-Val, Tyr-Gln-Glu-Pro-Val-Leu-Gly-Pro-Val, Leu-Leu-Val-Arg-Tyr-Thr-Lys-Lys-Val-Pro-Gln-Val-Ser-Thr-Pro-Thr-Leu, Ile-Gln-Ile-Tyr-Lys-Lys-Leu-Arg-Thr-Ser-Ser-Phe, Ser-Val-Ser-Ala-Gly-Ser-Pro-His-Pro-Ala-Ile-Ala-Arg-Leu, His-Pro-Gly-Ile-Ala-Glu-Phe-Pro-Ser-Arg-Gly, Thr-Phe-Glu-Ser-Lys-Ser-Tyr, Ile-Glu-Gly-Val-Asp-Ala-Glu-Asp-Gly-His-Gly-Pro-Gly-Glu-Gln-Gln-Lys-Arg, Val-Leu-Val-Pro-Ala-Gly-Ser-Ala-Arg-Pro-Val-Ala-Phe, Asp-Ala-His-Lys-Ser-Glu-Val-Ala-His-Arg-Phe-Lys-Asp-Leu-Gly-Glu-Glu-Asn-Phe, Lys-Val-Lys-Glu-Lys-Leu-Lys-Ile-Asp-Ser, Glu-Phe-Val-Ser-Glu-Thr-Glu-Ser, His-Ala-Leu-Gln-Leu-Asn-Asn-Arg-Gln, Ser-Glu-Ser-Gly-Ile-Phe-Thr-Asn-Thr-Lys, Tyr-Val-Gly-Lys-Lys-Gln-Leu, Leu-Thr-Gln-Val-Lys-Glu-Ser-Leu-Ser-Ser-Tyr, Ser-Ser-His-His-Pro-Gly-Ile-Ala-Glu-Phe-Pro-Ser, Gly-Phe-Tyr-Glu-Ser-Asp-Val-Met-Gly-Arg, Gly-Gly-His-Leu-Asp-Ala-Lys-Gly-Ser-Phe, Asp-Val-Leu-Lys-Asp-Ser-Gly-Arg-Asp, His-Arg-His-Pro-Asp-Glu-Ala-Ala-Phe-Phe-Asp-Thr-Ala, Trp-Ser-Thr-Val-Lys-Asp-Lys-Phe-Ser-Glu-Phe, Arg-Gly-Ser-Glu-Ser-Gly-Ile-Phe-Thr-Asn-Thr, Glu-Glu-Ala-Pro-Ser-Leu-Arg-Pro-Ala-Pro-Pro-Pro-Ile-Ser-Gly-Gly-Gly-Tyr-Arg, Gly-Thr-Phe-Glu-Glu-Val-Ser-Gly-Asn-Val-Ser-Pro-Gly-Thr-Arg-Arg-Glu-Tyr-His-Thr-Glu, Gly-Ser-Ser-Gly-Thr-Gly-Gly-Thr-Ala-Thr-Trp-Lys-Pro-Gly-Ser-Ser, Ser-Ser-Ser-Tyr-Ser-Lys-Gln-Phe-Thr-Ser-Ser-Thr-Ser-Tyr-Asn-Arg-Gly-Asp-Ser-Thr-Phe-Glu-Ser-Lys-Ser-Tyr-Lys, Trp-Glu-Ser-Ala-Lys-Thr-Ala-Ala-Gln-Asn-Leu, Phe-Ser-Lys-Ala-Leu-Pro-Arg, Pro-Ser-Glu-Leu-Arg-Asp-Glu-His, Val-Val-Phe-Thr-Ala-Asn-Asp-Ser-Gly-Pro-Arg-Arg-Tyr-Thr-Ile-Ala, Leu-Gly-Glu-Phe-Val-Ser-Glu-Thr-Glu-Ser-Arg wobei die Serumspiegel der genannten 100 Peptide in Seren von Patienten mit AIS im Vergleich zu Personen ohne Schlaganfall und Patienten mit intrakraniellem hämorrhagischem Schlaganfall um das Zweifache oder mehr erhöht sind, und eine Liste der nachstehenden 54 qualitativen Peptide: Asp-Asp-Tyr-Val-Asn-Thr-Gln-Gly-Ala-Ser-Leu, Tyr-Pro-Trp-Thr-Gln-Arg-Phe, Phe-Arg-Leu-Leu-Gly-Asn-Val-Leu, Tyr-Gln-Lys-Val-Val-Ala-Gly-Val-Ala-Asn-Ala-Leu-Ala-His-Lys-Tyr-His, Phe-Ser-Glu-Thr-Phe-Gln-Lys-Val-Lys-Glu-Lys-Leu-Lys-Ile-Asp-Ser, Val-Lys-Glu-Lys-Leu-Lys-Ile-Asp-Ser, Tyr-Glu-Lys-Thr-Tyr-Leu-Pro-Ala-Val-Asp-Glu-Lys-Leu, Thr-Gln-Gln-Pro-Gln-Gln-Asp-Glu-Met-Pro-Ser-Pro-Thr-Phe-Leu, Thr-Gly-Ile-Phe-Thr-Asp-Gln-Val-Leu, Ser-Thr-Val-Lys-Asp-Lys-Phe-Ser-Glu-Phe, Tyr-Trp-Ser-Thr-Val-Lys-Asp-Lys-Phe, Tyr-Trp-Ser-Thr-Val-Lys-Asp-Lys-Phe-Ser-Glu-Phe, His-Thr-Asp-Leu-Ser-Gly-Lys-Val, His-Thr-Asp-Leu-Ser-Gly-Lys-Val-Phe-Val-Phe, Tyr-Val-Ile-Ile-Lys-Pro-Leu-Val-Trp-Val, Ala-Val-Arg-Gly-Ser-Pro-Ala-Ile-Asn, Glu-Val-Val-Phe-Thr-Ala-Asn-Asp-Ser-Gly-Pro-Arg-Arg-Tyr-Thr-Ile-Ala, Glu-Val-Val-Phe-Thr-Ala-Asn-Asp-Ser-Gly-Pro-Arg-Arg-Tyr-Thr-Ile-Ala-Ala, Phe-Thr-Ala-Asn-Asp-Ser-Gly-Pro-Arg-Arg-Tyr-Thr-Ile-Ala-Ala, Phe-Val-Glu-Gly-Ile-Tyr-Lys-Val-Glu, Leu-Leu-Ser-Pro-Tyr-Ser-Tyr-Ser-Thr-Thr-Ala, Leu-Ser-Pro-Tyr-Ser-Tyr-Ser-Thr-Thr-Ala, Thr-Ala-Asn-Asp-Ser-Gly-Pro-Arg-Arg-Tyr-Thr-Ile-Ala-Ala-Leu-Leu-Ser-Pro-Tyr-Ser-Tyr-Ser-Thr-Thr-Ala, Val-Val-Phe-Thr-Ala-Asn-Asp-Ser-Gly-Pro-Arg-Arg-Tyr-Thr-Ile-Ala-Ala, Phe-Lys-Asp-Ser-Ala-His-Gly-Phe, Phe-Ser-Ser-Pro-His-Gly-Lys-Asp-Leu-Leu-Phe-Lys-Asp-Ser-Ala-His-Gly-Phe, Asn-Gln-Ala-Gln-Glu-His-Phe-Gly-Lys-Asp-Lys-Ser-Lys-Glu-Phe-Gln-Leu, Tyr-Ala-Val-Ala-Val-Val-Lys-Lys-Asp, Tyr-Ala-Val-Ala-Val-Val-Lys-Lys-Asp-Ser, Tyr-Val-Thr-Ala-Ile-Arg-Asn-Leu-Arg-Glu, Ser-Lys-Glu-Asp-Pro-Gln-Thr-Phe-Tyr-Tyr-Ala-Val-Ala, Tyr-Gly-Ser-Lys-Glu-Asp-Pro-Gln-Thr-Phe, Ala-Tyr-Leu-Ala-Pro-Asn-Asn-Leu-Lys-Pro-Val-Val-Ala-Glu, Ala- Tyr-Leu-Ala-Pro-Asn-Asn-Leu-Lys-Pro- Val- Val-Ala-Glu-Phe, Ala-Tyr-Leu-Ala-Pro-Asn-Asn-Leu-Lys-Pro-Val-Val-Ala-Glu-Phe-Tyr-Gly-Ser-Lys-Glu-Asp, Asp-Ala-Gly-Leu-Val-Tyr-Asp-Ala-Tyr, Asp-Ala-Tyr-Leu-Ala-Pro-Asn-Asn-Leu-Lys-Pro-Val-Val-Ala-Glu-Phe, Tyr-Leu-Ala-Pro-Asn-Asn-Leu-Lys-Pro-Val-Val-Ala-Glu, Tyr-Leu-Ala-Pro-Asn-Asn-Leu-Lys-Pro-Val-Val-Ala-Glu-Phe, Tyr-Leu-Ala-Pro-Asn-Asn-Leu-Lys-Pro-Val-Val-Ala-Glu-Phe-Tyr-Gly-Ser-Lys-Glu-Asp, Ser-Ala-Leu-Thr-Ser-Asp-Asn-His-Gly-Ala-Thr-Tyr, Ser-Ala-Leu-Thr-Ser-Asp-Asn-His-Gly-Ala-Thr-Tyr-Ala-Phe-Ser-Gly-Thr-His-Tyr, Phe-Arg-Gln-Gly-His-Asn-Ser-Val-Phe, Val-Ala-Glu-Lys-Ala-Leu-Gln-Glu-Tyr-Arg-Lys-Lys-His-Arg-Glu-Glu, Phe-Thr-Arg-Glu-Pro-Gln-Asp-Thr-Tyr-His-Tyr-Leu-Pro, Arg-Pro-His-Phe-Phe-Phe-Pro, Arg-Pro-His-Phe-Phe-Phe-Pro-Lys-Ser-Arg-Ile-Val, Phe-Leu-Arg-Asp-Thr-Lys-Thr-Leu, Val-Ile-Glu-Pro-Gln-Gly-Leu-Arg-Phe, Phe-Val-Thr-Pro-Leu-Thr-Ser-Met, Val-His-Leu-Thr-Pro-Glu-Glu-Lys-Ser, Trp-Gly-Lys-Val-Asn-Val-Asp-Glu-Val-Gly-Gly-Glu-Ala-Leu-Gly-Arg-Leu, Arg-Gly-Pro-Leu-Val-Lys-Tyr-Ser-Ser-Asp-Tyr, Ala-Ser-Glu-Gly-Gly-Phe-Thr-Ala-Thr-Gly-Gln-Arg wobei die erwähnten 54 Peptide in Seren von Patienten mit AIS im Vergleich zu Personen ohne Schlaganfall und in Seren von Patienten mit intrakraniellem hämorrhagischem Schlaganfall nachweisbar sind.

2. Verfahren nach Anspruch 1, wobei die Serumspiegel der folgenden 32 quantitativen Peptide: Val-Ser-Glu- Thr-Glu-Ser-Arg-Gly-Ser-Glu-Ser-Gly- Ile-Phe- Thr-Asn-Thr-Lys, His-Arg-His-Pro-Asp-Glu-Ala-Ala-Phe-Phe-Asp-Thr-Ala-Ser-Thr, Phe-Phe-Asp-Thr-Ala-Ser-Thr-Gly-Lys, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Ala-Ala-Thr-Val-Gly-Ser-Leu-Ala-Gly-Gln-Pro-Leu-Gln-Glu-Arg, Pro-Gly-Ser-Thr-Gly-Thr-Trp-Asn-Pro-Gly-Ser-Ser-Glu-Arg, His-Asp-Trp-Gly-His-Glu-Lys-Gln-Arg, His-Pro-Asp-Glu-Ala-Ala-Phe-Phe-Asp-Thr-Ala-Ser-Thr-Gly-Lys, Ser-Ser-His-His-Pro-Gly-Ile-Ala-Glu-Phe-Pro-Ser-Arg, Glu-Ser-Ser-Ser-His-His-Pro-Gly-Ile-Ala-Glu-Phe-Pro-Ser-Arg, Ser-Gly-Asn-Val-Ser-Pro-Gly-Thr-Arg-Arg-Glu-Tyr-His-Thr-Glu-Lys, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Leu-Asp-Asp-Asp-Leu-Glu-His-Gln-Gly-Gly-His-Val-Leu-Asp-His-Gly-His, Phe-Val-Ser-Glu-Thr-Glu-Ser-Arg-Gly-Ser-Glu-Ser-Gly-Ile-Phe-Thr-Asn-Thr, Arg-Glu-Tyr-His-Thr-Glu-Lys-Leu-Val-Thr-Ser, Gly-Ser-Ser-Gly-Thr-Gly-Gly-Thr-Ala-Thr-Trp-Lys, Gly-Ser-Trp-Asn-Ser-Gly-Ser-Ser-Gly-Pro-Gly-Ser-Thr-Gly-Asn-Arg, Glu-Leu-Glu-Arg-Pro-Gly-Gly-Asn-Glu-Ile, Leu-Gly-Glu-Phe-Val-Ser-Glu-Thr-Glu-Ser-Arg, Glu-Leu-Glu-Arg-Pro-Gly-Gly-Asn-Glu, Asp-Glu-Ala-Gly-Ser-Glu-Ala-Asp-His-Glu-Gly-Thr-His-Ser-Thr, Asp-Glu-Ala-Ala-Phe-Phe-Asp-Thr-Ala-Ser-Thr-Gly-Lys, Met-Ala-Asp-Glu-Ala-Gly-Ser-Glu-Ala-Asp-His-Glu-Gly-Thr-His-Ser-Thr-Lys, Arg-Glu-Glu-Ala-Pro-Ser-Leu-Arg, Glu-Glu-Val-Ser-Gly-Asn-Val-Ser-Pro-Gly-Thr, Tyr-Gln-Glu-Pro-Val-Leu-Gly-Pro-Val, Ser-Glu-Ser-Gly-Ile-Phe-Thr-Asn-Thr-Lys, His-Arg-His-Pro-Asp-Glu-Ala-Ala-Phe-Phe-Asp-Thr-Ala in den Seren von AIS-Patienten um das 30-fache oder mehr erhöht sind im Vergleich zu Personen ohne Schlaganfall und Patienten mit intrakraniellen hämorrhagischen Schlaganfällen.

3. Verfahren nach Anspruch 1 oder 2, wobei die Serumspiegel der folgenden 24 quantitativen Peptide: Val-Ser-Glu- Thr-Glu-Ser-Arg-Gly-Ser-Glu-Ser-Gly- Ile-Phe- Thr-Asn-Thr-Lys, Phe-Phe-Asp-Thr-Ala-Ser-Thr-Gly-Lys, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Pro-Gly-Ser-Thr-Gly-Thr-Trp-Asn-Pro-Gly-Ser-Ser-Glu-Arg, His-Pro-Asp-Glu-Ala-Ala-Phe-Phe-Asp-Thr-Ala-Ser-Thr-Gly-Lys, Glu-Ser-Ser-Ser-His-His-Pro-Gly-Ile-Ala-Glu-Phe-Pro-Ser-Arg, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Leu-Asp-Asp-Asp-Leu-Glu-His-Gln-Gly-Gly-His-Val-Leu-Asp-His-Gly-His, Arg-Glu-Tyr-His-Thr-Glu-Lys-Leu-Val-Thr-Ser, Gly-Ser-Ser-Gly-Thr-Gly-Gly-Thr-Ala-Thr-Trp-Lys, Gly-Ser-Trp-Asn-Ser-Gly-Ser-Ser-Gly-Pro-Gly-Ser-Thr-Gly-Asn-Arg, Glu-Leu-Glu-Arg-Pro-Gly-Gly-Asn-Glu-Ile, Leu-Gly-Glu-Phe-Val-Ser-Glu-Thr-Glu-Ser-Arg, Glu-Leu-Glu-Arg-Pro-Gly-Gly-Asn-Glu, Asp-Glu-Ala-Ala-Phe-Phe-Asp-Thr-Ala-Ser-Thr-Gly-Lys, Arg-Glu-Glu-Ala-Pro-Ser-Leu-Arg, Glu-Glu-Val-Ser-Gly-Asn-Val-Ser-Pro-Gly-Thr, Tyr-Gln-Glu-Pro-Val-Leu-Gly-Pro-Val, Ser-Glu-Ser-Gly-Ile-Phe-Thr-Asn-Thr-Lys, His-Arg-His-Pro-Asp-Glu-Ala-Ala-Phe- Phe-Asp-Thr-Ala in den Seren von AIS-Patienten um das 50-fache oder mehr erhöht sind im Vergleich zu Personen ohne Schlaganfall und Patienten mit intrakraniellen hämorrhagischen Schlaganfällen.

4. Verfahren nach einem der Ansprüche 1 bis 3, wobei die Serumspiegel der folgenden 16 quantitativen Peptide: Val-Ser-Glu- Thr-Glu-Ser-Arg-Gly-Ser-Glu-Ser-Gly- Ile-Phe- Thr-Asn-Thr-Lys, Phe-Phe-Asp-Thr-Ala-Ser-Thr-Gly-Lys, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Pro-Gly-Ser-Thr-Gly-Thr-Trp-Asn-Pro-Gly-Ser-Ser-Glu-Arg, Glu-Ser-Ser-Ser-His-His-Pro-Gly-Ile-Ala-Glu-Phe-Pro-Ser-Arg, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Arg-Glu-Tyr-His-Thr-Glu-Lys-Leu-Val-Thr-Ser, Gly-Ser-Ser-Gly-Thr-Gly-Gly-Thr-Ala-Thr-Trp-Lys, Glu-Leu-Glu-Arg-Pro-Gly-Gly-Asn-Glu-Ile, Arg-Glu-Glu-Ala-Pro-Ser-Leu-Arg, Glu-Glu-Val-Ser-Gly-Asn-Val-Ser-Pro-Gly-Thr, Ser-Glu-Ser-Gly-Ile-Phe-Thr-Asn-Thr-Lys in den Seren von AIS-Patienten um das 100-fache oder mehr erhöht sind im Vergleich zu Personen ohne Schlaganfall und Patienten mit intrakraniellen hämorrhagischen Schlaganfällen.

5. Verfahren nach einem der Ansprüche 1 bis 4, wobei der Serumspiegel des folgenden 1 quantitativen Peptids: Val-Ser-Glu- Thr-Glu-Ser-Arg-Gly-Ser-Glu-Ser-Gly- Ile-Phe- Thr-Asn-Thr-Lys in Seren von AIS-Patienten im Vergleich zu Personen ohne Schlaganfall und Patienten mit intrakraniellen hämorrhagischen Schlaganfällen um das 1000-fache oder mehr erhöht ist.

6. Verfahren nach einem der Ansprüche 1 bis 5, wobei Peptide in den erhaltenen Serumproben nachgewiesen und gemessen werden.

7. Verfahren nach einem der Ansprüche 1 bis 6, wobei der Serumpeptidspiegel mit Tandem-Massenspektrometrie gemessen wird.

## Revendications

1. Une méthode *in vitro* de diagnostic de l'accident vasculaire cérébral ischémique aigu (AIS) chez l'homme et de diagnostic différentiel par rapport à l'accident vasculaire cérébral hémorragique intracrânien comprenant la détection des 100 peptides sériques quantitatifs suivants: Val-Ser-Glu- Thr-Glu-Ser-Arg-Gly-Ser-Glu-Ser-Gly-Ile-Phe-Thr-Asn-Thr-Lys, His-Arg-His-Pro-Asp-Glu-Ala-Ala-Phe-Phe-Asp-Thr-Ala-Ser-Thr, Phe-Phe-Asp-Thr-Ala-Ser-Thr-Gly-Lys, Gln-Leu-Gly-Leu-Pro-Gly-Pro-Pro-Asp-Val-Pro-Asp-His-Ala-Ala, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Ala-Ala-Thr-Val-Gly-Ser-Leu-Ala-Gly-Gln-Pro-Leu-Gln-Glu-Arg, Pro-Gly-Ser-Thr-Gly-Thr-Trp-Asn-Pro-Gly-Ser-Ser-Glu-Arg, His-Asp-Trp-Gly-His-Glu-Lys-Gln-Arg, His-Pro-Asp-Glu-Ala-Ala-Phe-Phe-Asp-Thr-Ala-Ser-Thr-Gly-Lys, Ser-Ser-His-His-Pro-Gly-Ile-Ala-Glu-Phe-Pro-Ser-Arg, Val-His-Leu-Thr-Pro-Glu-Glu-Lys-Ser-Ala-Val-Thr-Ala-Leu, Glu-Ser-Ser-Ser-His-His-Pro-Gly-Ile-Ala-Glu-Phe-Pro-Ser-Arg, Thr-Ala-Asn-Asp-Ser-Gly-Pro-Arg-Arg-Tyr-Thr-Ile-Ala-Ala-Leu, Arg-His-Asp-Trp-Gly-His-Glu-Lys-Gln-Arg, Val-Lys-Leu-Val-Asn-Glu-Val-Thr-Glu, Gln-Phe-Thr-Ser-Ser-Thr-Ser-Tyr-Asn, Ser-Gly-Asn-Val-Ser-Pro-Gly-Thr-Arg-Arg-Glu-Tyr-His-Thr-Glu-Lys, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Leu-Asp-Asp-Asp-Leu-Glu-His-Gln-Gly-Gly-His-Val-Leu-Asp-His-Gly-His, Ala-Phe-Arg-Gln-Pro-Ser-Ser-Ala, Phe-Val-Ser-Glu-Thr-Glu-Ser-Arg-Gly-Ser-Glu-Ser-Gly-Ile-Phe-Thr-Asn-Thr, Phe-Ser-Thr-Tyr-Asp-Arg-Asp-Asn-Asp-Gly-Trp, Thr-Val-Ile-Gln-Asn-Arg-Gln-Asp-Gly-Ser-Val-Asp-Phe, Arg-Glu-Tyr-His-Thr-Glu-Lys-Leu-Val-Thr-Ser, Gln-Ala-Gly-Ala-Ala-Gly-Ser-Arg-Met-Asn-Phe-Arg-Pro-Gly-Val-Leu-Ser-Ser, Ser-Pro-Gly-Ser-Gly-Asn-Ala-Arg-Pro-Asn-Asn-Pro-Asp-Trp-Gly-Thr-Phe, Ser-Asp-Ile-Ser-Ser-Thr-Arg-Gly-Phe-Pro-Ser-Val-Leu-Arg, Ile-Thr-His-Arg-Ile-His-Trp-Glu-Ser-Ala-Ser-Leu-Leu, Val-Ser-Gly-Trp-Gly-Arg-Asn-Ala-Asn-Phe, Phe-Asp-Thr-Ala-Ser-Thr-Gly-Lys-Thr-Phe-Pro-Gly-Phe, Ser-Ala-Gly-Ser-Trp-Asn-Ser-Gly-Ser-Ser-Gly-Pro-Gly-Ser-Thr-Gly-Asn-Arg-Asn, Val-His-Leu-Thr-Pro-Glu-Glu-Lys, Asp-Ala-Val-Arg-Gly-Ser-Pro-Ala-Ile-Asn-Val, Asp-Glu-Ala-Ala-Phe-Phe-Asp-Thr, Val-His-Leu-Thr-Pro-Glu-Glu-Lys-Ser-Ala-Val-Thr, Pro-Gly-Ser-Pro-Arg-Pro-Gly-Ser-Thr-Gly-Thr-Trp-Asn-Pro-Gly-Ser-Ser-Glu-Arg, Gly-Ser-Ser-Gly-Thr-Gly-Gly-Thr-Ala-Thr-Trp-Lys, Gly-Ser-Trp-Asn-Ser-Gly-Ser-Ser-Gly-Pro-Gly-Ser-Thr-Gly-Asn-Arg, Ser-Glu-Thr-Glu-Ser-Arg-Gly-Ser-Glu-Ser-Gly-Ile-Phe-Thr-Asn-Thr-Lys, Glu-Leu-Glu-Arg-Pro-Gly-Gly-Asn-Glu-Ile, Leu-Gly-Glu-Phe-Val-Ser-Glu-Thr-Glu-Ser-Arg, Ala-Leu-Glu-Glu-Tyr-Thr-Lys-Lys-Leu-Asn-Thr-Gln, Glu-Leu-Glu-Arg-Pro-Gly-Gly-Asn-Glu, Asp-Glu-Ala-Gly-Ser-Glu-Ala-Asp-His-Glu-Gly-Thr-His-Ser-Thr, Arg-His-Asp-Trp-Gly-His-Glu-Lys, Ala-Ala-Ser-Arg-Tyr-Leu-Asp-Lys-Thr-Glu-Gln, Arg-His-Pro-Asp-Glu-Ala-Ala-Phe, Pro-Asn-Ile-Leu-Arg-Leu-Glu-Ser-Glu-Glu-Thr, Ile-Glu-Gly-Val-Asp-Ala-Glu-Asp-Gly-His-Gly-Pro-Gly-Glu-Gln-Gln-Lys, Pro-Ser-Leu-Arg-Pro-Ala-Pro-Pro-Pro-Ile-Ser-Gly-Gly-Gly-Tyr, Thr-Asn-Thr-Lys-Glu-Ser-Ser-Ser-His-His-Pro-Gly-Ile-Ala-Glu-Phe-Pro-Ser-Arg-Gly, Ala-Glu-Val-Gly-Arg-Val-Gly-Tyr-Val-Ser-Gly-Trp-Gly-Arg-Asn-Ala-Asn-Phe, Asp-Glu-Ala-Ala-Phe-Phe-Asp-Thr-Ala-Ser-Thr-Gly-Lys, Lys-Val-Glu-Gln-Ala-Val-Glu-Thr-Glu-Pro-Glu-Pro-Glu-Leu-Arg-Gln-Gln-Thr, Met-Ala-Asp-Glu-Ala-Gly-Ser-Glu-Ala-Asp-His-Glu-Gly-Thr-His-Ser-Thr-Lys, Ser-Gly-Pro-Ser-Ser-Pro-Gln-Gln-Ala-Pro-Pro-Ala-Val-His, Gly-Ser-Glu-Ser-Gly-Ile-Phe-Thr-Asn-Thr-Lys-Glu-Ser-Ser-Ser-His-His-Pro-Gly-Ile-Ala-Glu-Phe-Pro-Ser-Arg-Gly, Arg-Glu-Glu-Ala-Pro-Ser-Leu-Arg, Glu-Glu-Val-Ser-Gly-Asn-Val-Ser-Pro-Gly-Thr, Glu-Asn-Glu-Gly-Phe-Thr-Val-Thr-Ala-Glu-Gly-Lys, Val-Thr-Glu-Pro-Ile-Ser-Ala-Glu-Ser-Gly-Glu-Gln-Val-Glu-Arg, Gly-Asp-Asp-Pro-Ser-Asp-Lys-Phe, Gly-Pro-Pro-Asp-Val-Pro-Asp-His-Ala, Glu-Glu-Val-Ser-Gly-Asn-Val-Ser-Pro-Gly-Thr-Arg-Arg-Glu, Tyr-His-Thr-Glu-Lys-Leu-Val, Tyr-Gln-Glu-Pro-Val-Leu-Gly-Pro-Val, Leu-Leu-Val-Arg-Tyr-Thr-Lys-Lys-Val-Pro-Gln-Val-Ser-Thr-Pro-Thr-Leu, Ile-Gln-Ile-Tyr-Lys-Lys-Leu-Arg-Thr-Ser-Ser-Phe, Ser-Val-Ser-Ala-Gly-Ser-Pro-His-Pro-Ala-Ile-Ala-Arg-Leu, His-Pro-Gly-Ile-Ala-Glu-Phe-Pro-Ser-Arg-Gly, Thr-Phe-Glu-Ser-Lys-Ser-Tyr, Ile-Glu-Gly-Val-Asp-Ala-Glu-Asp-Gly-His-Gly-Pro-Gly-Glu-Gln-Gln-Lys-Arg, Val-Leu-Val-Pro-Ala-Gly-Ser-Ala-Arg-Pro-Val-Ala-Phe, Asp-Ala-His-Lys-Ser-Glu-Val-Ala-His-Arg-Phe-Lys-Asp-Leu-Gly-Glu-Glu-Asn-Phe, Lys-Val-Lys-Glu-Lys-Leu-Lys-Ile-Asp-Ser, Glu-Phe-Val-Ser-Glu-Thr-Glu-Ser, His-Ala-Leu-Gln-Leu-Asn-Asn-Arg-Gln, Ser-Glu-Ser-Gly-Ile-Phe-Thr-Asn-Thr-Lys, Tyr-Val-Gly-Lys-Lys-Gln-Leu, Leu-Thr-Gln-Val-Lys-Glu-Ser-Leu-Ser-Ser-Tyr, Ser-Ser-His-His-Pro-Gly-Ile-Ala-Glu-Phe-Pro-Ser, Gly-Phe-Tyr-Glu-Ser-Asp-Val-Met-Gly-Arg, Gly-Gly-His-Leu-Asp-Ala-Lys-Gly-Ser-Phe, Asp-Val-Leu-Lys-Asp-Ser-Gly-Arg-Asp, His-Arg-His-Pro-Asp-Glu-Ala-Ala-Phe-Phe-Asp-Thr-Ala, Trp-Ser-Thr-Val-Lys-Asp-Lys-Phe-Ser-Glu-Phe, Arg-Gly-Ser-Glu-Ser-Gly-Ile-Phe-Thr-Asn-Thr, Glu-Glu-Ala-Pro-Ser-Leu-Arg-Pro-Ala-Pro-Pro-Pro-Ile-Ser-Gly-Gly-Gly-Tyr-Arg, Gly-Thr-Phe-Glu-Glu-Val-Ser-Gly-Asn-Val-Ser-Pro-Gly-Thr-Arg-Arg-Glu-Tyr-His-Thr-Glu, Gly-Ser-Ser-Gly-Thr-Gly-Gly-Thr-Ala-Thr-Trp-Lys-Pro-Gly-Ser-Ser, Ser-Ser-Ser-Tyr-Ser-Lys-Gln-Phe-Thr-Ser-Ser-Thr-Ser-Tyr-Asn-Arg-Gly-Asp-Ser-Thr-Phe-Glu-Ser-Lys-Ser-Tyr-Lys, Trp-Glu-Ser-Ala-Lys-Thr-Ala-Ala-Gln-Asn-Leu, Phe-Ser-Lys-Ala-Leu-Pro-Arg, Pro-Ser-Glu-Leu-Arg-Asp-Glu-His, Val-Val-Phe-Thr-Ala-Asn-Asp-Ser-Gly-Pro-Arg-Arg-Tyr-Thr-Ile-Ala, Leu-Gly-Glu-Phe-Val-Ser-Glu-Thr-Glu-Ser-Arg dans lequel les concentrations sériques des 100 peptides mentionnés dans le sérum des patients atteints d'AIS sont augmentées d'un facteur égal ou supérieur à 2 par rapport aux individus sans accident vasculaire cérébral et aux patients atteints d'un accident vasculaire cérébral hémorragique intracrânien, ainsi qu'une liste des 54 peptides qualitatifs suivants: Asp-Asp-Tyr-Val-Asn-Thr-Gln-Gly-Ala-Ser-Leu, Tyr-Pro-Trp-Thr-Gln-Arg-Phe, Phe-Arg-Leu-Leu-Gly-Asn-Val-Leu, Tyr-Gln-Lys-Val-Val-Ala-Gly-Val-Ala-Asn-Ala-Leu-Ala-His-Lys-Tyr-His, Phe-Ser-Glu-Thr-Phe-Gln-Lys-Val-Lys-Glu-Lys-Leu-Lys-Ile-Asp-Ser, Val-Lys-Glu-Lys-Leu-Lys-Ile-Asp-Ser, Tyr-Glu-Lys-Thr-Tyr-Leu-Pro-Ala-Val-Asp-Glu-Lys-Leu, Thr-Gln-Gln-Pro-Gln-Gln-Asp-Glu-Met-Pro-Ser-Pro-Thr-Phe-Leu, Thr-Gly-Ile-Phe-Thr-Asp-Gln-Val-Leu, Ser-Thr-Val-Lys-Asp-Lys-Phe-Ser-Glu-Phe, Tyr-Trp-Ser-Thr-Val-Lys-Asp-Lys-Phe, Tyr-Trp-Ser-Thr-Val-Lys-Asp-Lys-Phe-Ser-Glu-Phe, His-Thr-Asp-Leu-Ser-Gly-Lys-Val, His-Thr-Asp-Leu-Ser-Gly-Lys-Val-Phe-Val-Phe, Tyr-Val-Ile-Ile-Lys-Pro-Leu-Val-Trp-Val, Ala-Val-Arg-Gly-Ser-Pro-Ala-Ile-Asn, Glu-Val-Val-Phe-Thr-Ala-Asn-Asp-Ser-Gly-Pro-Arg-Arg-Tyr-Thr-Ile-Ala, Glu-Val-Val-Phe-Thr-Ala-Asn-Asp-Ser-Gly-Pro-Arg-Arg-Tyr-Thr-Ile-Ala-Ala, Phe-Thr-Ala-Asn-Asp-Ser-Gly-Pro-Arg-Arg-Tyr-Thr-Ile-Ala-Ala, Phe-Val-Glu-Gly-Ile-Tyr-Lys-Val-Glu, Leu-Leu-Ser-Pro-Tyr-Ser-Tyr-Ser-Thr-Thr-Ala, Leu-Ser-Pro-Tyr-Ser-Tyr-Ser-Thr-Thr-Ala, Thr-Ala-Asn-Asp-Ser-Gly-Pro-Arg-Arg-Tyr-Thr-Ile-Ala-Ala-Leu-Leu-Ser-Pro-Tyr-Ser-Tyr-Ser-Thr-Thr-Ala, Val-Val-Phe-Thr-Ala-Asn-Asp-Ser-Gly-Pro-Arg-Arg-Tyr-Thr-Ile-Ala-Ala, Phe-Lys-Asp-Ser-Ala-His-Gly-Phe, Phe-Ser-Ser-Pro-His-Gly-Lys-Asp-Leu-Leu-Phe-Lys-Asp-Ser-Ala-His-Gly-Phe, Asn-Gln-Ala-Gln-Glu-His-Phe-Gly-Lys-Asp-Lys-Ser-Lys-Glu-Phe-Gln-Leu, Tyr-Ala-Val-Ala-Val-Val-Lys-Lys-Asp, Tyr-Ala-Val-Ala-Val-Val-Lys-Lys-Asp-Ser, Tyr-Val-Thr-Ala-Ile-Arg-Asn-Leu-Arg-Glu, Ser-Lys-Glu-Asp-Pro-Gln-Thr-Phe-Tyr-Tyr-Ala-Val-Ala, Tyr-Gly-Ser-Lys-Glu-Asp-Pro-Gln-Thr-Phe, Ala-Tyr-Leu-Ala-Pro-Asn-Asn-Leu-Lys-Pro-Val-Val-Ala-Glu, Ala- Tyr-Leu-Ala-Pro-Asn-Asn-Leu-Lys-Pro- Val- Val-Ala-Glu-Phe, Ala-Tyr-Leu-Ala-Pro-Asn-Asn-Leu-Lys-Pro-Val-Val-Ala-Glu-Phe-Tyr-Gly-Ser-Lys-Glu-Asp, Asp-Ala-Gly-Leu-Val-Tyr-Asp-Ala-Tyr, Asp-Ala-Tyr-Leu-Ala-Pro-Asn-Asn-Leu-Lys-Pro-Val-Val-Ala-Glu-Phe, Tyr-Leu-Ala-Pro-Asn-Asn-Leu-Lys-Pro-Val-Val-Ala-Glu, Tyr-Leu-Ala-Pro-Asn-Asn-Leu-Lys-Pro-Val-Val-Ala-Glu-Phe, Tyr-Leu-Ala-Pro-Asn-Asn-Leu-Lys-Pro-Val-Val-Ala-Glu-Phe-Tyr-Gly-Ser-Lys-Glu-Asp, Ser-Ala-Leu-Thr-Ser-Asp-Asn-His-Gly-Ala-Thr-Tyr, Ser-Ala-Leu-Thr-Ser-Asp-Asn-His-Gly-Ala-Thr-Tyr-Ala-Phe-Ser-Gly-Thr-His-Tyr, Phe-Arg-Gln-Gly-His-Asn-Ser-Val-Phe, Val-Ala-Glu-Lys-Ala-Leu-Gln-Glu-Tyr-Arg-Lys-Lys-His-Arg-Glu-Glu, Phe-Thr-Arg-Glu-Pro-Gln-Asp-Thr-Tyr-His-Tyr-Leu-Pro, Arg-Pro-His-Phe-Phe-Phe-Pro, Arg-Pro-His-Phe-Phe-Phe-Pro-Lys-Ser-Arg-Ile-Val, Phe-Leu-Arg-Asp-Thr-Lys-Thr-Leu, Val-Ile-Glu-Pro-Gln-Gly-Leu-Arg-Phe, Phe-Val-Thr-Pro-Leu-Thr-Ser-Met, Val-His-Leu-Thr-Pro-Glu-Glu-Lys-Ser, Trp-Gly-Lys-Val-Asn-Val-Asp-Glu-Val-Gly-Gly-Glu-Ala-Leu-Gly-Arg-Leu, Arg-Gly-Pro-Leu-Val-Lys-Tyr-Ser-Ser-Asp-Tyr, Ala-Ser-Glu-Gly-Gly-Phe-Thr-Ala-Thr-Gly-Gln-Arg dans lequel les 54 peptides mentionnés sont détectables dans les sérums des patients atteints d'AIS comparés aux individus sans accident vasculaire cérébral et dans les sérums des patients atteints d'accident vasculaire cérébral hémorragique intracrânien.

2. La méthode selon la revendication 1, dans laquelle les niveaux de sérums des 32 peptides quantitatifs suivants: Val-Ser-Glu- Thr-Glu-Ser-Arg-Gly-Ser-Glu-Ser-Gly- Ile-Phe- Thr-Asn-Thr-Lys, His-Arg-His-Pro-Asp-Glu-Ala-Ala-Phe-Phe-Asp-Thr-Ala-Ser-Thr, Phe-Phe-Asp-Thr-Ala-Ser-Thr-Gly-Lys, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Ala-Ala-Thr-Val-Gly-Ser-Leu-Ala-Gly-Gln-Pro-Leu-Gln-Glu-Arg, Pro-Gly-Ser-Thr-Gly-Thr-Trp-Asn-Pro-Gly-Ser-Ser-Glu-Arg, His-Asp-Trp-Gly-His-Glu-Lys-Gln-Arg, His-Pro-Asp-Glu-Ala-Ala-Phe-Phe-Asp-Thr-Ala-Ser-Thr-Gly-Lys, Ser-Ser-His-His-Pro-Gly-Ile-Ala-Glu-Phe-Pro-Ser-Arg, Glu-Ser-Ser-Ser-His-His-Pro-Gly-Ile-Ala-Glu-Phe-Pro-Ser-Arg, Ser-Gly-Asn-Val-Ser-Pro-Gly-Thr-Arg-Arg-Glu-Tyr-His-Thr-Glu-Lys, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Leu-Asp-Asp-Asp-Leu-Glu-His-Gln-Gly-Gly-His-Val-Leu-Asp-His-Gly-His, Phe-Val-Ser-Glu-Thr-Glu-Ser-Arg-Gly-Ser-Glu-Ser-Gly-Ile-Phe-Thr-Asn-Thr, Arg-Glu-Tyr-His-Thr-Glu-Lys-Leu-Val-Thr-Ser, Gly-Ser-Ser-Gly-Thr-Gly-Gly-Thr-Ala-Thr-Trp-Lys, Gly-Ser-Trp-Asn-Ser-Gly-Ser-Ser-Gly-Pro-Gly-Ser-Thr-Gly-Asn-Arg, Glu-Leu-Glu-Arg-Pro-Gly-Gly-Asn-Glu-Ile, Leu-Gly-Glu-Phe-Val-Ser-Glu-Thr-Glu-Ser-Arg, Glu-Leu-Glu-Arg-Pro-Gly-Gly-Asn-Glu, Asp-Glu-Ala-Gly-Ser-Glu-Ala-Asp-His-Glu-Gly-Thr-His-Ser-Thr, Asp-Glu-Ala-Ala-Phe-Phe-Asp-Thr-Ala-Ser-Thr-Gly-Lys, Met-Ala-Asp-Glu-Ala-Gly-Ser-Glu-Ala-Asp-His-Glu-Gly-Thr-His-Ser-Thr-Lys, Arg-Glu-Glu-Ala-Pro-Ser-Leu-Arg, Glu-Glu-Val-Ser-Gly-Asn-Val-Ser-Pro-Gly-Thr, Tyr-Gln-Glu-Pro-Val-Leu-Gly-Pro-Val, Ser-Glu-Ser-Gly-Ile-Phe-Thr-Asn-Thr-Lys, His-Arg-His-Pro-Asp-Glu-Ala-Ala-Phe-Phe-Asp-Thr-Ala sont multipliés par 30 ou plus dans le sérum des patients atteints d'AIS par rapport aux personnes n'ayant pas subi d'AVC et aux patients ayant subi un AVC hémorragique intracrânien.

3. La méthode selon les revendications 1 ou 2, dans laquelle les concentrations sériques des 24 peptides quantitatifs suivants: Val-Ser-Glu- Thr-Glu-Ser-Arg-Gly-Ser-Glu-Ser-Gly- IIe-Phe-Thr-Asn-Thr-Lys, Phe-Phe-Asp-Thr-Ala-Ser-Thr-Gly-Lys, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Pro-Gly-Ser-Thr-Gly-Thr-Trp-Asn-Pro-Gly-Ser-Ser-Glu-Arg, His-Pro-Asp-Glu-Ala-Ala-Phe-Phe-Asp-Thr-Ala-Ser-Thr-Gly-Lys, Glu-Ser-Ser-Ser-His-His-Pro-Gly-Ile-Ala-Glu-Phe-Pro-Ser-Arg, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Leu-Asp-Asp-Asp-Leu-Glu-His-Gln-Gly-Gly-His-Val-Leu-Asp-His-Gly-His, Arg-Glu-Tyr-His-Thr-Glu-Lys-Leu-Val-Thr-Ser, Gly-Ser-Ser-Gly-Thr-Gly-Gly-Thr-Ala-Thr-Trp-Lys, Gly-Ser-Trp-Asn-Ser-Gly-Ser-Ser-Gly-Pro-Gly-Ser-Thr-Gly-Asn-Arg, Glu-Leu-Glu-Arg-Pro-Gly-Gly-Asn-Glu-Ile, Leu-Gly-Glu-Phe-Val-Ser-Glu-Thr-Glu-Ser-Arg, Glu-Leu-Glu-Arg-Pro-Gly-Gly-Asn-Glu, Asp-Glu-Ala-Ala-Phe-Phe-Asp-Thr-Ala-Ser-Thr-Gly-Lys, Arg-Glu-Glu-Ala-Pro-Ser-Leu-Arg, Glu-Glu-Val-Ser-Gly-Asn-Val-Ser-Pro-Gly-Thr, Tyr-Gln-Glu-Pro-Val-Leu-Gly-Pro-Val, Ser-Glu-Ser-Gly-Ile-Phe-Thr-Asn-Thr-Lys, His-Arg-His-Pro-Asp-Glu-Ala-Ala-Phe- Phe-Asp-Thr-Ala sont multipliés par 50 ou plus dans le sérum des patients atteints d'AIS par rapport aux personnes n'ayant pas subi d'AVC et aux patients ayant subi un AVC hémorragique intracrânien.

4. La méthode selon l'une quelconque des revendications 1 à 3, dans laquelle les concentrations sériques des 16 peptides quantitatifs suivants: Val-Ser-Glu- Thr-Glu-Ser-Arg-Gly-Ser-Glu-Ser-Gly- Ile-Phe- Thr-Asn-Thr-Lys, Phe-Phe-Asp-Thr-Ala-Ser-Thr-Gly-Lys, Gly-Ser-Glu-Ser-Gly-Ile-Phe-Thr-Asn-Thr-Lys, Asn-Pro-Gly-Ser-Ser-Gly-Thr-Gly-Gly-Thr-Ala-Thr-Trp-Lys, Gln-Phe-Thr-Ser-Ser-Thr-Ser-Tyr-Asn-Arg, Ala-Pro-Pro-Pro-Ile-Ser-Gly-Gly-Gly-Tyr, Pro-Gly-Ser-Thr-Gly-Thr-Trp-Asn-Pro-Gly-Ser-Ser-Glu-Arg, Glu-Ser-Ser-Ser-His-His-Pro-Gly-Ile-Ala-Glu-Phe-Pro-Ser-Arg, Pro-Gly-Ser-Ser-Gly-Pro-Gly-Ser-Thr-Gly-Ser-Trp-Asn-Ser-Gly-Ser-Ser-Gly-Thr-Gly-Ser-Thr-Gly-Asn-Gln-Asn-Pro-Gly-Ser-Pro-Arg, Thr-Ser-Lys-Gly-Asp-Lys-Glu-Leu, Arg-Glu-Tyr-His-Thr-Glu-Lys-Leu-Val-Thr-Ser, Gly-Ser-Ser-Gly-Thr-Gly-Gly-Thr-Ala-Thr-Trp-Lys, Glu-Leu-Glu-Arg-Pro-Gly-Gly-Asn-Glu-Ile, Arg-Glu-Glu-Ala-Pro-Ser-Leu-Arg, Glu-Glu-Val-Ser-Gly-Asn-Val-Ser-Pro-Gly-Thr, Ser-Glu-Ser-Gly-Ile-Phe-Thr-Asn-Thr-Lys sont multipliés par 100 ou plus dans le sérum des patients atteints d'AIS par rapport aux personnes n'ayant pas subi d'AVC et aux patients ayant subi un AVC hémorragique intracrânien.

5. La méthode selon l'une quelconque des revendications 1 à 4, dans laquelle la concentration sérique du peptide quantitatif suivant: Val-Ser-Glu- Thr-Glu-Ser-Arg-Gly-Ser-Glu-Ser-Gly- Ile-Phe- Thr-Asn-Thr-Lys est multiplié par 1000 ou plus dans le sérum des patients atteints d'AIS par rapport aux personnes n'ayant pas subi d'AVC et aux patients ayant subi un AVC hémorragique intracrânien.

6. La méthode selon l'une quelconque des revendications 1 à 5, dans laquelle les peptides sont détectés et quantifiés dans les échantillons de sérum obtenus.

7. La méthode selon l'une quelconque des revendications 1 à 6, dans laquelle le niveau des peptides sériques est mesuré par spectrométrie de masse en tandem.
